# EUROPEAN PATENT APPLICATION

(11) **EP 0 974 573 A1**
(43) Date of publication of application: **26.01.2000**
(21) Application number: 98905736.9
(22) Date of filing: 03.03.1998
(51) Int. Cl.: C07C 49/255, C07C 235/38, C07D 295/02, C07D 295/04

(54) **PHENYLPROPENONE COMPOUNDS AND MEDICINES CONTAINING THE SAME**

(30) Priority: 03.03.1997 JP 4776097
(71) Applicant: Kabushiki Kaisha Yakult Honsha, Minato-ku, Tokyo 105-8660 (JP)
(72) Inventor: SAWADA, Haruji, Kabushiki Kaisha Yakult Honsha, Minato-ku, Tokyo 105-8660 (JP); AIYAMA, Ritsuo, Kabushiki Kaisha Yakult Honsha, Minato-ku, Tokyo 105-8660 (JP); HATANO, Hiroshi, Kabushiki Kaisha Yakult Honsha, Minato-ku, Tokyo 105-8660 (JP); OISHI, Kenji, Kabushiki Kaisha Yakult Honsha, Minato-ku, Tokyo 105-8660 (JP); YOSHIDA, Yasuto, Kabushiki Kaisha Yakult Honsha, Minato-ku, Tokyo 105-8660 (JP); WADA, Yasue, Kabushiki Kaisha Yakult Honsha, Minato-ku, Tokyo 105-8660 (JP); URAKAWA, Takako, Kabushiki Kaisha Yakult Honsha, Minato-ku, Tokyo 105-8660 (JP); MORI, Wakae, Kabushiki Kaisha Yakult Honsha, Minato-ku, Tokyo 105-8660 (JP); OHWAKI, Makoto, Kabushiki Kaisha Yakult Honsha, Minato-ku, Tokyo 105-8660 (JP); WATANABE,Tsunekazu Kabushiki Kaisha Yakult Honsha, Minato-ku, Tokyo o 105-8660 (JP); YOKOKURA, Teruo, Kabushiki Kaisha Yakult Honsha, Minato-ku, Tokyo 105-8660 (JP)
(74) Representative: Hartz, Nikolai F., Dr.
(86) International application number: JP9800867
(87) International publication number: WO9839280

(57) **Abstract**

This invention relates to compounds represented by the following formula (1) or (2) and also to medicines containing the same. 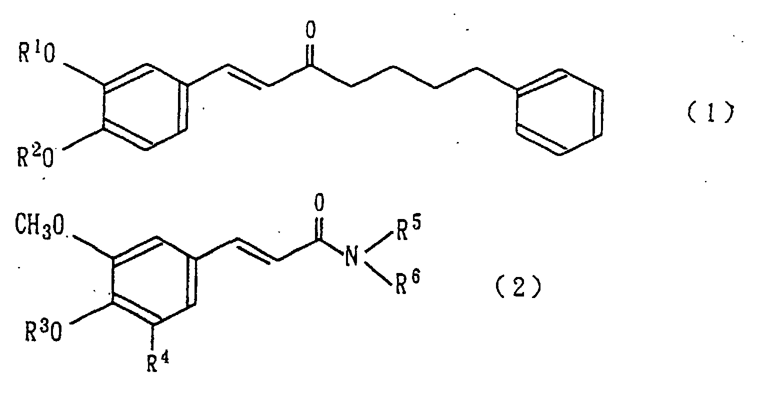 wherein R¹, R² and R³ each independently represent an alkyl group or the like, R⁴ represents an alkyl group, an alkoxyl group or the like, and -N(R⁵)R⁶ represents a substituted phenyl amino group, a substituted phenylpiperazinyl group or the like.

These compounds are useful as prophylatic and therapeutic agents for human arteriosclerosis, as they have strong ACAT inhibitory activity, lower the concentration of cholesterol in blood and inhibit accumulation of cholesterol.

## Description

This invention relates to novel compounds having inhibitory activities against acyl-CoA:cholesterol acyltransferase (hereinafter called "ACAT") and also to medicines containing the same, such as blood cholesterol lowering agents and prophylactic and therapeutic agents for arteriosclerosis.

### Background Art

Reflecting westernization in eating habits, the incidence of arteriosclerosis is significantly increasing in Japan. The relationship between the ever-increasing concentration of cholesterol in blood due to the western-style diet and arteriosclerosis has been elucidated through numerous epidemiological studies. Described specifically, it is considered that there is a significant correlation between the concentration of cholesterol in serum and the probability of incidence of an ischemic heart disease and that treatment of hypercholesterolemia will lead to prevention of arteriosclerosis. For hypercholesterolemia, hypertrophy of a tunica intima due to accumulation of a lipid on a blood vessel is a characteristic lesion. In recent years, foam cells in which macrophage origin cells store cholesterol esters as fat droplets are observed at lesions of atherosclerosis. Cholesterol is therefore postulated to have a deep relation to the progression of the pathema.

On the other hand, ACAT is a fatty acid esterification enzyme for cholesterol, and an inhibitor against this enzyme is expected as a therapeutic for arteriosclerosis [Drago R. et al., Tips, **194**(12), 1991]. Namely, it is reported that ACAT activity is high in the wall of a blood vessel at the site of an arteriosclerotic lesion and that cholesterol is accumulated on the wall of the blood vessel. Accordingly, use of a substance equipped with ACAT activity inhibitory effects can inhibit the esterification of cholesterol, free cholesterol which exist in cells is removed from ta deposited site by high density lipoproteins (HDL), is carried to the liver, and is metabolized there. It is therefore possible to inhibit accumulation of cholesterol esters at the site of the lesion, thereby making it possible to obtain direct anti-arteriosclerotic effects.

Further, cholesterol contained in a diet is esterified by ACAT in intestinal mucosal cells and is then released as a chylomicron component into a stream of blood. Hence, use of an ACAT inhibitor can inhibit the absorption of cholesterol from a diet into intestinal mucosal cells, thereby making it possible to lower the concentration of cholesterol in blood. As a result, arteriosclerosis can be inhibited.

In addition, cholesterol esters brought to the liver by chylomicrons are decomposed into free cholesterol by the cholesterolesterase, and together with free cholesterol synthesized in the liver, are then converted again into cholesterol esters by ACAT. These cholesterol esters are taken in very low density lipoproteins (VLDL) and are then released into blood. If the esterification of cholesterol is inhibited by such an ACAT inhibitor, the amount of free cholesterol in the liver increases, the cholesterol synthesis system is inhibited, and at the same time, the activity of 7α-hydroxylase which is a rate-limiting enzyme in the conversion pathway of cholesterol into bile acid becomes sthenic, thereby enhancing the dissimilation and excretion of cholesterol into bile acid.

As has been described above, use of an ACAT inhibiting substance inhibits the absorption of cholesterol from a diet through the small intestine and also inhibits the re-esterification of cholesterol in the liver. Coupled further with the accelerating action of dissimilation and excretion of cholesterol, the concentration of cholesterol in blood can be lowered. As a result, the concentration of cholesterol in the liver is lowered, thereby prohibiting fatty liver.

Under the foregoing background, screening has been conducted toward ACAT inhibiting substances, and some ACAT inhibiting substances which are produced by microorganisms are known. These substances are however still not regarded to be satisfactory when their action and safety are taken into parallel consideration.

An object of the present invention is therefore to provide a medicine, which has high ACAT inhibitory activity, involves no problem in safety, and is useful as a blood cholesterol lowering agent, an arteriosclerosis preventive or the like.

### Disclosure of the Invention

With the foregoing circumstances in view, the present inventors have proceeded with screening toward substances having ACAT activity inhibitory effects. As a result, it was found diallyl heptanoid (yakutinone B), a component of bitter cardamon which is a species of plant, exhibits ACAT activity inhibitory effects. Using it as a leadoff compound, a variety of derivatives have been prepared. By studying correlations between their structures and their ACAT inhibitory activities, a series of substances having strong ACAT inhibitory activities has been obtained. These substances also exhibit superb blood cholesterol lowering effects and fatty acid inhibiting effects on animals and hence, are also useful as anti-hyperlipemic medicines.

Described specifically, the present invention provides a phenylpropenone compound represented by the following formula (1): 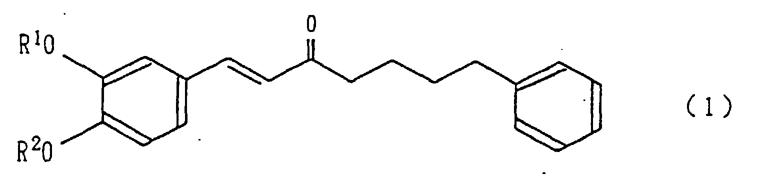 wherein R¹ and R² may be the same or different and each independently represent a hydrogen atom, a phenyl group, an aralkyl group, or a saturated or unsaturated, linear or branched hydrocarbon group.

The present invention also provides a phenylpropenone compound represented by the following formula (2): 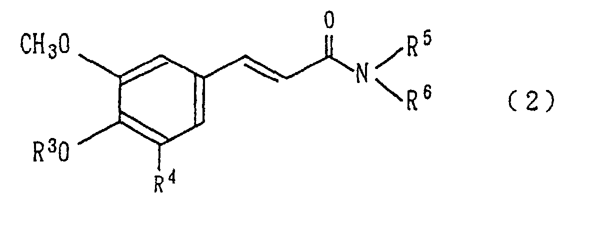 wherein R³ represents a hydrogen atom, a phenyl group, an aralkyl group, a saturated or unsaturated, linear or branched hydrocarbon group, an alkoxyalkyl group or an aryloxyalkyl group, R⁴ represents a hydrogen atom, a halogen atom, a lower alkyl group, a lower alkoxyl group, a phenyl group, an aralkyl group, a halogeno(lower alkyl) group or a nitro group, and concerning R⁵ and R⁶, R⁶ represents a substituted or unsubstituted phenyl group, a substituted or unsubstituted aralkyl group, a substituted or unsubstituted aralkylpiperidino group or a substituted or unsubstituted anilinocarbonylmethyl group when R⁵ is a hydrogen atom, or R⁵ and R⁶ may be coupled together with the adjacent nitrogen atom to form a piperazine ring which may be substituted at the 4-position thereof with a substituted or unsubstituted phenyl group or with a substituted or unsubstituted heterocyclic group.

In a further aspect, the present invention also provides an acyl-CoA:cholesterol acyltransferase (ACAT) inhibitor which comprises as an active ingredient the phenylpropenone compound represented by the formula (1) or (2).

In a still further aspect, the present invention also provides a medicine which comprises as an active ingredient the phenylpropenone compound represented by the formula (1) or (2).

In a still further aspect, the present invention also provides a medicinal composition which comprises the phenylpropenone compound represented by the formula (1) or (2) and a medicinally acceptable carrier.

In a still further aspect, the present invention also provides use of the phenylpropenone compound represented by the formula (1) or (2) as an acyl-CoA:cholesterol acyltransferase (ACAT) inhibitor.

In a still further aspect, the present invention also provides use of the phenylpropenone compound represented by the formula (1) or (2) as a medicine.

In a still further aspect, the present invention also provides a method for the treatment of hypercholesterolemia, fatty liver or arteriosclerosis, which comprises administering an effective amount of the phenylpropenone compound represented by the formula (1) or (2) to a patient.

### Brief Description of the Drawings

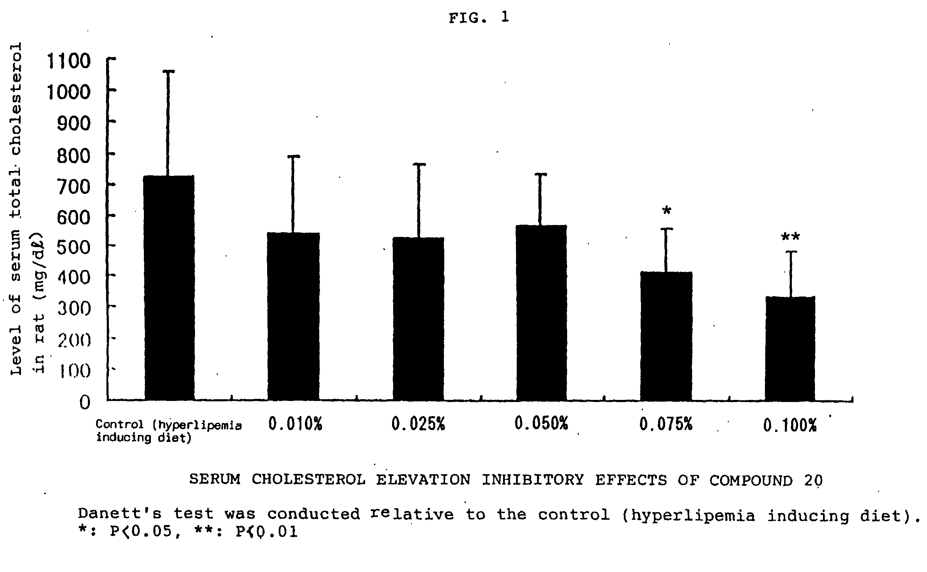

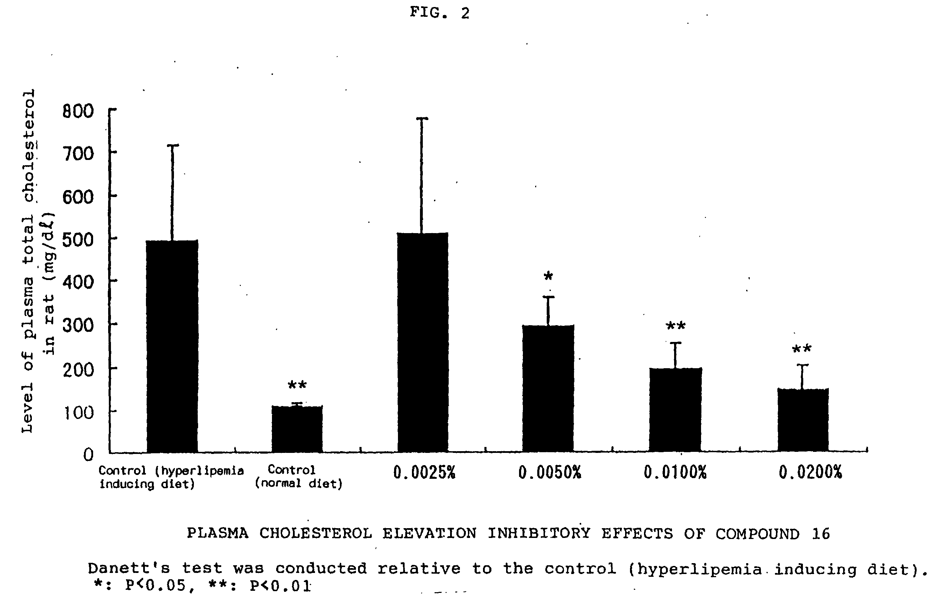

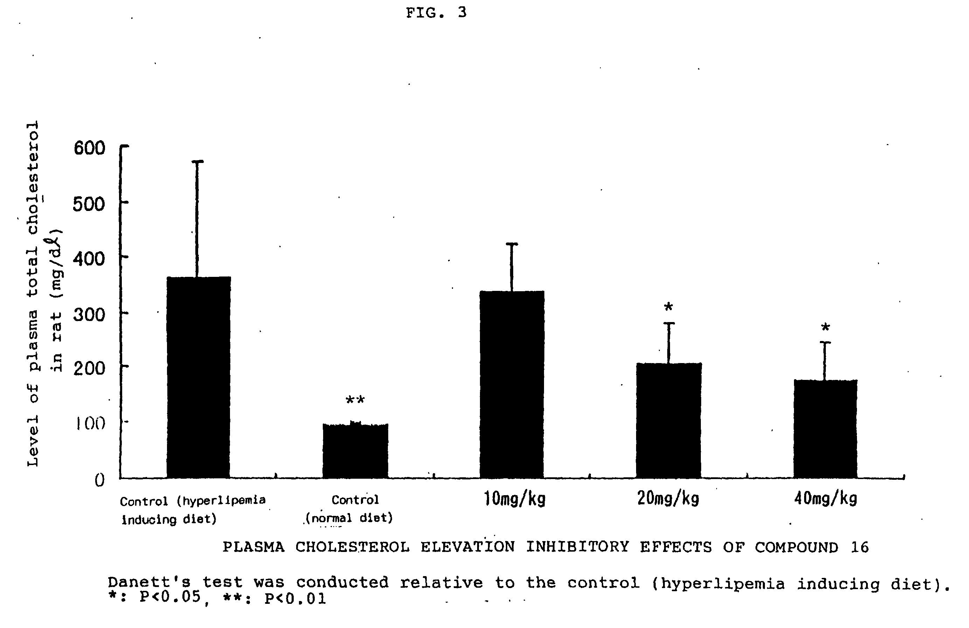

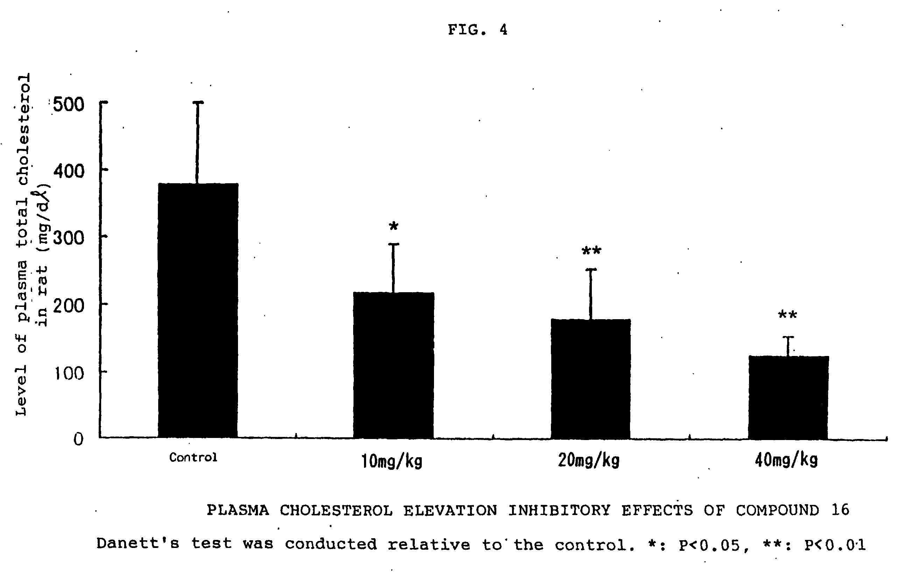

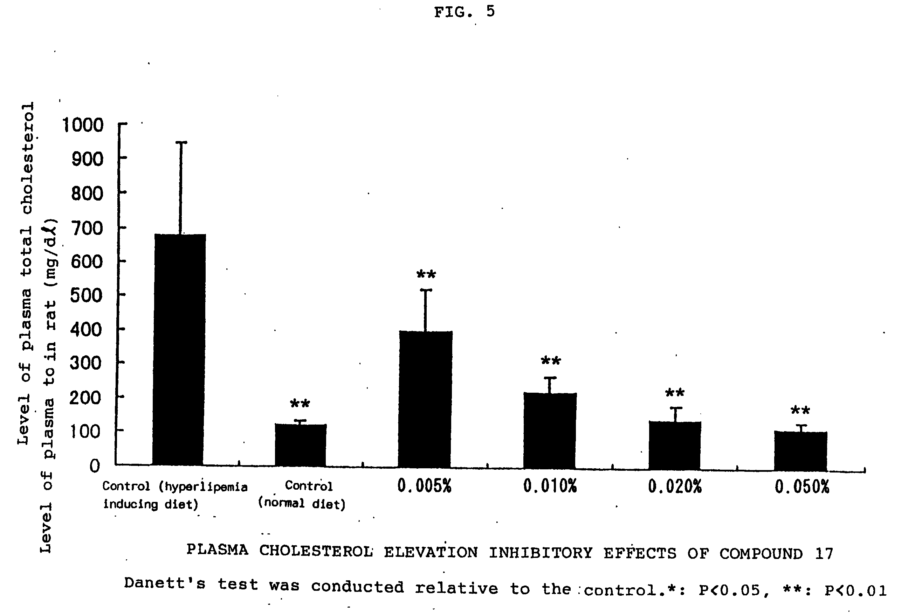

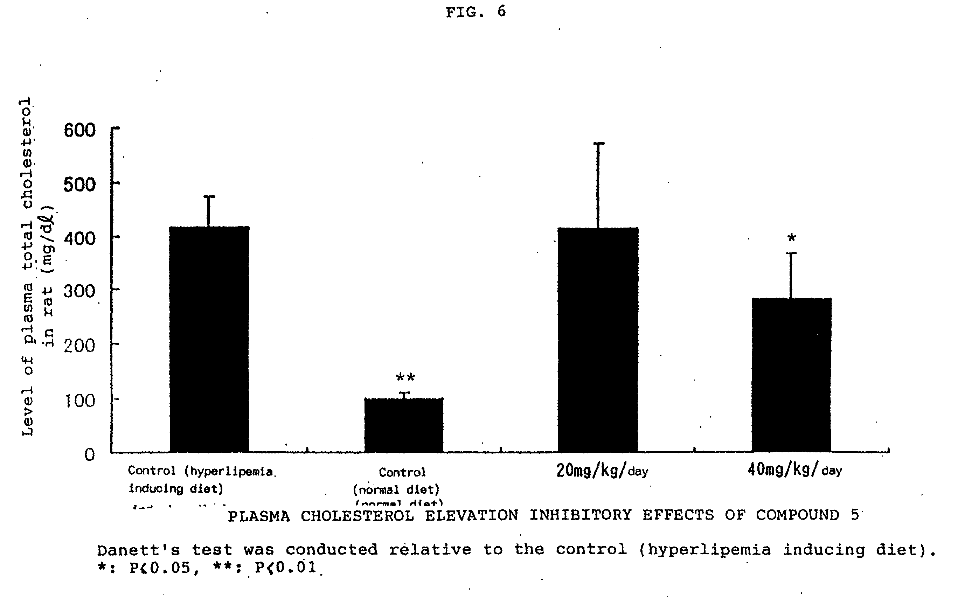

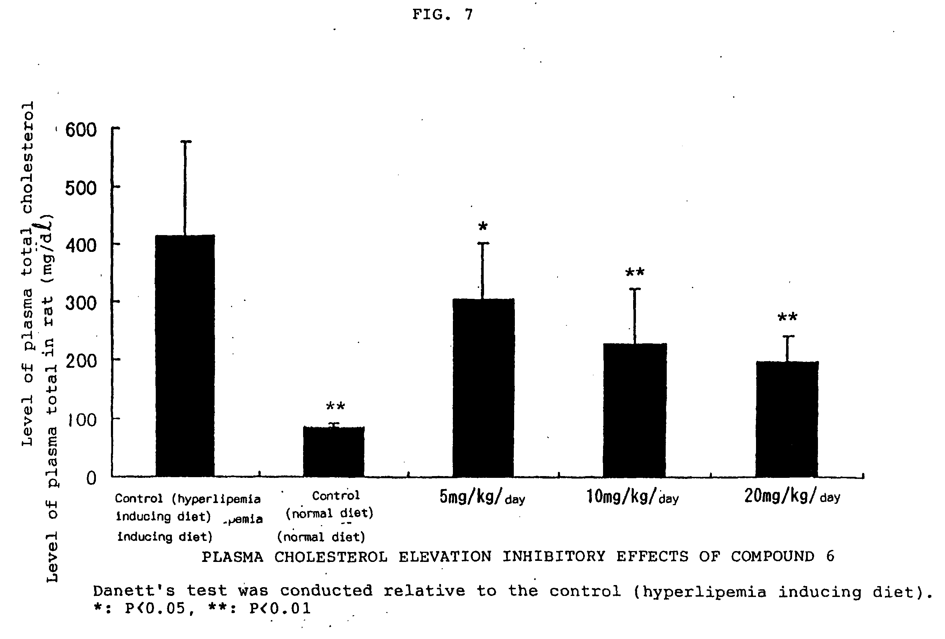

FIG. 1 illustrates serum cholesterol elevation inhibiting effects of Compound 20. FIG. 2 shows plasma cholesterol elevation inhibiting effects of Compound 16. FIG. 3 depicts plasma cholesterol elevation inhibiting effects of Compound 16. FIG. 4 illustrates plasma cholesterol elevation inhibiting effects of Compound 16. FIG. 5 shows plasma cholesterol elevation inhibiting effects of Compound 17. FIG. 6 depicts plasma cholesterol elevation inhibiting effects of Compound 5. FIG. 7 shows plasma cholesterol elevation inhibiting effects of Compound 6.

### Best Modes for Carrying Out the Invention

In the formulas (1) and (2) which represent phenylpropenone compounds according to the present invention, illustrative of aralkyl groups represented by R¹, R², R³, R⁴ ad R⁶ are aralkyl groups having 7 to 14 carbon atoms in total, preferably phenyl-C₁₋₅alkyl groups, and especially benzyl, phenylethyl, phenylpropyl, phenylbutyl and the like. Further, the "aralkyl"s in the aralkylamino group and aralkylpiperidino group represented by R⁶ are similar to those exemplified above. Incidentally, groups which can substitute on the aralkyl group and aralkylpiperidino group in R⁶ are those substitutable on aromatic rings, including 1 to 3 lower alkyl groups, lower alkoxyl groups, halogen atoms or the like.

Illustrative of the saturated or unsaturated, linear or branched hydrocarbon groups represented by R¹, R² and R³ are linear or branched alkyl or alkenyl groups having 1 to 24 carbon atoms, with those containing 1 to 20 carbon atoms being more preferred (these alkenyl groups may optionally contain plural unsaturated bonds). Examples of such alkyl groups can include methyl, ethyl, n-propyl, isopropyl, n-butyl, isobutyl, sec-butyl, tert-butyl, n-pentyl, n-hexyl, octyl, decyl, dodecyl, tetradecyl, hexadecyl, and octadecyl. On the other hand, examples of such alkenyl groups can include vinyl, allyl, geranyl and farnesyl.

Illustrative of the alkoxyalkyl group represented by R³ are C₁₋₆alkoxyC₁₋₆alkyl groups, including methoxymethyl, ethoxyethyl, methoxyethyl and the like, for example. Illustrative of the aryloxyalkyl group represented by R³ are phenyloxyC₁₋₆alkyl groups, including phenyloxymethyl, phenyloxyethyl and the like, for example.

Examples of halogen atoms represented by R⁴ and substitutable on aromatic groups or the like can include chlorine, bromine, fluorine and iodine atoms. Illustrative of the lower alkyl group are linear or branched alkyl groups having 1 to 6 carbon atoms, specifically methyl, ethyl, n-propyl, isopropyl, n-butyl, isobutyl, sec-butyl, n-pentyl, n-hexyl and the like. Illustrative of the lower alkoxyl group are linear or branched alkoxyl groups having 1 to 6 carbon atoms, specifically methoxyl, ethoxyl, isopropoxyl, n-propoxyl, n-butoxyl and the like. Further, Illustrative of the halogeno(lower alkyl) group are alkyl groups having 1 to 6 carbon atoms and substituted by 1 to 3 carbon atoms, specifically trifluoromethyl, chloroethyl and the like.

Where R⁵ is a hydrogen atom and R⁶ is a substituted or unsubstituted phenyl group, -N(R⁵)R⁶ can preferably be those represented by the following formula (a): 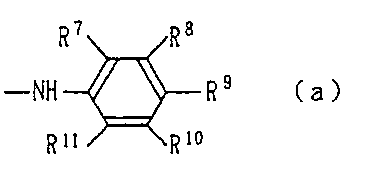 wherein R⁷, R⁸, R⁹, R¹⁰ and R¹¹ may be the same or different and each independently represent a hydrogen atom, a halogen atom, a lower alkyl group, a lower alkoxyl group, a phenyl group, an aralkyl group, a halogeno(lower alkyl) group, a lower alkanoyl group, a nitro group, a substituted amino group, an aryloxy group, or an aryloxyaryl group.

Examples of the halogen atom, lower alkyl group, lower alkoxyl group, aralkyl group and halogeno(lower alkyl) group can be those described above in connection with R¹ to R⁶. Further, illustrative of the substituted amino group are C₁₋₆alkylamino groups, di(C₁₋₆alkyl) amino groups and the like, specifically methylamino, ethylamino, N,N-dimethylamino, N,N-diethylamino, N,N-diisopropylamino and the like. Examples of the aryloxy group can include phenoxy, naphthyloxy and the like, and examples of the aryloxyaryl group can include phenoxyphenyl and the like. Furthermore, illustrative of the lower alkanoyl group are alkanoyl groups having 1 to 6 carbon atoms, specifically acetyl, propionyl, butylyl and the like.

Illustrative of the piperazine ring which R⁵ and R⁶ together form in combination with the adjacent nitrogen atom are piperazinyl groups substituted by substituted or unsubstituted heterocyclic groups and piperazinyl groups substituted by substituted or unsubstituted phenyl groups. Of these, the piperazinyl groups substituted by substituted or unsubstituted heterocyclic groups can be piperazinyl groups substituted by 5- or 6-membered aromatic heterocyclic groups having 1 or 2 nitrogen atoms as heteroatoms. Specific examples can include pyridylpiperazinyl, pyrimidinylpiperazinyl, imidazolylpiperazinyl, pyrrolylpiperazinyl and the like.

As examples of the piperazinyl groups substituted by substituted or unsubstituted phenyl groups, on the other hand, those represented by the following formula (b) are preferred. 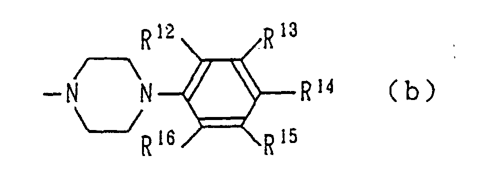 wherein R¹², R¹³, R¹⁴, R¹⁵ and R¹⁶ may be the same or different and each independently represent a hydrogen atom, a halogen atom, a lower alkyl group, a lower alkoxyl group, a phenyl group, an aralkyl group, an aralkyloxy group, a halogeno(lower alkyl) group, a lower alkanoyl group, or a nitro group.

Examples of the halogen atom, lower alkyl group, lower alkoxyl group, aralkyl group, halogeno(lower alkyl) group and lower alkanoyl group can be those described above in connection with R¹ to R⁶. Further, illustrative of the aralkyloxy group are phenyl(C₁₋₆alkyloxy) groups, specifically benzyloxy, phenylethyloxy and the like.

Groups substitutable on the anilinocarbonylmethyl group in R⁶ are those substitutable on the benzene ring of an anilino group, and their examples can include 1 to 3 lower alkyl groups (preferably C₁₋₆alkyl groups), lower alkoxyl groups (preferably C₁₋₆alkoxyl groups), halogen atoms and the like.

R⁵ and R⁶ may also form a piperazinyl group together with the adjacent nitrogen atom.

The compound according to the present invention may include cis-trans isomers and isomers based on an asymmetric carbon atom. The present invention shall include individual isomers and mixtures. Furthermore, each compound according to the present invention shall include its solvates typified by its hydrates.

Among the compounds according to the present invention, the compounds represented by the formula (1) can be prepared, for example, in accordance with the following, reaction formula: 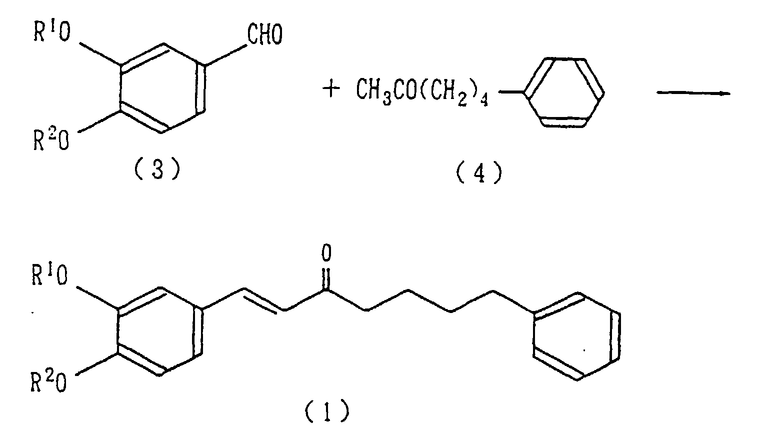 wherein R¹ and R² have the same meanings as defined above.

Namely, each compound (1) is obtained by reacting a benzaldehyde (3) and phenylhexanone (4) in the presence of a base.

The reaction is conducted, for example, by dissolving the benzaldehyde (3) and phenylhexanone (4) in a suitable solvent such as ethanol and then causing a base such as potassium hydroxide to act at room temperature or under heating.

The compounds represented by the formula (2) can be prepared, for example, in accordance with the following reaction formula: 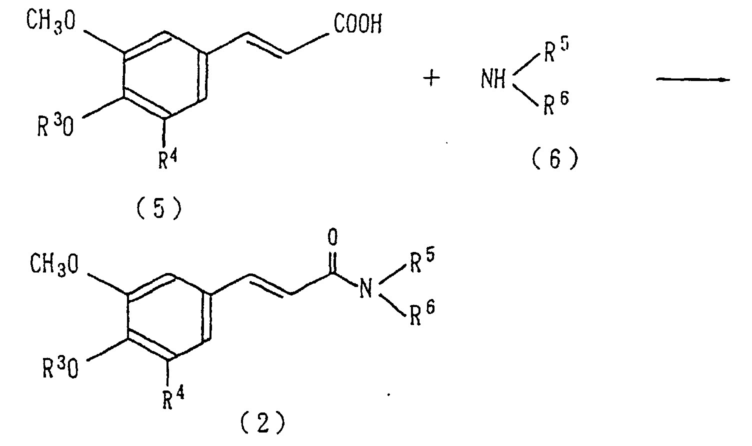 wherein R³, R⁴, R⁵ and R⁶ have the same meanings as defined above.

Namely, each compound (2) can be obtained by condensing an amine (6) with a cinnamic acid (5).

It is preferable to conduct the reaction in a similar manner as in usual acid amidation reactions, for example, in the presence of a carbodiimide. Incidentally, when the resulting compound (2) contains a hydroxyl group, it can be converted into an alkoxyl-containing compound by further alkylating it in a conventional manner.

As will be demonstrated in Tests 1-3 subsequently herein, these compounds represented by the formula (1) or (2) show excellent ACAT inhibitory activity and have inhibitory effects on the accumulation of cholesterol esters in cells, blood cholesterol lowering effects and fatty liver inhibiting effects, so that they are useful as blood cholesterol lowering agents, fatty liver inhibiting agents and prophylactic and therapeutic agents for arteriosclerosis, that is, as anti-hyperlipemic medicines.

As medicines according to the present invention, the compounds represented by the formula (1) or (2) may be administered orally or parenterally, for example, by injection either singly or along with another active ingredient (for example, an anti-arteriosclerotic medicine having a different mechanism of action) and further, together with a pharmaceutically-acceptable carrier as needed, with oral administration being preferred.

Suitable examples of pharmaceutical carriers or diluents, which are usable in combination with the compounds of the formula (1) or (2) in the medicines according to the present invention, can include glucose; saccharose; lactose; ethyl alcohol; glycerin; mannitol; sorbitol; pentaerythritol; diethylene glycol; triethylene glycol; ethylene glycol; propylene glycol; dipropylene glycol; polyethylene glycol 400; other polyethylene glycols; mono-, di- and triglycerides of saturated fatty acids, such as glyceryl trilaurate, glyceryl monostearate and glyceryl distearate; pectin; starch; corn starch; alginic acid; xylose; talc; lycopodium; oils and fats, such as olive oil, peanut oil, castor oil, corn oil, wheat malt oil, sesame oil, cotton seed oil, sunflower oil and cod liver oil; the magnesium or calcium salts of fatty acid having 12 to 22 carbon atoms, such as calcium stearate, calcium laurate, magnesium oleate, calcium palmitate, calcium behenate and magnesium stearate; cyclodextrins, for example, α-cyclodextrin, γ-cyclodextrin, hydroxyethyl-β-cyclodextrin, hydroxypropyl-β-cyclodextrin, hydroxypropyl-β-cyclodextrin, dihydroxypropyl-β-cyclodextrin, carboxymethylethyl-β-cyclodextrin and dimethyl-β-cyclodextrin; emulsifiers, for example, esters between saturated or unsaturated fatty acids having 2 to 22 carbon atoms, especially 1 to 18 carbon atoms and monohydric aliphatic alcohols (for example, those having 1 to 20 carbon atoms, such as alkanols) or polyhydric alcohols, such as glycol, glycerin, diethylene glycol, pentaerythritol ethyl alcohol, butyl alcohol and octadecyl alcohol; silicones such as dimethylpolysiloxane; and pyrogen-free distilled water.

Illustrative oral preparations can include tablets, capsules, granules, powders, troches, syrups and the like. Solid compositions - such as tablets, capsules, granules, powders and troches - may contain excipients such as starch, lactose, carboxymethylcellulose and precipitated calcium carbonate; binders such as gum arabic, tragacanth gum, gelatin, methylcellulose and carboxymethylcellulose; disintegrators such as alginic acid and corn starch; lubricants such as magnesium stearate, hydrated silicon dioxide and light anhydrous silicic acid; sweetenings such as saccharose; and flavors such as menthol. Liquid compositions such as syrups may contain - in addition to sorbitol, gelatin, methylcellulose, vegetable oils and emulsifiers - sweetenings, flavors, coloring matters and the like.

Desirably, each of these preparations contains 1 to 95 wt.% of one or more of the above-described compounds (1) and (2).

The dosage of each medicine according to the present invention varies depending on the severity of a symptom, the age and body weight of a patient, the administration route and the like. In the case of an oral administration, however, it is generally preferred to administer, in several portions, 0.2 to 150 mg in terms of the effective ingredient compound per adult, kilogram of body weight and day.

### Examples

The present invention will next be described in further detail by Examples. It should however be borne in mind that the present invention is by no means limited to these Examples.

### Compound 1: Synthesis of 1-(4-benzyloxy-3-methoxyphenyl)-7-phenylhepto-1-en-3-one

1-Phenyl-5-hexanone (2.64 g) was dissolved in ethanol (100 mℓ), followed by the addition of 10% potassium hydroxide (10 mℓ). The mixture was stirred for 1 hour. 4-Benzyloxy-3-methoxybenzaldehyde (3.63 g) was added further, and a reaction was then conducted overnight at room temperature. Precipitated crystals were collected by filtration and then recrystallized from chloroform-ethanol, whereby white crystalline Compound 1 (4.69 g) was obtained. The data of ¹H-NMR, mass spectrum, infrared absorption spectrum and melting point of this compound are as follows:
¹H-NMR (400MHz,CDCℓ₃) δ: 1.60-1.76(4H,m), 2.62(4H,t), 3.86(3H,s), 5.13(2H,s), 6.58(1H,d,J=16.1Hz), 6.83(1H,d,J=8.3Hz), 7.01(1H,dd,J=8.3,2.0Hz), 7.05(1H,d,J=2.0Hz), 7.10-7.42(10H,m), 7.45(1H,d,J=16.1Hz).
MS (EI) m/z: 400(M+), 309, 118, 92.
IR (KBr) νₘₐₓ cm⁻¹: 1635.
Melting point °C: 85.7-86.4.

### Compound 2: 1-(3-benzyloxy-4-methoxyphenyl)-7-phenylhepto-1-en-3-one

1-Phenyl-5-hexanone (5.29 g) was dissolved in ethanol (100 mℓ), followed by the addition of 10% potassium hydroxide (10 mℓ) and potassium (10 mℓ). The mixture was stirred for 1 hour. 3-Benzyloxy-4-methoxybenzaldehyde (6.18 g) was added further, and a reaction was then conducted overnight at room temperature. Precipitated crystals were collected by filtration and then recrystallized from chloroform-ethanol, whereby slightly yellowish crystalline Compound 2 (5.52 g) was obtained. The data of ¹H-NMR, mass spectrum, infrared absorption spectrum and melting point of this compound are as follows:
¹H-NNR (400MHz,CDCℓ₃) δ: 1.58-1.74(4H,m), 2.60(4H,m), 3.82(3H,s), 5.10(2H,s), 6.52(1H,d,J=16.1Hz), 6.81(1H,d,J=8.8Hz), 7.05-7.46(13H,m).
MS (EI) m/z: 400(M+), 309, 117, 92.
IR (KBr) νₘₐₓ cm⁻¹: 1675.
Melting point °C: 116.0-118.6.

### Compound 3: 1- (3,4-dibenzyloxyphenyl)-7-phenylhepto-1-en-3-one

1-Phenyl-5-hexanone (5.29 g) was dissolved in ethanol (100 mℓ), followed by the addition of 10% potassium hydroxide (10 mℓ). The mixture was stirred for 1 hour. 3,4-Dibenzyloxybenzaldehyde (9.55 g) was added further, and a reaction was then conducted overnight at room temperature. Precipitated crystals were collected by filtration and then recrystallized from chloroform-ethanol, whereby slightly yellowish crystalline compound 3 (4.69 g) was obtained.
¹H-NMR (400MHz,CDCℓ₃) δ: 1.58-1.74(4H,m), 2.60(4H,q), 5.12(43,s), 6.52(1H,d,J=16.1Hz), 6.86(1H,d,J=8.3Hz), 7.03(1H,dd,J=8.3,2.0Hz), 7.11(1H,d,J=2.0Hz), 7.12-7.45(16H,m).
MS (EI) m/z: 476(M+), 385, 117, 92.
IR (KBr) νₘₐₓ cm⁻¹: 1665.
Melting point °C: 95.8-96.3.

### Compound 4: N-[4-(3,4-dimethylphenyl)-1,4-diazacyclohexyl]-(2E)-3-(3,5-dimethoxy-4-hexyloxyphenyl)-2-propenamide

N-[4-(3,4-Dimethylphenyl)-1,4-diazacyclohexyl]-(2E)-3-(3,5-dimethoxy-4-hydroxyphenyl)-2-propenamide (3.00 g) was dissolved in anhydrous dimethylformamide (300 mℓ), followed by the addition of sodium hydride (394 mg) while paying attention to exothermic heat. After production of gas subsided, 1-bromohexane (1.62 g) was added, followed by a reaction with stirring for 7 days at room temperature. After completion of the reaction, the reaction mixture was poured into a great deal of water and subsequent to adjustment to pH 6 with 1 N hydrochloric acid, was allowed to stand overnight. The reaction mixture was filtered with Celite, and the Celite was washed with water and then dried in air. The Celite was subjected to extraction in dichloromethane, and the dichloromethane layer was concentrated to dryness under reduced pressure. By recrystallization from ethanol, pale yellow crystalline Compound 4 (1.18 g) was obtained. Incidentally, the propenamide derivative, the raw material, was obtained in a good yield by treating 3,5-dimethoxy-4-hydroxycinnamic acid and 1-(3,4-dimethylphenyl)piperazine with 1-ethyl-3-(3-dimethylaminopropyl)carbodiimide hydrochloride in DMF. The data of ¹H-NMR, mass spectrum, infrared absorption spectrum and melting point of this compound are as follows:
¹H-NMR (400MHZ,CDCℓ₃) δ: 0.90(3H,t,), 1.30-1.50(6H,m), 1.72-1.80(2H,m), 2.19(3H,s), 2.24(3H,s), 3.16(4H,t), 3.86(4H,brs), 3.88(6H,s), 4.00(2H,t), 6.70(1H,dd,J=8.0,2.4Hz), 6.75(2H,s), 6.77(1H,d,J=2.4Hz), 6.80(1H,d,J=15.4Hz), 7.04(1H,d,J=8.0Hz), 7.62(1H,d,J=15.4Hz).
MS (EI) m/z: 480(M+), 291, 207, 160.
IR (KBr) νₘₐₓ cm⁻¹: 1640.
Melting point °C: 91.5-92.8.

### Compound 5: N-[4-(3,4-dimethylphenyl)-1,4-diazacyclohexyl]-(2E)-3-(3,5-dimethoxy-4-heptyloxyphenyl)-2-propenamide

N-[4-(3,4-Dimethylphenyl)-1,4-diazacyclohexyl]-(2E)-3-(3,5-dimethoxy-4-hydroxyphenyl)-2-propenamide (3.00 g) was dissolved in anhydrous dimethylformamide (300 mℓ), followed by the addition of sodium hydride (394 mg) while paying attention to exothermic heat. After production of gas subsided, 1-bromohexane (1.76 g) was added, followed by a reaction with stirring for 7 days at room temperature. After completion of the reaction, the reaction mixture was poured into a great deal of water and subsequent to adjustment to pH 6 with 1 N hydrochloric acid, was allowed to stand overnight. The reaction mixture was filtered with Celite, and the Celite was thoroughly washed with water and then dried in air. The Celite was subjected to extraction in dichloromethane, and the dichloromethane layer was concentrated to dryness under reduced pressure. By recrystallization from ethanol, pale yellow crystalline compound 5 (0.80 g) was obtained. The data of ¹H-NMR, mass spectrum, infrared absorption spectrum and melting point of this compound are as follows:
¹H-NMR (400MHz,CDCℓ₃) δ: 0.89(3H,t), 1.26-1.48(8H,m), 1.72-1.80(2H,m), 2.19(3H,s), 2.24(3H,s), 3.16(4H,t), 3.85(4H,brs), 3.88(6H,s), 3.99(2H,t), 6.70(1H,dd,J=8.0,2.4Hz), 6.75(2H,s), 6.77(1H,d,J=2.4Hz), 6.80(1H,d,J=15.4Hz), 7.04(1H,d,J=8.0Hz), 7.62(1H,d,J=15.4Hz).
MS (EI) m/z: 494(M+), 305, 208, 161.
IR (KBr) νₘₐₓ cm⁻¹: 1640.
Melting point °C: 91.5-103.7.

### Compound 6: N-[4-(3,4-dimethylphenyl)-1,4-diazacyclohexyl]-(2E)-3-(3,5-dimethoxy-4-octyloxyphenyl)-2-propenamide

N-[4-(3,4-Dimethylphenyl)-1,4-diazacyclohexyl]-(2E)-3-(3,5-dimethoxy-4-hydroxyphenyl)-2-propenamide (3.00 g) was dissolved in anhydrous dimethylformamide (300 mℓ), followed by the addition of sodium hydride (394 mg) while paying attention to exothermic heat. After production of gas subsided, 1-bromohexane (1.90 g) was added, followed by a reaction with stirring for 7 days at room temperature. After completion of the reaction, the reaction mixture was poured into a great deal of water and subsequent to adjustment to pH 6 with 1 N hydrochloric acid, was allowed to stand overnight. The reaction mixture was filtered with Celite, and the Celite was thoroughly washed with water and then dried in air. The Celite was subjected to extraction in dichloromethane, and the dichloromethane layer was concentrated to dryness under reduced pressure. By recrystallization from ethanol, pale yellow crystalline Compound 6 (2.22 g) was obtained. The data of ¹H-NMR, mass spectrum, infrared absorption spectrum and melting point of this compound are as follows:
¹H-NMR (400MHz,CDCℓ₃) δ: 0.88(3H,t), 1.28-1.50 (10H,m), 1.72-1.80(2H,m), 2.19(3H,s), 2.24(3H,s), 3.16(4H,t), 3.86(4H,brs), 3.88(6H,s), 3.99(2H,t), 6.70(1H,dd,J=8.0,2.4Hz), 6.75(2H,s) 6.77(1H,d,J=2.4Hz), 6.79(1H,d,J=15.4Hz), 7.05(1H,d,J=8.0Hz), 7.62(1H,d,J=15.4Hz).
MS (EI) m/z: 508(M+), 319, 208, 161.
IR (KBr) νₘₐₓ cm⁻¹: 1640.
Melting point °C: 103.6-105.5.

### Compound 7: N-[4-(3,4-dimethylphenyl)-1,4-diazacyclohexyl ]-(2E)-3-(3,5-dimethoxy-4-nonylphenyl)-2-propenamide

3,5-Dimethoxy-4-nonyloxysuccinic acid (5.53 g) was dissolved in ethyl acetate (800 mℓ), followed by the addition of 1-ethyl-3-(3-dimethylaminopropyl)-carbodiimide hydrochloride (3.02 g) and further of 1-(3,4-dimethylphenyl)piperazine (3.00 g). A reaction was conducted with stirring at room temperature. The reaction mixture was fractionated by a silica gel column and target fractions were obtained. After the target fractions were concentrated to dryness under reduced pressure, the residue was recrystallized from ethanol, whereby slightly red crystalline Compound 7 (2.20 g) was obtained. The data of ¹H-NMR, mass spectrum, infrared absorption spectrum and melting point of this compound are as follows:
¹H-NMR (400MHz,CDCℓ₃) δ: 0.88(3H,t), 1.23-1.48(12H,m), 1.70-1.78(2H,m), 2.20(3H,s), 2.24(2H,s), 3.17(4H,t), 3.86(4H,brs), 3.88(6H,s), 3.99(2H,t), 6.70(1H,dd,J=8.0,2.4Hz), 6.75(2H,s), 6.77(1H,d,J=2.4Hz), 6.79(1H,d,J=15.4Hz), 7.05(1H,d,J=8.0Hz), 7.62(1H,d,J=15.4Hz).
MS (EI) m/z: 522(M+), 333, 208, 161.
IR (KBr) νₘₐₓ cm⁻¹: 1640.
Melting point °C: 91.7-100.4.

### Compound 8: N-[4- (3,4-dimethylphenyl)-1,4-diazacyclohexyl]-(2E)-3-(3,5-dimethoxy-4-decyloxyphenyl)-2-propenamide

3,5-Dimethoxy-4-decyloxysuccinic acid (5.75 g) was dissolved in ethyl acetate (800 mℓ), followed by the addition of 1-ethyl-3-(3-dimethylaminopropyl)-carbodiimide hydrochloride (3.02 g) and further of 1-(3,4-dimethylphenyl)piperazine (3.00 g). A reaction was conducted with stirring at room temperature. The reaction mixture was fractionated by a silica gel column and target fractions were obtained. After the target fractions were concentrated to dryness under reduced pressure, the residue was recrystallized from ethanol, whereby white crystalline Compound 8 (6.66 g) was obtained. The data of ¹H-NMR, mass spectrum, infrared absorption spectrum and melting point of this compound are as follows:
¹H-NMR (400MHz,CDCℓ₃) δ: 0.88(3H,t), 1.22-1.48(14H,m), 1.70-1.78(2H,m), 2.19(3H,s), 2.24(3H,s), 3.15(4H,t), 3.85(4H,brs), 3.87(6H,s), 3.99(2H,t), 6.69(1H,dd,J=8.0,2.4Hz), 6.75(2H,s), 6.77(1H,d,J=2.4Hz), 6.81(1H,d,J=15.4Hz), 7.04(1H,d,J=8.0Hz), 7.62(1H,d,J=15.4Hz).
MS (EI) m/z: 536(M+), 347, 208, 161.
IR (KBr) νₘₐₓ cm⁻¹: 1640.
Melting point °C: 100.2-100.8.

### Compound 9: N-[4-(3,4-dimethylphenyl)-1,4-diazacyclohexyl]-(2E)-3-(3,5-dimethoxy-4-undecyloxyphenyl)-2-propenamide

N-[4-(3,4-Dimethylphenyl)-1,4-diazacyclohexyl]-(2E)-3-(3,5-dimethoxy-4-hydroxyphenyl)-2-propenamide (3.00 g) was dissolved in anhydrous dimethylformamide (300 mℓ), followed by the addition of sodium hydride (394 mg) while paying attention to exothermic heat. After production of gas subsided, 1-bromoundecane (2.31 g) was added, followed by a reaction with stirring for 7 days at room temperature. After completion of the reaction, the reaction mixture was poured into a great deal of water and subsequent to adjustment to pH 6 with 1 N hydrochloric acid, was allowed to stand overnight. The reaction mixture was filtered with Celite, and the Celite was thoroughly washed with water and then dried in air. The Celite was subjected to extraction in dichloromethane, and the dichloromethane layer was concentrated to dryness under reduced pressure. By recrystallization from ethanol, slightly yellow crystalline Compound 9 (1.26 g) was obtained. The data of ¹H-NMR, mass spectrum, infrared absorption spectrum and melting point of this compound are as follows:
¹H-NMR (400MHz,CDCℓ₃) δ: 0.86(3H,t), 1.22-1.47(16H,m), 1.69-1.77(2H,m), 2.17(3H,s), 2.22(3H,s), 3.14(4H,t), 3.83(4H,brs), 3.86(6H,s), 3.97(2H,t), 6.68(1H,dd,J=8.0,2.4Hz), 6.73(2H,s), 6.75(1H,d,J=2.4Hz), 6.77(1H,d,J=15.4Hz), 7.03(1H,d,J=8.0Hz), 7.60(1H,d,J=15.4Hz).
MS (EI) m/z: 550(M+), 361, 208, 161.
IR (KBr) νₘₐₓ cm⁻¹: 1640.
Melting point °C: 94.0-99.7.

### Compound 10: N-[4-(3,4-dimethylphenyl)-1,4-diazacyclohexyl]-(2E)-3-(3,5-dimethoxy-4-dodecyloxyphenyl)-2-propenamide

N-[4-(3,4-Dimethylphenyl)-1,4-diazacyclohexyl]-(2E)-3-(3,5-dimethoxy-4-hydroxyphenyl)-2-propenamide (3.00 g) was dissolved in anhydrous dimethylformamide (300 mℓ), followed by the addition of sodium hydride (394 mg) while paying attention to exothermic heat. After production of gas subsided, 1-bromododecane (2.45 g) was added, followed by a reaction with stirring for 7 days at room temperature. After completion of the reaction, the reaction mixture was poured into a great deal of water and subsequent to adjustment to pH 6 with 1 N hydrochloric acid, was allowed to stand overnight. The reaction mixture was filtered with Celite, and the Celite was thoroughly washed with water and then dried in air. The Celite was subjected to extraction in dichloromethane, and the dichloromethane layer was concentrated to dryness under reduced pressure. By recrystallization from ethanol, slightly yellow crystalline Compound 10 (1.26 g) was obtained. The data of ¹H-NMR, mass spectrum, infrared absorption spectrum and melting point of this compound are as follows:
¹H-NMR (400MHz,CDCℓ₃) δ: 0.88(3H,t), 1.23-1.48(18H,m), 1.72-1.78(2H,m), 2.19(3H,s), 2.24(3H,s), 3.16(4H,t), 3.84(4H,brs), 3.88(6H,s), 3.99(2H,t), 6.70(1H,dd,J=8.0, 2.4Hz), 6.75(2H,s), 6.77(1H,d,J=2.4Hz), 6.79(1H,d,J=15.4Hz), 7.04(1H,d,J=8.0Hz), 7.62(1H,d,J=15.4Hz).
MS (EI) m/z: 564(M+), 375, 203, 161.
IR (KBr) νₘₐₓ cm⁻¹: 1640.
Melting point °C: 83.7-99.7.

### Compound 11: N-[4-(3,4-dimethylphenyl)-1,4-diazacyclohexyl]-(2E)-(3,5-dimethoxy-4-benzyloxyphenyl)-2-propenamide

4-Benzyloxy-3,5-dimethoxysuccinic acid (0.99 g) was dissolved in chloroform (50 mℓ), followed by the addition of 1-ethyl-3-(3-dimethylaminopropyl)-carbodiimide hydrochloride (600 mg) and further of 1-(3,4-dimethylphenyl)piperazine (0.95 g). A reaction was conducted with stirring at room temperature. The reaction mixture was fractionated through a silica gel column and target fractions were obtained. After the target fractions were concentrated to dryness under reduced pressure, the residue was recrystallized from ethanol, whereby slightly yellow crystalline Compound 11 (1.33 g) was obtained. The data of ¹H-NMR, mass spectrum, infrared absorption spectrum and melting point of this compound are as follows:
¹H-NMR (400MHZ,CDCℓ₃) δ: 2.19(3H,s), 2.23(3H,s), 3.14(4H,t), 3.82(4H,brs), 3.84(6H,s), 5.04(2H,s), 3.68(1H,dd,J=8.0,2.4Hz), 6.74(2H,s), 6.75(1H,d,J=2.4Hz), 6.80(1H,d,J=15.4Hz), 7.04(1H,d,J=8.0Hz), 7.22-7.50(5H,m), 7.61(1H,d,J=15.4Hz).
MS (EI) m/z: 486(M+), 395, 190, 161.
IR (KBr) νₘₐₓ cm⁻¹: 1645.
Melting point °C: 99.2-99.7.

### Compound 12: N-[4-(2,4-dimethylphenyl)-1,4-diazacyclohexyl]-(2E)-3-(3,5-dimethoxy-4-propyloxyphenyl)-2-propenamide

N-[4-(2,4-Dimethylphenyl)-1,4-diazacyclohexyl)-(2E)-3-(3, 5-dimethoxy-4-hydroxyphenyl)-2-propenamide (5.00 g) was dissolved in anhydrous dimethylformamide (400), followed by the addition of sodium hydride (656 mg) while paying attention to exothermic heat. After production of gas subsided, 1-bromopropane (2.02 g) was added, followed by a reaction with stirring for 7 days at room temperature. After completion of the reaction, the reaction mixture was poured into a great deal of water and subsequent to adjustment to pH 6 with 1 N hydrochloric acid, was allowed to stand overnight. The reaction mixture was filtered with Celite, and the Celite was thoroughly washed with water and then dried in air. The Celite was subjected to extraction in dichloromethane, and the dichloromethane layer was concentrated to dryness under reduced pressure. By recrystallization from ethanol, slightly yellow crystalline Compound 12 (3.63 g) was obtained. The data of ¹H-NMR, mass spectrum, infrared absorption spectrum and melting point of this compound are as follows:
¹H-NMR (400MHz,CDCℓ₃) δ: 1.01(3H,t), 1.73-1.82(2H,m), 2.28(3H,s), 2.31(3H,s), 2.91(4H,brs), 3.81(4H,brs), 3.88(6H,s), 3.97(2H,t), 6.76(2H,s), 6.82(1H,d,J=15.3Hz), 6.89(1H,d,J=8.0Hz), 6.97(1H,dd,J=8.0,2.1Hz), 7.02(1H,d,J=2.1Hz), 7.62(1H,d,J=15.3Hz).
MS (EI) m/z: 438(M+), 239, 190, 161.
IR (KBr) νₘₐₓ cm⁻¹: 1635.
Melting point °C: 92.5-101.4.

### Compound 13: N-[4-(2,4-dimethylphenyl)-1,4-dimethylphenyl)-1,4-diazacyclohexyl]-(2E)-3-(3,5-dimethoxy-4-hexyloxyphenyl)-2-propenamide

N-[4-(2,4-Dimethylphenyl)-1,4-diazacyclohexyl]-(2E)-3-(3,5-dimethoxy-4-hydroxyphenyl)-2-propenamide (5.00 g) was dissolved in anhydrous dimethylformamide (400 mℓ), followed by the addition of sodium hydride (656 mg) while paying attention to exothermic heat. After production of gas subsided, 1-bromohexane (2.71 g) was added, followed by a reaction with stirring for 7 days at room temperature. After completion of the reaction, the reaction mixture was poured into a great deal of water and subsequent to adjustment to pH 6 with 1 N hydrochloric acid, was allowed to stand overnight. The reaction mixture was filtered with Celite, and the Celite was thoroughly washed with water and then dried in air. The Celite was subjected to extraction in dichloromethane, and the dichloromethane layer was concentrated to dryness under reduced pressure. By recrystallization from ethanol, slightly yellow crystalline Compound 13 (4.30 g) was obtained. The data of ¹H-NMR, mass spectrum, infrared absorption spectrum and melting point of this compound are as follows:
¹H-NMR (400MHz,CDCℓ₃) δ: 0.90(3H,t), 1.30-1.50(6H,m), 1.70-1.78(2H,m), 2.26(3H,s), 2.29(3H,s), 2.88(4H,brs), 3.81(4H,brs), 3.86(6H,s), 3.99(2H,t), 6.76(2H,s), 6.85(1H,d,J=15.4Hz), 6.87(1H,d,J=8.0Hz), 6.96(1H,dd,J=8.0,2.1Hz), 7.01(1H,d,J=2.1Hz), 7.62(1H,d,J=15.4Hz).
MS (EI) m/z: 480(M+), 255, 190, 161.
IR (KBr) νₘₐₓ cm⁻¹: 1635.
Melting point °C: 101.0-114.3.

### Compound 14: N-[4-(2,4-dimethylphenyl)-1,4-diazacyclohexyl]-(2E)-3-(3,5-dimethoxy-4-heptyloxyphenyl)-2-propenamide

N-[4-(2,4-Dimethylphenyl)-1,4-diazacyclohexyl]-(2E)-3-(3,5-dimethoxy-4-hydroxyphenyl)-2-propenamide (5.00 g) was dissolved in anhydrous dimethylformamide (400 mℓ), followed by the addition of sodium hydride (656 mg) while paying attention to exothermic heat. After production of gas subsided, 1-bromoheptane (2.94 g) was added, followed by a reaction with stirring for 7 days at room temperature. After completion of the reaction, the reaction mixture was poured into a great deal of water and subsequent to adjustment to pH 6 with 1 N hydrochloric acid, was allowed to stand overnight. The reaction mixture was filtered with Celite, and the Celite was thoroughly washed with water and then dried in air. The Celite was subjected to extraction in dichloromethane, and the dichloromethane layer was concentrated to dryness under reduced pressure. By recrystallization from ethanol, slightly yellow crystalline Compound 14 (2.40 g) was obtained. The data of ¹H-NMR, mass spectrum, infrared absorption spectrum and melting point of this compound are as follows:
¹H-NMR (400MHz,CDCℓ₃) δ: 0.89(3H,t), 1.27-1.48(8H,m), 1.72-1.80(2H,m), 2.28(3H,s), 2.31(3H,s), 2.91(4H,brs), 3.81(4H,brs), 3.88(6H,s), 3.99(2H,t), 6.76(2H,s), 6.82(1H,d,J=15.4Hz), 6.89(1H,d,J=8.0Hz), 6.98(1H,dd,J=8.0,2.1Hz), 7.02(1H,d,J=2.1Hz), 7.62(1H,d,J=15.4Hz).
MS (EI) m/z: 494(M+), 305, 190, 161.
IR (KBr) νₘₐₓ cm⁻¹: 1635.
Melting point °C: 88.1-89.4.

### Compound 15: N-[4-(2,4-dimethylphenyl)-1,4-diazacyclohexyl]-(2E)-3-(3,5-dimethoxy-4-octyloxyphenyl)-2-propenamide

N-[4-(2,4-Dimethylphenyl)-1,4-diazacyclohexyl]-(2E)-3-(3,5-dimethoxy-4-hydroxyphenyl)-2-propenamide (4.00 g) was dissolved in anhydrous dimethylformamide (400 mℓ), followed by the addition of sodium hydride (656 mg) while paying attention to exothermic heat. After production of gas subsided, 1-bromooctane (2.53 g) was added, followed by a reaction with stirring for 7 days at room temperature. After completion of the reaction, the reaction mixture was poured into a great deal of water and subsequent to adjustment to pH 6 with 1 N hydrochloric acid, was allowed to stand overnight. The reaction mixture was filtered with Celite, and the Celite was thoroughly washed with water and then dried in air. The Celite was subjected to extraction in dichloromethane, and the dichloromethane layer was concentrated to dryness under reduced pressure. By recrystallization from ethanol, slightly yellow crystalline Compound 15 (4.13 g) was obtained. The data of ¹H-NMR, mass spectrum, infrared absorption spectrum and melting point of this compound are as follows:
¹H-NMR (400MHz,CDCℓ₃) δ: 0.88(3H,t), 1.26-1.48(10H,m), 1.72-1.80(2H,m), 2.27(3H,s), 2.30(3H,s), 2.89(4H,brs), 3.81(4H,brs), 3.87(6H,s), 3.99(2H,t), 6.76(2H,s), 6.83(1H,d,J=15.4Hz), 6.88(1H,d,J=8.0Hz), 6.97(1H,dd,J=8.0,2.1Hz), 7.01(1H,d,J=2.1Hz), 7.62(1H,d,J=15.4Hz).
MS (EI) m/z: 508(M+), 319, 208, 161.
IR (KBr) νₘₐₓ cm⁻¹: 1635.
Melting point °C: 88.7-108.7.

### Compound 16: N-[4-(2,4-dimethylphenyl)-1,4-diazacyclohexyl]-(2E)-3-(3,5-dimethoxy-4-nonyloxyphenyl)-2-propenamide

N-[4-(2,4-Dimethylphenyl)-1,4-diazacyclohexyl]-(2E)-3-(3,5-dimethoxy-4-hydroxyphenyl)-2-propenamide (5.00 g) was dissolved in anhydrous dimethylformamide (400 mℓ), followed by the addition of sodium hydride (656 mg) while paying attention to exothermic heat. After production of gas subsided, 1-bromononane (3.40 g) was added, followed by a reaction with stirring for 7 days at room temperature. After completion of the reaction, the reaction mixture was poured into a great deal of water and subsequent to adjustment to pH 6 with 1 N hydrochloric acid, was allowed to stand overnight. The reaction mixture was filtered with Celite, and the Celite was thoroughly washed with water and then dried in air. The Celite was subjected to extraction in dichloromethane, and the dichloromethane layer was concentrated to dryness under reduced pressure. By recrystallization from ethanol, slightly yellow crystalline Compound 16 (4.64 g) was obtained. The data of ¹H-NMR, mass spectrum, infrared absorption spectrum and melting point of this compound are as follows:
¹H-NMR (400MHz,CDCℓ₃) δ: 0.88(3H,t), 1.23-1.47(12H,m), 1.72-1.78(2H,m), 2.28(3H,s), 2.31(3H,s), 2.91(4H,brs), 3.82(4H,brs), 3.88(6H,s), 3.99(2H,t), 6.75(2H,s), 6.80(1H,d,J=15.4Hz), 6.90(1H,d,J=8.0Hz), 6.98(1H,dd,J=8.0,2.1Hz), 7.03(1H,d,J=15.4Hz), 6.90(1H,d,J=8.0Hz), 6.98(1H,dd,J=8.0,2.1Hz), 7.03(1H,d,J=2.1Hz), 7. 62(1H,d,J=15.4Hz).
MS (EI) m/z: 522(M+), 333, 190, 161.
IR (KBr) νₘₐₓ cm⁻¹: 1635.
Melting point °C: 89.5-101.3.

### Compound 17: N-[4-(2,4-dimethylphenyl)-1,4-diazacyclohexyl]-(2E)-3-(3,5-dimethoxy-4-decyloxyphenyl)-2-propenamide

N-[4-(2,4-Dimethylphenyl)-1,4-diazacyclohexyl]-(2E)-3-(3,5-dimethoxy-4-hydroxyphenyl)-2-propenamide (7.00 g) was dissolved in anhydrous dimethylformamide (450 mℓ), followed by the addition of sodium hydride (920 mg) while paying attention to exothermic heat. After production of gas subsided, 1-bromodecane (5.09 g) was added, followed by a reaction with stirring for 7 days at room temperature. After completion of the reaction, the reaction mixture was poured into a great deal of water and subsequent to adjustment to pH 6 with 1 N hydrochloric acid, was allowed to stand overnight. The reaction mixture was filtered with Celite, and the Celite was thoroughly washed with water and then dried in air. The Celite was subjected to extraction in dichloromethane, and the dichloromethane layer was concentrated to dryness under reduced pressure. By recrystallization from ethanol, white crystalline Compound 17 (4.31 g) was obtained. The data of ¹H-NMR, mass spectrum, infrared absorption spectrum and melting point of this compound are as follows:
¹H-NMR (400MHz,CDCℓ₃) δ: 0.88(3H,t), 1.22-1.48(14H,m), 1.71-1.77(2H,m), 2.28(3H,s), 2.31(3H,s), 2.91(4H,brs), 3.82(4H,brs), 3.88(6H,s), 3.99(2H,t), 6.75(2H,s), 6.80(1H,d,J=15.4Hz), 6.90(1H,d,J=8.0Hz), 6.98(1H,dd,J=8.0,2.1Hz), 7.03(1H,d,J=2.1Hz), 7.62(1H,d,J=15.4Hz).
MS (EI) m/z: 536(M+), 347, 208, 161.
IR (KBr) νₘₐₓ cm⁻¹: 1635.
Melting point °C: 90.7-98.6.

### Compound 18: N-[4-(2,4-dimethylphenyl)-1,4-diazacyclohexyl]-(2E)-3-(2,5-dimethoxy-4-undecyloxyphenyl)-2-propenamide

N- [4-(2,4-Dimethylphenyl)-1,4-diazacyclohexyl]-(2E)-3-(3,5-dimethoxy-4-hydroxyphenyl)-2-propenamide (4.00 g) was dissolved in anhydrous dimethylformamide (400 mℓ), followed by the addition of sodium hydride (656 mg) while paying attention to exothermic heat. After production of gas subsided, 1-bromoundecane (3.09 g) was added, followed by a reaction with stirring for 7 days at room temperature. After completion of the reaction, the reaction mixture was poured into a great deal of water and subsequent to adjustment to pH 6 with 1 N hydrochloric acid, was allowed to stand overnight. The reaction mixture was filtered with Celite, and the Celite was thoroughly washed with water and then dried in air. The Celite was subjected to extraction in dichloromethane, and the dichloromethane layer was concentrated to dryness under reduced pressure. By recrystallization from ethanol, slightly yellow crystalline Compound 18 (3.79 g) was obtained. The data of ¹H-NMR, mass spectrum, infrared absorption spectrum and melting point of this compound are as follows:
¹H-NMR (400MHz,CDCℓ₃) δ: 0.88(3H,t), 1.21-1.48(16H,m), 1.72-1.78(2H,m), 2.27(3H,s), 2.30(3H,s), 2.90(4H,brs), 3.81(4H,brs), 3.87(6H,s), 3.99(2H,t), 6.75(2H,s), 6.82(1H,d,J=15.4Hz), 6.89(1H,d,J=8.0Hz), 6.97(1H,dd,J=8.0,2.1Hz), 7.02(1H,d,J=2.1Hz), 7.62(1H,d,J=15.4Hz).
MS (EI) m/z: 550(M+), 361, 208, 161.
IR (KBr) νₘₐₓ cm⁻¹: 1640.
Melting point °C: 83.3-84.1.

### Compound 19: N-[4-(2,4-dimethylphenyl)-1,4-diazacyclohexyl]-(2E)-3-(3,5-dimethoxy-4-dodecyloxyphenyl) -2-propenamide

N-[4-(2,4-Dimethylphenyl)-1,4-diazacyclohexyl]-(2E)-3-(3,5-dimethoxy-4-hydroxyphenyl)-2-propenamide (5.08 g) was dissolved in anhydrous dimethylformamide (400 mℓ), followed by the addition of sodium hydride (656 mg) while paying attention to exothermic heat. After production of gas subsided, 1-bromododecane (4.09 g) was added, followed by a reaction with stirring for 7 days at room temperature. After completion of the reaction, the reaction mixture was poured into a great deal of water and subsequent to adjustment to pH 6 with 1 N hydrochloric acid, was allowed to stand overnight. The reaction mixture was filtered with Celite, and the Celite was thoroughly washed with water and then dried in air. The Celite was subjected to extraction in dichloromethane, and the dichloromethane layer was concentrated to dryness under reduced pressure. By recrystallization from ethanol, slightly yellow crystalline Compound 19 (5.12 g) was obtained. The data of ¹H-NMR, mass spectrum, infrared absorption spectrum and melting point of this compound are as follows:
¹H-NMR (400MHz,CDCℓ₃) δ: 0.88(3H,t), 1.22-1.48(18H,m), 1.72-1.78(2H,m), 2.27(3H,s), 2.30(3H,s), 2.90(4H,brs), 3.81(4H,brs), 3.87(6H,s), 3.99(2H,t), 6.76(2H,s), 6.83(1H,d,J=15.4Hz), 6.88(1H,d,J=8.0Hz), 6.97(1H,dd,J=8.0,2.1Hz), 7.02(1H,d,J=2.1Hz), 7.62(1H,d,J=15.4Hz).
MS (EI) m/z: 564(M+), 375, 208, 161.
IR (KBr) νₘₐₓ cm⁻¹: 1645.
Melting point °C: 78.6-80.5.

### Compound 20: N-[4-(2,4-dimethylphenyl)-1,4-diazacyclohexyl]-(2E)-3-(3,5-dimethoxy-4-benzyloxyphenyl)-2-propenamide

4-Benzyloxy-3,5-dimethoxysuccinic acid (15.72 g) was dissolved in chloroform (500 mℓ), followed by the addition of 1-ethyl-3-(3-dimethylaminopropyl)-carbodiimide hydrochloride (9.59 g) and further of 1-(2,4-dimethylphenyl)piperazine (9.51 g). A reaction was conducted with stirring at room temperature. The reaction mixture was fractionated through a silica gel column and target fractions were obtained. After the target fractions were concentrated to dryness under reduced pressure, the residue was recrystallized from ethanol, whereby slightly yellow crystalline Compound 20 (9.86 g) was obtained. The data of ¹H-NMR, mass spectrum, infrared absorption spectrum and melting point of this compound are as follows:
¹H-NMR (400MHz,CDCℓ₃) δ: 2.28(3H,s), 2.31(3H,s), 2.91(4H,brs), 3.82(4H,brs), 3.86(6H,s), 5.04(2H,s), 6.74(2H,s), 6.80(1H,d,J=15.4Hz), 6.90(1H,d,J=8.0Hz), 6.98(1H,dd,J=8.0,2.1Hz), 7.03(1H,d,J=2.1Hz), 7.27-7.36(4H,m), 7.47-7.49(2H,m), 7.61(1H,d,J=15.4Hz).
MS (EI) m/z: 486(M+), 395, 190, 161.
IR (KBr) νₘₐₓ cm⁻¹: 1635.
Melting point °C: 92.2-104.1.

### Compound 21: N-[4-(2,4-dimethylphenyl)-1,4-diazacyclohexyl]-(2E)-3-[3,5-dimethoxy-4-geranyloxyphenyl]-2-propenamide

N-[4-(2,4-Dimethylphenyl)-1,4-diazacyclohexyl]-(2E)-3-(3,5-dimethoxy-4-hydroxyphenyl)-2-propenamide (5.08 g) was dissolved in anhydrous dimethylformamide (400 mℓ), followed by the addition of sodium hydride (656 mg) while paying attention to exothermic heat. After production of gas subsided, geranyl bromide (3.56 g) was added, followed by a reaction with stirring for 7 days at room temperature. After completion of the reaction, the reaction mixture was poured into a great deal of water and subsequent to adjustment to pH 6 with 1 N hydrochloric acid, was allowed to stand overnight. The reaction mixture was filtered with Celite, and the Celite was thoroughly washed with water and then dried in air. The Celite was subjected to extraction in dichloromethane, and the dichloromethane layer was concentrated to dryness under reduced pressure. By recrystallization from ethanol, slightly yellow crystalline Compound 21 (5.67 g) was obtained. The data of ¹H-NMR, mass spectrum, infrared absorption spectrum and melting point of this compound are as follows:
¹H-NMR (400MHz,CDCℓ₃) δ: 1.59(3H,s), 1.66(3H,s), 1.67(3H,s), 1.93-2.10(4H,m), 2.28(3H,s), 2.31(3H,s), 2.91(4H,brs), 3.81(4H,brs), 3.89(6H,s), 4.57(2H,d,J=7.0Hz), 5.08(1H,t), 5.56(1H,t), 6.75(2H,s), 6.81(1H,d,J=15.4Hz, 6.89(1H,d,J=8.0Hz), 6.98(1H,dd,J=8.0,2.1Hz), 7.03(1H,d,J=2.1Hz), 7.62(1H,d,J=15.4Hz).
MS (EI) m/z: 532(M+), 397, 208, 161.
IR (KBr) νₘₐₓ cm⁻¹: 1635.
Melting point °C: 55.2-70.5.

### Compound 22: N-[4-(2,4-dimethylphenyl)-1,4-diazacyclohexyl]-(2E)-3-[3,5-dimethoxy-4-farnesyloxyphenyl]-2-propenamide

N-[4-(2,4-Dimethylphenyl)-1,4-diazacyclohexyl]-(2E)-3-(3,5-dimethoxy-4-hydroxyphenyl)-2-propenamide (5.00 g) was dissolved in anhydrous dimethylformamide (400 mℓ), followed by the addition of sodium hydride (656 mg) while paying attention to exothermic heat. After production of gas subsided, trans-farnesyl bromide (4.68 g) was added, followed by a reaction with stirring for 7 days at room temperature. After completion of the reaction, the reaction mixture was poured into a great deal of water and subsequent to adjustment to pH 6 with 1 N hydrochloric acid, was allowed to stand overnight. The reaction mixture was filtered with Celite, and the Celite was thoroughly washed with water and then dried in air. The Celite was subjected to extraction in dichloromethane, and the dichloromethane layer was concentrated to dryness under reduced pressure. By recrystallization from ethanol, slightly yellow crystalline Compound 22 (5.22 g) was obtained. The data of ¹H-NMR, mass spectrum, infrared absorption spectrum and melting point of this compound are as follows:
¹H-NMR (400MHz,CDCℓ₃) δ: 1.59(6H,s), 1.66(3H,s), 1.68(3H,s), 1.94-2.12(8H,m), 2.27(3H,s), 2.30(3H,s), 2.90(4H,brs), 3.81(4H,brs), 3.88(6H,s), 4.57(2H,d,J=7.0Hz), 5.09(1H,t), 5.10(1H,t), 5.57(1H,t), 6.75(2H,s), 6.82(1H,d,J=15.4Hz), 6.88(1H,d,J=8.0Hz), 6.97(1H,dd,J=8.0,2.1Hz), 7.02(1H,d,J=2.1Hz), 7.62(1H,d,J=15.4Hz).
MS (EI) m/z: 600(M+), 397, 208, 161.
IR (KBr) νₘₐₓ cm⁻¹: 1635.
Melting point °C: 55.1-59.2.

### Compound 23: N-[4-(2,4-dimethylphenyl)-1,4-diazacyclohexyl]-(2E)-3-[3,5-dimethoxy-4-(3-phenyl)propyloxy]-2-propenamide

N-[4-(2,4-Dimethylphenyl)-1,4-diazacyclohexyl]-(2E)-3-(3,5-dimethoxy-4-hydroxyphenyl)-2-propenamide (5.00 g) was dissolved in anhydrous dimethylformamide (400 mℓ), followed by the addition of sodium hydride (656 mg) while paying attention to exothermic heat. After production of gas subsided, 3-phenylpropyl bromide (3.26 g) was added, followed by a reaction with stirring for 7 days at room temperature. After completion of the reaction, the reaction mixture was poured into a great deal of water and subsequent to adjustment to pH 6 with 1 N hydrochloric acid, was allowed to stand overnight. The reaction mixture was filtered with Celite, and the Celite was thoroughly washed with water and then dried in air. The Celite was subjected to extraction in dichloromethane, and the dichloromethane layer was concentrated to dryness under reduced pressure. By recrystallization from ethanol, slightly yellow crystalline Compound 23 (6.27 g) was obtained. The data of ¹H-NMR, mass spectrum, infrared absorption spectrum and melting point of this compound are as follows:
¹H-NMR (400MHz,CDCℓ₃) δ: 2.06(2H,m), 2.28(3H,s), 2.31(3H,s), 2.84(2H,t), 2.91(4H,brs), 3.82(4H,brs), 3.87(6H,s), 4.04(2H,t), 6.75(2H,s), 6.81(1H,d,J=15.4Hz), 6.87-7.04(3H,m), 7.15-7.31(5H,m), 7.62(1H,d,J=15.4Hz).
MS (EI) m/z: 514(M+), 325, 190, 161.
IR (KBr) νₘₐₓ cm⁻¹: 1635.
Melting point °C: 92.2-104.1.

### Compound 24: N-[4-(2,4-dimethylphenyl)-1,4-diazacyclohexyl]-(2E)-3-[3,5-dimethoxy-4-(2-phenoxy)ethyloxy]-2-propenamide

N-[4-(2,4-Dimethylphenyl)-1,4-diazacyclohexyl]-(2E)-3-(3,5-dimethoxy-4-hydroxyphenyl)-2-propenamide (5.00 g) was dissolved in anhydrous dimethylformamide (400 mℓ), followed by the addition of sodium hydride (656 mg) while paying attention to exothermic heat. After production of gas subsided, β-chlorophenetole (2.57 g) was added, followed by a reaction with stirring for 7 days at room temperature. After completion of the reaction, the reaction mixture was poured into a great deal of water and subsequent to adjustment to pH 6 with 1 N hydrochloric acid, was allowed to stand overnight. The reaction mixture was filtered with Celite, and the Celite was thoroughly washed with water and then dried in air. The Celite was subjected to extraction in dichloromethane, and the dichloromethane layer was concentrated to dryness under reduced pressure. By recrystallization from ethanol, slightly yellow crystalline Compound 24 (1.36 g) was obtained. The data of ¹H-NMR, mass spectrum, infrared absorption spectrum and melting point of this compound are as follows:
¹H-NMR (400MHz,CDCℓ₃) δ: 2.27(3H,s), 2.30(3H,s), 2.91(4H,brs), 3.82(4H,brs), 3.83(6H,s), 4.27(2H,t), 4.39(2H,t), 6.74(2H,s), 6.80(1H,d,J=15.4Hz), 6.87-7.05(6H,m), 7.23-7.30(2H,m), 7.62(1H,d,J=15.4Hz).
MS (EI) m/z: 516(M+), 327, 208, 161.
IR (KBr) νₘₐₓ cm⁻¹: 1645.
Melting point °C: 123.0-123.9.

### compound 25: N-(4-phenyl-1,4-diazacyclohexyl)-(2E)-3-(3,5-dimethoxy-4-dodecyloxyphenyl)-2-propenamide

3,5-Dimethoxy-4-decyloxysuccinic acid (3.00 g) was dissolved in chloroform (200 mℓ), followed by the addition of 1-ethyl-3-(3-dimethylaminopropyl)-carbodiimide hydrochloride (1.58 g) and further of 1-phenylpiperazine (1.34 g). A reaction was conducted with stirring at room temperature. The reaction mixture was fractionated through a silica gel column and target fractions were obtained. After the target fractions were concentrated to dryness under reduced pressure, the residue was recrystallized from ethanol, whereby slightly yellow crystalline Compound 25 (1.03 g) was obtained. The data of ¹H-NMR, mass spectrum, infrared absorption spectrum and melting point of this compound are as follows:
¹H-NMR (400MHz,CDCℓ₃) δ: 0.88(3H,t), 1.22-1.49(14H,m), 1.73-1.79(2H,m), 3.19(4H,brs), 3.83(4H,brs), 1.86(6H,s), 4.00(2H,t), 6.76(2H,s), 6.82(1H,d,J=15.4Hz), 6.86-6.94(3H,m), 7.24-7.30(2H,m), 7.63(1H,J=15.4Hz).
MS (EI) m/z: 508(M+), 347, 208, 132.
IR (KBr) νₘₐₓ cm⁻¹: 1640.
Melting point °C: 77.6-78.0.

### Compound 26: N-[4-(2-methylphenyl)-1,4-diazacyclohexyl]-(2E)-3-(3,5-dimethoxy-4-decyloxyphenyl)-2-propenamide

3,5-Dimethoxy-4-decyloxysuccinic acid (3.00 g) was dissolved in chloroform (200 mℓ), followed by the addition of 1-ethyl-3-(3-dimethylaminopropyl)-carbodiimide hydrochloride (1.58 g) and further of 1-(2-methylphenyl)piperazine (2.05 g). A reaction was conducted with stirring at room temperature. The reaction mixture was fractionated through a silica gel column and target fractions were obtained. After the target fractions were concentrated to dryness under reduced pressure, the residue was recrystallized from ethanol, whereby slightly yellow crystalline Compound 26 (1.43 g) was obtained. The data of ¹H-NMR, mass spectrum, infrared absorption spectrum and melting point of this compound are as follows:
¹H-NMR (400MHZ,CDCℓ₃) δ: 0.88(3H,t), 1.22-1.48(14H,m), 1.72-1.79(2H,m), 2.34(3H,s), 2.94(4H,brs), 3.82(4H,brs), 3.88(6H,s), 3.99(2H,t), 6.76(2H,s), 6.82(1H,d,J=15.4Hz), 6.97-7.22(4H,m), 7.63(1H,d,J=15.4Hz).
MS (EI) m/z: 522(M+), 347, 208, 147.
IR (KBr) νₘₐₓ cm⁻¹: 1645.
Melting point °C: 85.0-95.0.

### Compound 27: N-[ 4-(3-methylphenyl)-1,4-diazacyclohexyl]-(2E)-3-(3,5-dimethoxy-4-decyloxyphenyl)-2-propenamide

3,5-Dimethoxy-4-decyloxysuccinic acid (3.00 g) was dissolved in chloroform (200 mℓ), followed by the addition of 1-ethyl-3-(3-dimethylaminopropyl)-carbodiimide hydrochloride (1.58 g) and further of 1-(3-methylphenyl)piperazine (0.75 g). A reaction was conducted with stirring at room temperature. The reaction mixture was fractionated through a silica gel column and target fractions were obtained. After the target fractions were concentrated to dryness under reduced pressure, the residue was recrystallized from ethanol, whereby slightly yellow crystalline Compound 27 (0.75 g) was obtained. The data of ¹H-NMR, mass spectrum, infrared absorption spectrum and melting point of this compound are as follows:
¹H-NMR (400MHz,CDCℓ₃) δ: 0.88(3H,t), 1.22-1.48(14H,m), 1.71-1.79(2H,m), 2.32(3H,s), 3.19(4H,brs), 3.85(4H,brs), 3.87(6H,s), 4.00(2H,t), 6.70-6.77(5H,m), 6.81(1H,d,J=15.4Hz), 7.16(1H,t), 7.62(1H,d,J=15.4Hz).
MS (EI) m/z: 522(M+), 347, 208, 147.
IR (KBr) νₘₐₓ cm⁻¹: 1642.
Melting point °C: 76.1-76.8.

### Compound 28: N-[4-(4-methylphenyl)-1,4-diazacyclohexyl]-(2E)-3-(3,5-dimethoxy-4-decyloxyphenyl)-2-propenamide

3,5-Dimethoxy-4-decyloxysuccinic acid (3.00 g) was dissolved in chloroform (200 mℓ), followed by the addition of 1-ethyl-3-(3-dimethylaminopropyl)-carbodiimide hydrochloride (1.58 g) and further of 1-(4-methylphenyl)piperazine (2.10 g). A reaction was conducted with stirring at room temperature. The reaction mixture was fractionated through a silica gel column and target fractions were obtained. After the target fractions were concentrated to dryness under reduced pressure, the residue was recrystallized from ethanol, whereby slightly yellow crystalline Compound 28 (0.29 g) was obtained. The data of ¹H-NMR, mass spectrum, infrared absorption spectrum and melting point of this compound are as follows:
¹H-NMR (400MHz,CDCℓ₃) δ: 0.88(3H,t), 1.22-1.48(14H,m), 1.72-1.78(2H,m), 2.27(3H,s), 3.15(4H,brs), 3.84(4H,brs), 3.99(2H,t), 6.75(2H,s), 6.80(1H,d,J=15.4Hz), 6.82(2H,d,J=8.2Hz), 7.09(2H,d,J=8.2Hz), 7.62(1H,d,J=15.4Hz).
MS (EI) m/z: 522(M+), 347, 208, 147.
IR (KBr) νₘₐₓ cm⁻¹: 1640.
Melting point °C: 108.0-117.5.

### Compound 29: N-[4-(2,3-dimethylphenyl)-1,4-diazacyclohexyl]-(2E)-3-(3,5-dimethoxy-4-decyloxyphenyl)-2-propenamide

3,5-Dimethoxy-4-decyloxysuccinic acid (3.00 g) was dissolved in chloroform (600 mℓ), followed by the addition of 1-ethyl-3-(3-dimethylaminopropyl)-carbodiimide hydrochloride (1.58 g) and further of 1-(2,3-dimethylphenyl)piperazine (1.57 g). A reaction was conducted with stirring at room temperature. The reaction mixture was fractionated through a silica gel column and target fractions were obtained. After the target fractions were concentrated to dryness under reduced pressure, the residue was recrystallized from ethanol, whereby slightly yellow crystalline Compound 29 (1.34 g) was obtained. The data of ¹H-NMR, mass spectrum, infrared absorption spectrum and melting point of this compound are as follows:
¹H-NMR (400MHz,CDCℓ₃) δ: 0.88(3H,t), 1.22-1.48(14H,m), 1.72-1.78(2H,m), 2.26(3H,s), 2.28(3H,s), 2.91(4H,brs), 3.82(4H,brs), 3.87(6H,s), 3.99(2H,t), 6.75(2H,s), 6.82(1H,d,J=15.4Hz), 6.87(1H,d,J=7.8Hz), 6.93(1H,d,J=7.8Hz), 7.08(1H,t), 7.62(1H,d,J=15.4Hz).
MS (EI) m/z: 536(M+), 347, 208, 161.
IR (KBr) νₘₐₓ cm⁻¹: 1645.
Melting point °C: 98.0-109.2.

### Compound 30: N-[4-(2,4-dimethylphenyl)-1,4-diazacyclohexyl]-(2E)-3-(3,5-dimethoxy-4-decyloxyphenyl)-2-propenamide

3,5-Dimethoxy-4-decyloxysuccinic acid (3.00 g) was dissolved in chloroform (300 mℓ), followed by the addition of 1-ethyl-3-(3-dimethylaminopropyl)-carbodiimide hydrochloride (1.58 g) and further of 1-(2,5-dimethylphenyl)piperazine (1.57 g). A reaction was conducted with stirring at room temperature. The reaction mixture was fractionated through a silica gel column and target fractions were obtained. After the target fractions were concentrated to dryness under reduced pressure, the residue was recrystallized from ethanol, whereby slightly yellow crystalline Compound 30 (1.32 g) was obtained. The data of ¹H-NMR, mass spectrum, infrared absorption spectrum and melting point of this compound are as follows:
¹H-NMR (400MHz,CDCℓ₃) δ: 0.88(3H,t), 1.22(14H,m), 1.72-1.78(2H,m), 2.29(3H,s), 2.29(3H,s), 2.92(4H,brs), 3.83(4H,brs), 3.87(6H,s), 3.99(2H,t), 6.76(2H,s), 6.78-6.86(3H,m), 7.07(1H,d,J=7.8Hz), 7.62(1H,d,J=15.4Hz).
MS (EI) m/z: 536(M+), 347, 208, 161.
IR (KBr) νₘₐₓ cm⁻¹: 1645.
Melting point °C: 66.6-67.1.

### Compound 31: N-[4-(2,6-dimethylphenyl)-1,4-diazacyclohexyl]-(2E)-3-(3,5-dimethoxy-4-decyloxyphenyl)-2-propenamide

3,5-Dimethoxy-4-decyloxysuccinic acid (3.00 g) was dissolved in chloroform (300 mℓ), followed by the addition of 1-ethyl-3-(3-dimethylaminopropyl)-carbodiimide hydrochloride (1.58 g) and further of 1-(2,6-dimethylphenyl)piperazine (1.57 g). A reaction was conducted with stirring at room temperature. The reaction mixture was fractionated through a silica gel column and target fractions were obtained. After the target fractions were concentrated to dryness under reduced pressure, the residue was recrystallized from ethanol, whereby slightly yellow crystalline Compound 31 (1.22 g) was obtained. The data of ¹H-NMR, mass spectrum, infrared absorption spectrum and melting point of this compound are as follows:
¹H-NMR (400MHz,CDCℓ₃) δ: 0.88(3H,t), 1.23-1.48(14H,m), 1.72-1.78(2H,m), 2.32(6H,s), 3.13(4H,brs), 3.85(4H,brs), 3.87(6H,s), 3.99(2H,t), 6.76(2H,s), 6.83(1H,d,J=15.4Hz), 6.94-7.02(3H,m), 7.63(1H,d,J=15.4Hz).
MS (EI) m/z: 536(M+), 347, 208, 161.
IR (KBr) νₘₐₓ cm⁻¹: 1635.
Melting point °C: 65.1-70.6.

### Compound 32: N-[4-(3,5-dimethylphenyl)-1,4-diazacyclohexyl]-(2E)-3-(3,5-dimethoxy-4-decyloxyphenyl)-2-propenamide

3,5-Dimethoxy-4-decyloxysuccinic acid (2.64 g) was dissolved in chloroform (300 mℓ), followed by the addition of 1-ethyl-3-(3-dimethylaminopropyl)-carbodiimide hydrochloride (1.58 g) and further of 1-(3,5-dimethylphenyl)piperazine (1.15 g). A reaction was conducted with stirring at room temperature. The reaction mixture was fractionated through a silica gel column and target fractions were obtained. After the target fractions were concentrated to dryness under reduced pressure, the residue was recrystallized from ethanol, whereby slightly yellow crystalline Compound 32 (1.43 g) was obtained. The data of ¹H-NMR, mass spectrum, infrared absorption spectrum and melting point of this compound are as follows:
¹H-NMR (400MHz,CDCℓ₃) δ: 0.88(3H,t), 1.22-1.48(14H,m), 1.72-1.78(2H,m), 2.29(6H,s), 3.20(4H,t), 3.84(brs), 3.88(6H,s), 4.00(2H,t), 6.57(3H,s), 6.75(2H,s), 6.80(1H,d,J=15.4Hz), 7.62(1H,d,J=15.4Hz).
MS (EI) m/z: 536(M+), 347, 208, 161.
IR (KBr) νₘₐₓ cm⁻¹: 1640.
Melting point °C: 79.9-80.3.

### Compound 33: N-[4-(4-tert-butylphenyl)-1,4-diazacyclohexyl]-(2E)-3-(3,5-dimethoxy-4-decyloxyphenyl)-2-propenamide

3,5-Dimethoxy-4-decyloxysuccinic acid (3.00 g) was dissolved in chloroform (300 mℓ), followed by the addition of 1-ethyl-3-(3-dimethylaminopropyl)-carbodiimide hydrochloride (1.58 g) and further of 1-(4-tert-butylphenyl)piperazine (1.80 g). A reaction was conducted with stirring at room temperature. The reaction mixture was fractionated through a silica gel column and target fractions were obtained. After the target fractions were concentrated to dryness under reduced pressure, the residue was recrystallized from ethanol, whereby slightly yellow crystalline Compound 33 (0.55 g) was obtained. The data of ¹H-NMR, mass spectrum, infrared absorption spectrum and melting point of this compound are as follows:
¹H-NMR (400MHz,CDCℓ₃) δ: 0.88(3H,t), 1.23-1.48(23H,m), 1.72-1.78(2H,m), 3.19(4H,t), 3.84(4H,brs), 3.88(6H,s), 3.99(2H,t), 6.75(2H,s), 6.80(1H,d,J=15.4Hz), 6.86-6.91(2H,m), 7.28-7.34(2H,m), 7.62(1H,d,J=15.4Hz).
MS (EI) m/z: 564(M+), 347, 208, 189.
IR (KBr) νₘₐₓ cm⁻¹: 1640.
Melting point °C: 121.5-122.2.

### Compound 34: N-[4-(2-methoxyphenyl)-1,4-diazacyclohexyl]-(2E)-3-(3,5-dimethoxy-4-decyloxyphenyl)-2-propenamide

3,5-Dimethoxy-4-decyloxysuccinic acid (3.00 g) was dissolved in chloroform (300 mℓ), followed by the addition of 1-ethyl-3-(3-dimethylaminopropyl)-carbodiimide hydrochloride (1.58 g) and further of 1-(2-methoxyphenyl)piperazine (1.58 g). A reaction was conducted with stirring at room temperature. The reaction mixture was fractionated through a silica gel column and target fractions were obtained. After the target fractions were concentrated to dryness under reduced pressure, the residue was recrystallized from ethanol, whereby slightly yellow crystalline Compound 34 (0.89 g) was obtained. The data of ¹H-NMR, mass spectrum, infrared absorption spectrum and melting point of this compound are as follows:
¹H-NMR (400MHz,CDCℓ₃) δ: 0.88(3H,t), 1.22-1.49(14H,m), 1.72-1.79(2H,m), 3.09(4H,brs), 3.87(9H,s), 3.89(4H,brs), 3.99(2H,t), 6.76(2H,s), 6.84(1H,d,J=15.4Hz), 6.86-7.06(4H,m), 7.63(1H,d,J=15.4Hz).
MS (EI) m/z: 538(M+), 347, 208, 163.
IR (KBr) νₘₐₓ cm⁻¹: 1645.
Melting point °C: 76.8-90.0.

### Compound 35: N-[4-(3-methoxyphenyl)-1,4-diazacyclohexyl]-(2E)-3-(3,5-dimethoxy-4-decyloxyphenyl)-2-propenamide

3,5-Dimethoxy-4-decyloxysuccinic acid (3.00 g) was dissolved in chloroform (300 mℓ), followed by the addition of 1-ethyl-3-(3-dimethylaminopropyl)-carbodiimide hydrochloride (1.58 g) and further of 1-(3-methoxyphenyl)piperazine (1.58 g). A reaction was conducted with stirring at room temperature. The reaction mixture was fractionated through a silica gel column and target fractions were obtained. After the target fractions were concentrated to dryness under reduced pressure, the residue was recrystallized from ethanol, whereby slightly yellow crystalline Compound 35 (1.61 g) was obtained. The data of ¹H-NMR, mass spectrum, infrared absorption spectrum and melting point of this compound are as follows:
¹H-NMR (400MHZ,CDCℓ₃) δ: 0.88(3H,t), 1.22-1.49(14H,m), 1.72-1.78(2H,m), 3.20(4H,t), 3.77(3H,s), 3.84(4H,brs), 3.87(6H,s), 4.00(2H,t), 6.42-6.55(3H,m), 6.76(2H,s), 6.81(1H,d,J=15.4Hz), 7.18(1H,t), 7.62(1H,d,J=15.4Hz).
MS (EI) m/z: 538(M+), 347, 208, 163.
IR (KBr) νₘₐₓ cm⁻¹: 1640.
Melting point °C: 85.9-87.0.

### Compound 36: N-[4-(4-methoxyphenyl)-1,4-diazacyclohexyl]-(2E)-3-(3,5-dimethoxy-4-decyloxyphenyl)-2-propenamide

3,5-Dimethoxy-4-decyloxysuccinic acid (3.00 g) was dissolved in chloroform (300 mℓ), followed by the addition of 1-ethyl-3-(3-dimethylaminopropyl)-carbodiimide hydrochloride (1.58 g) and further of 1-(4-methoxyphenyl)piperazine (2.20 g). A reaction was conducted with stirring at room temperature. The reaction mixture was fractionated through a silica gel column and target fractions were obtained. After the target fractions were concentrated to dryness under reduced pressure, the residue was recrystallized from ethanol, whereby slightly yellow crystalline Compound 36 (0.79 g) was obtained. The data of ¹H-NMR, mass spectrum, infrared absorption spectrum and melting point of this compound are as follows:
¹H-NMR (400MHz,CDCℓ₃) δ: 0.88(3H,t), 1.22-1.48(14H,m), 1.75(2H,m), 3.08(4H,brs), 3.76(3H,s), 3.84(4H,brs), 3.87(6H,s), 3.99(2H,t), 6.76(2H,s), 6.80-6.93(5H,m), 7.62(1H,d,J=15.4Hz).
MS (EI) m/z: 538(M+), 347, 208, 163.
IR (KBr) νₘₐₓ cm⁻¹: 1640.
Melting point °C: 113.1-114.3.

### Compound 37: N-[4-(2,4-dimethoxyphenyl)-1,4-diazacyclohexyl]-(2E)-3-(3,5-dimethoxy-4-decyloxyphenyl)-2-propenamide

3,5-Dimethoxy-4-decyloxysuccinic acid (2.89 g) was dissolved in chloroform (300 mℓ), followed by the addition of 1-ethyl-3-(3-dimethylaminopropyl)-carbodiimide hydrochloride (1.58 g) and further of 1-(2,4-dimethoxyphenyl)piperazine (1.47 g). A reaction was conducted with stirring at room temperature. The reaction mixture was fractionated through a silica gel column and target fractions were obtained. After the target fractions were concentrated to dryness under reduced pressure, the residue was recrystallized from ethanol, whereby slightly yellow crystalline Compound 37 (1.90 g) was obtained. The data of ¹H-NMR, mass spectrum, infrared absorption spectrum and melting point of this compound are as follows:
¹H-NMR (400MHz,CDCℓ₃) δ: 0.88(3H,t), 1.22-1.49(14H,m), 1.72-1.78(2H,m), 3.01(4H,brs), 3.77(3H,s), 3.84(3H,s), 3.85(4H,brs), 3.87(6H,s), 3.99(2H,t), 6.42(1H,dd,J=8.5,2.6Hz), 6.49(1H,d,J=2.6Hz), 6.76(2H,s), 6.82(1H,d,J=8.5Hz), 6.84(1H,d,J=15.4Hz), 7.62(1H,d,J=15.4Hz).
MS (EI) m/z: 568(M+), 347, 208, 193.
IR (KBr) νₘₐₓ cm⁻¹: 1640.
Melting point °C: 74.8-75.7.

### Compound 38: N-[4-(3,4-dimethoxyphenyl)-1,4-diazacyclohexyl]-(2E)-3-(3,5-dimethoxy-4-decyloxyphenyl)-2-propenamide

3,5-Dimethoxy-4-decyloxysuccinic acid (2.00 g) was dissolved in chloroform (200 mℓ), followed by the addition of 1-ethyl-3-(3-dimethylaminopropyl)-carbodiimide hydrochloride (1.05 g) and further of 1-(3,4-dimethoxyphenyl)piperazine (1.22 g). A reaction was conducted with stirring at room temperature. The reaction mixture was fractionated through a silica gel column and target fractions were obtained. After the target fractions were concentrated to dryness under reduced pressure, the residue was recrystallized from ethanol, whereby slightly yellow crystalline Compound 38 (0.43 g) was obtained. The data of ¹H-NMR, mass spectrum, infrared absorption spectrum and melting point of this compound are as follows:
¹H-NMR (400MHz,CDCℓ₃) δ: 0.88(3H,t), 1.22-1.49(14H,m), 1.72-1.79(2H,m), 3.12(4H,t), 3.84(3H,s), 3.86(4H,brs), 3.88(6H,s), 4.01(2H,t), 6.46(1H,dd,J=8.5,2.5Hz), 6.59(1H,d,J=8.5Hz), 6.76(2H,s), 6.78-6.84(2H,m), 7.62(1H,d,J=15.8Hz).
MS (EI) m/z: 568(M+), 347, 208, 193.
IR (KBr) νₘₐₓ cm⁻¹: 1640.
Melting point °C: 82.8-89.4.

### Compound 39: N-[4-(3,4,5-trimethoxyphenyl)-1,4-diazacyclohexyl]-(2E)-3-(3,5-dimethoxy-4-decyloxyphenyl)-2-propenamide

3,5-Dimethoxy-4-decyloxysuccinic acid (3.00 g) was dissolved in chloroform (300 mℓ), followed by the addition of 1-ethyl-3-(3-dimethylaminopropyl)-carbodiimide hydrochloride (1.58 g) and further of 1-(3,4,5-trimethoxyphenyl)piperazine (2.08 g). A reaction was conducted with stirring at room temperature. The reaction mixture was fractionated through a silica gel column and target fractions were obtained. After the target fractions were concentrated to dryness under reduced pressure, the residue was recrystallized from ethanol, whereby slightly yellow crystalline Compound 39 (1.38 g) was obtained. The data of ¹H-NMR, mass spectrum, infrared absorption spectrum and melting point of this compound are as follows:
¹H-NMR (400MHz,CDCℓ₃) δ: 0.88(3H,s), 1.22-1.49(14H,m), 1.72-1.79(2H,m), 3.16(4H,brs), 3.79(3H,s), 3.82(48,brs), 3.84(6H,s), 3.87(6H,s), 4.00(2H,t), 6.18(2H,s), 6.76(2H,s), 6.84(1H,d,J=15.2Hz), 7.62(1H,d,J=15.2Hz).
MS (EI) m/z: 598(M+), 347, 223, 208.
IR (KBr) νₘₐₓ cm⁻¹: 1645.
Melting point °C: 90.8-92.4.

### Compound 40: N-[4-(3-trifluoromethylphenyl)-1,4-diazacyclohexyl]-(2E)-3-(3,5-dimethoxy-4-decyloxyphenyl)-2-propenamide

3,5-Dimethoxy-4-decyloxysuccinic acid (3.00 g) was dissolved in chloroform (300 mℓ), followed by the addition of 1-ethyl-3-(3-dimethylaminopropyl)-carbodiimide hydrochloride (1.58 g) and further of 1-(3-trifluoromethyl)piperazine (1.89 g). A reaction was conducted with stirring at room temperature. The reaction mixture was fractionated through a silica gel column and target fractions were obtained. After the target fractions were concentrated to dryness under reduced pressure, the residue was recrystallized from ethanol, whereby white crystalline compound 40 (1.96 g) was obtained. The data of ¹H-NMR, mass spectrum, infrared absorption spectrum and melting point of this compound are as follows:
¹H-NMR (400MHz,CDCℓ₃) δ: 0.88(3H,s), 1.22-1.50(14H,m), 1.72-1.79(2H,m), 3.36(4H,brs), 3.83(4H,brs), 1.87(6H,s), 4.01(2H,s), 6.76(2H,s), 6.82(1H,d,J=15.4Hz), 7.04-7.15(3H,m), 7.36(1H,t), 7.64(1H,J=15.4Hz).
MS (EI) m/z: 576(M+), 347, 208, 201.
IR (KBr) νₘₐₓ cm⁻¹: 1645.
Melting point °C: 66.5-69.5.

### Compound 41: N-[4-(4-nitrophenyl)-1,4-diazacyclohexyl]-(2E)-3-(3,5-dimethoxy-4-decyloxyphenyl)-2-propenamide

3,5-Dimethoxy-4-decyloxysuccinic acid (3.00 g) was dissolved in chloroform (300 mℓ), followed by the addition of 1-ethyl-3-(3-dimethylaminopropyl)-carbodiimide hydrochloride (1.58 g) and further of 1-(4-nitrophenyl)piperazine (1.71 g). A reaction was conducted with stirring at room temperature. The reaction mixture was fractionated through a silica gel column and target fractions were obtained. After the target fractions were concentrated to dryness under reduced pressure, the residue was recrystallized from ethanol, whereby slightly yellow crystalline Compound 41 (2.04 g) was obtained. The data of ¹H-NMR, mass spectrum, infrared absorption spectrum and melting point of this compound are as follows:
¹H-NMR (400MHz,CDCℓ₃) δ: 0.88(3H,t), 1.22-1.49(14H,m), 1.72-1.80(2H,m), 3.52(4H,brs), 3.88(6H,s), 3.91(4H,brs), 4.00(2H,t), 6.76-6.85(5H,m), 7.65(1H,d,J=15.2Hz), 8.08(2H,d,J=9.2Hz).
MS (EI) m/z: 553(M+), 347, 208, 178.
IR (KBr) νₘₐₓ cm⁻¹: 1645.
Melting point °C: 117.0-126.9.

### Compound 42: N-[4-(2-fluorophenyl)-1,4-diazacyclohexyl)-(2E)-3-(3,5-dimethoxy-4-decyloxyphenyl)-2-propenamide

3,5-Dimethoxy-4-decyloxysuccinic acid (3.00 g) was dissolved in chloroform (300 mℓ), followed by the addition of 1-ethyl-3-(3-dimethylaminopropyl)-carbodiimide hydrochloride (1.58 g) and further of 1-(2-fluorophenyl)piperazine (1.48 g). A reaction was conducted with stirring at room temperature. The reaction mixture was fractionated through a silica gel column and target fractions were obtained. After the target fractions were concentrated to dryness under reduced pressure, the residue was recrystallized from ethanol, whereby white crystalline Compound 42 (1.74 g) was obtained. The data of ¹H-NMR, mass spectrum, infrared absorption spectrum and melting point of this compound are as follows:
¹H-NMR (400MHz,CDCℓ₃) δ: 0.88(3H,t), 1.21-1.48(14H,m), 1.72-1.79(2H,m), 3.13(4H,brs), 3.85(4H,brs), 1.88(6H,m), 3.99(2H,t), 6.76(2H,s), 6.83(1H,d,J=15.2Hz), 6.89-7.08(4H,m), 7.63(1H,d,J=15.2Hz).
MS (EI) m/z: 526(M+), 347, 208, 151.
IR (KBr) νₘₐₓ cm⁻¹: 1645.
Melting point °C: 58.8-61.0.

### Compound 43: N-[4-(4-fluorophenyl)-1,4-diazacyclohexyl]-(2E)-3-(3,5-dimethoxy-4-decyloxyphenyl)-2-propenamide

3,5-Dimethoxy-4-decyloxysuccinic acid (3.00 g) was dissolved in chloroform (300 mℓ), followed by the addition of 1-ethyl-3-(3-dimethylaminopropyl)-carbodiimide hydrochloride (1.58 g) and further of 1-(4-fluorophenyl)piperazine (1.48 g). A reaction was conducted with stirring at room temperature. The reaction mixture was fractionated through a silica gel column and target fractions were obtained. After the target fractions were concentrated to dryness under reduced pressure, the residue was recrystallized from ethanol, whereby slightly yellow crystalline Compound 43 (1.58 g) was obtained. The data of ¹H-NMR, mass spectrum, infrared absorption spectrum and melting point of this compound are as follows:
¹H-NMR (400MHz,CDCℓ₃) δ: 0.88(3H,t), 1.21-1.49(14H,m), 1.72-1.79(2H,m), 3.12(4H,t), 3.84(4H,brs), 3.87(6H,s), 4.00(2H,t), 6.76(2H,s), 6.81(1H,d,J=15.4Hz), 6.85-7.01(4H,m), 7.62(1H,d,J=15.4Hz).
MS (EI) m/z: 526(M+), 347, 208, 151.
IR (KBr) νₘₐₓ cm⁻¹: 1645.
Melting point °C: 65.2-65.6.

### Compound 44: N-[4-(2,4-difluorophenyl)-1,4-diazacyclohexyl)-(2E)-3-(3,5-dimethoxy-4-decyloxyphenyl)-2-propenamide

3,5-Dimethoxy-4-decyloxysuccinic acid (3.00 g) was dissolved in chloroform (300 mℓ), followed by the addition of 1-ethyl-3-(3-dimethylaminopropyl)-carbodiimide hydrochloride (1.58 g) and further of 1-(2,4-difluorophenyl)piperazine (2.23 g). A reaction was conducted with stirring at room temperature. The reaction mixture was fractionated through a silica gel column and target fractions were obtained. After the target fractions were concentrated to dryness under reduced pressure, the residue was recrystallized from ethanol, whereby slightly yellow crystalline Compound 44 (1.58 g) was obtained. The data of ¹H-NMR, mass spectrum, infrared absorption spectrum and melting point of this compound are as follows:
¹H-NMR (400MHz,CDCℓ₃) δ: 0.88(3H,t), 1.21-1.50(14H,m), 1.71-1.79(2H,s), 3.03(4H,brs), 3.84(4H,brs), 3.87(6H,s), 4.00(2H,t), 6.77(2H,s), 6.78-6.92(4H,m), 7.62(1H,d,J=15.4Hz).
MS (EI) m/z: 544(M+), 347, 208, 169.
IR (KBr) νₘₐₓ cm⁻¹: 1645.
Melting point °C: 65.4-65.7.

### Compound 45: N-[4-(3-ethylphenyl)-1,4-diazacyclohexyl]-(2E)-3-(3,5-dimethoxy-4-decyloxyphenyl)-2-propenamide

3,5-Dimethoxy-4-decyloxysuccinic acid (3.00 g) was dissolved in chloroform (300 mℓ), followed by the addition of 1-ethyl-3-(3-dimethylaminopropyl)-carbodiimide hydrochloride (1.58 g) and further of 1-(3-ethylphenyl)piperazine (1.57 g). A reaction was conducted with stirring at room temperature. The reaction mixture was fractionated through a silica gel column and target fractions were obtained. After the target fractions were concentrated to dryness under reduced pressure, the residue was recrystallized from ethanol, whereby white crystalline Compound 45 (0.70 g) was obtained. The data of ¹H-NMR, mass spectrum, infrared absorption spectrum and melting point of this compound are as follows:
¹H-NMR (400MHz,CDCℓ₃) δ: 0.88(3H,t), 1.21-1.48(17H,m), 1.71-1.79(2H,m), 2.62(2H,q), 3.22(4H,t), 3.84(4H,brs), 3.87(6H,s), 4.00(2H,t), 6.72-6.84(6H,m), 7.20(1H,t), 7.62(1H,d,J=15.4Hz).
MS (EI) m/z: 536(M+), 347, 208, 161.
IR (KBr) νₘₐₓ cm⁻¹: 1640.
Melting point °C: 70.4-70.9.

### Compound 46: N-[4-(4-phenoxyphenyl)-1,4-diazacyclohexyl]-(2E)-3-(3,5-dimethoxy-4-decyloxyphenyl)-2-propenamide

3,5-Dimethoxy-4-decyloxysuccinic acid (5.00 g) was dissolved in chloroform (300 mℓ), followed by the addition of 1-ethyl-3-(3-dimethylaminopropyl)-carbodiimide hydrochloride (1.58 g) and further of 1-(4-phenoxyphenyl)piperazine (3.50 g). A reaction was conducted with stirring at room temperature. The reaction mixture was fractionated through a silica gel column and target fractions were obtained. After the target fractions were concentrated to dryness under reduced pressure, the residue was recrystallized from ethanol, whereby slightly yellow crystalline Compound 46 (3.58 g) was obtained. The data of ¹H-NMR, mass spectrum, infrared absorption spectrum and melting point of this compound are as follows:
¹H-NMR (400MHz,CDCℓ₃) δ: 0.88(3H,t), 1.22-1.49(14H,m), 1.71-1.80(2H,m), 4.14(4H,t), 3.84(4H,brs), 3.86(6H,s), 4.00(2H,t), 6.76(2H,s), 6.80-7.05(8H,m), 7.24-7.30(2H,m), 7.63(1H,d,J=15.2Hz).
MS (EI) m/z: 600(M+), 347, 208, 224.
IR (KBr) νₘₐₓ cm⁻¹: 1640.
Melting point °C: 101.9-102.5.

### compound 47: N-[4-(3,4,5-trimethoxyphenyl)-1,4-diazacyclohexyl]-(2E)-3-(3,5-dimethoxy-4-nonyloxyphenyl)-2-propenamide

3,5-Dimethoxy-4-decyloxysuccinic acid (3.00 g) was dissolved in chloroform (200 mℓ), followed by the addition of 1-ethyl-3-(3-dimethylaminopropyl)-carbodiimide hydrochloride (1.58 g) and further of 1-(3,4,5-trimethoxyphenyl)piperazine (2.16 g). A reaction was conducted with stirring at room temperature. The reaction mixture was fractionated through a silica gel column and target fractions were obtained. After the target fractions were concentrated to dryness under reduced pressure, the residue was recrystallized from ethanol, whereby slightly yellow crystalline Compound 47 (1.05 g) was obtained. The data of ¹H-NMR, mass spectrum, infrared absorption spectrum and melting point of this compound are as follows:
¹H-NMR (400MHz,CDCℓ₃) δ: 0.88(3H,t), 1.22-1.48(12H,m), 1.72-1.78(2H,m), 3.18(4H,t), 3.80(3H,s), 3.86(6H,s), 3.87(4H,brs), 3.88(6H,s), 3.99(2H,s), 6.19(2H,s), 6.75(2H,s), 6.78(1H,d,J=15.4Hz), 7.62(1H,d,J=15.4Hz).
MS (EI) m/z: 584(M+), 333, 222, 208.
IR (KBr) νₘₐₓ cm⁻¹: 1645.
Melting point °C: 107.4-107.8.

### Compound 48: N-[4-phenyl-1,4-diazacyclohexyl]-(2E)-3-(3,5-dimethoxy-4-benzyloxyphenyl)-2-propenamide

4-Benzyloxy-3,5-dimethoxysuccinic acid (10.00 g) was dissolved in chloroform (500 mℓ), followed by the addition of 1-ethyl-3-(3-dimethylaminopropyl)-carbodiimide hydrochloride (6.40 g) and further of 1-phenylpiperazine (8.80 g). A reaction was conducted with stirring at room temperature. The reaction mixture was fractionated through a silica gel column and target fractions were obtained. After the target fractions were concentrated to dryness under reduced pressure, the residue was recrystallized from ethanol, whereby slightly yellow crystalline Compound 48 (9.64 g) was obtained. The data of ¹H-NMR, mass spectrum, infrared absorption spectrum and melting point of this compound are as follows:
¹H-NMR (400MHz,CDCℓ₃) δ: 3.16(4H,brs), 3.81(4H,brs), 3.82(6H,s), 5.03(2H,s), 6.74(2H,s), 6.81(1H,d,J=15.4Hz), 6.85-6.92(3H,m), 7.23-7.48(7H,m), 7.62(1H,d,J=15.4Hz).
MS (EI) m/z: 458(M+), 367, 208, 132.
IR (KBr) νₘₐₓ cm⁻¹: 1645.
Melting point °C: 97.6-98.8.

### Compound 49: N-(1,4-diazacyclohexyl)-(2E)-3-(3,5-dimethoxy-4-benzyloxyphenyl)-2-propenamide

Piperazine hexahydrate (15.45 g) was dissolved in chloroform (600 mℓ) and methanol (100 mℓ), followed by the addition of 1-ethyl-3-(3-dimethylaminopropyl)-carbodiimide hydrochloride (3.05 g) and further of 4-benzyloxy-3,5-dimethoxysuccinic acid (5.00 g). A reaction was conducted with stirring at room temperature. After completion of the reaction, the reaction mixture was concentrated to half the volume and then partitioned with chloroform-water. The thus-obtained chloroform layer was concentrated to dryness under reduced pressure. The residue was recrystallized from diisopropyl ether, whereby slightly yellow crystalline Compound 49 (1.79 g) was obtained. The data of ¹H-NMR, mass spectrum, infrared absorption spectrum and melting point of this compound are as follows:
¹H-NMR (400MHz,CDCℓ₃) δ: 2.55(1H,brs), 2.90(4H,t), 3.66(4H,brs), 3.85(6H,s), 5.04(2H,s), 6.72(2H,s), 6.75(1H,d,J=15.4Hz), 7.28-7.36(3H,m), 7.45-7.49(2H,m), 7.58(1H,d,J=15.4Hz).
MS (EI) m/z: 382(M+), 255, 208, 91.
IR (KBr) νₘₐₓ cm⁻¹: 1645.
Melting point °C: 132.0-135.5.

### Compound 50: N-(4-phenyl-1,4-diazacylohexyl)-(2E)-3-(4-benzyloxy-3-methoxyphenyl)-2-propenamide

1-Phenylpiperazine (1.62 g) was dissolved in chloroform (300 mℓ), followed by the addition of 1-ethyl-3-(3-dimethylaminopropyl)-carbodiimide hydrochloride (1.10 g) and further of 4-benzyloxy-3,5-dimethoxysuccinic acid (1.98 g). A reaction was conducted with stirring at room temperature. After completion of the reaction, the reaction mixture was concentrated to half the volume and then partitioned with chloroform-water. The thus-obtained chloroform layer was concentrated to dryness under reduced pressure. The residue was recrystallized from diisopropyl ether, whereby slightly yellow crystalline Compound 50 (2.10 g) was obtained. The data of ¹H-NMR, mass spectrum, infrared absorption spectrum and melting point of this compound are as follows:
¹H-NMR (400MHz,CDCℓ₃) δ: 3.15(4H,t), 3.80(4H,brs), 3.88(3H,s), 5.12(2H,s), 6.75-6.92(5H,m), 7.02-7.07(2H,m), 7.22-7.43(7H,m), 7.65(1H,d,J=15.4Hz).
MS (EI) m/z: 428(M+), 337, 132, 91.
IR (KBr) νₘₐₓ cm⁻¹: 1645.
Melting point °C: 133.5-134.1.

### Compound 51: N-(3-phenylpropyl)-(2E)-3-(3,5-dimethoxy-4-decyloxyphenyl)-2-propenamide

3,5-Dimethoxy-4-decyloxysuccinic acid (5.00 g) was dissolved in chloroform (300 mℓ), followed by the addition of 1-ethyl-3-(3-dimethylaminopropyl)-carbodiimide hydrochloride (5.26 g) and further of 3-phenylpropylamine (3.71 g). A reaction was conducted with stirring at room temperature. The reaction mixture was fractionated through a silica gel column and target fractions were obtained. After the target fractions were concentrated to dryness under reduced pressure, the residue was recrystallized from methanol, whereby white crystalline Compound 51 (2.07 g) was obtained. The data of ¹H-NMR, mass spectrum, infrared absorption spectrum and melting point of this compound are as follows:
¹H-NMR (400MHz,CDCℓ₃) δ: 0.88(3H,t), 0.12-0.47(14H,m), 1.68-1.92(4H,m), 2.63(2H,s), 3.39(2H,q), 3.74(6H,s), 6.49(1H,d,J=15.6Hz), 6.67(2H,s), 6.80(1H,t), 7.08-7.25(5H,m), 7.54(1H,d,J=15.6Hz).
MS (EI) m/z: 481(M+), 347, 208, 119.
IR (KBr) νₘₐₓ cm⁻¹: 3270, 1650.
Melting point °C: 92.5-93.0.

### Compound 52: N-(2-phenoxyethyl)-(2E)-3-(3,5-dimethoxy-4-decyloxyphenyl)-2-propenamide

3,5-Dimethoxy-4-decyloxysuccinic acid (5.00 g) was dissolved in chloroform (300 mℓ), followed by the addition of 1-ethyl-3-(3-dimethylaminopropyl)-carbodiimide hydrochloride (5.26 g) and further of 2-phenoxyethylamine (3.76 g). A reaction was conducted with stirring at room temperature. The reaction mixture was fractionated through a silica gel column and target fractions were obtained. After the target fractions were concentrated to dryness under reduced pressure, the residue was recrystallized from methanol, whereby slightly red crystalline Compound 52 (2.67 g) was obtained. The data of ¹H-NMR, mass spectrum, infrared absorption spectrum and melting point of this compound are as follows:
¹H-NMR (400MHz,CDCℓ₃) δ: 0.90(3H,t), 1.22-1.50(14H,m), 1.72-1.80(2H,m), 3.75-3.83(2H,m), 3.77(6H,s), 3.99(2H,t), 4.06(2H,t), 6.56(1H,d,J=15.4Hz), 6.71(2H,s), 6.87(2H,d,J=7.8Hz), 6.93(1H,t), 7.14(1H,t), 7.24(2H,t), 7.62(1H,d,J=15.4Hz).
MS (EI) m/z: 483(M+), 347, 208, 176.
IR (KBr) νₘₐₓ cm⁻¹: 3235, 1665.
Melting point °C: 78.6-79.4.

### Compound 53: 2-[(3,5-dimethoxy-4-decyloxycinnamoylamino)-N-(2,4-dimethylphenyl)acetamide

N-3,5-Dimethoxy-4-decyloxy-cinnamoylglycin (2.50 g) was dissolved in chloroform (100 mℓ) and 1,4-dioxane (30 mℓ), followed by the addition of 1-ethyl-3-(3-dimethylaminopropyl)-carbodiimide hydrochloride (0.91 g) and further of 2,4-dimethylaniline (1.73 g). A reaction was conducted with stirring at room temperature. The reaction mixture was fractionated through a silica gel column and target fractions were obtained. After the target fractions were concentrated to dryness under reduced pressure, the residue was recrystallized from methanol, whereby white crystalline Compound 53 (1.43 g) was obtained. The data of ¹H-NMR, mass spectrum, infrared absorption spectrum and melting point of this compound are as follows:
¹H-NMR (400MHz,CDCℓ₃) δ: 0.88(3H,t), 1.20-1.48(14H,m), 1.68-1.77(2H,m), 2.17(3H,s), 2.23(3H,s), 3.79(6H,s), 3.97(2H,t), 4.24(2H,d,J=5.4Hz), 6.55(1H,d,J=15.6Hz), 6.67(2H,s) 6.90(1H,d,J=8.0Hz), 6.93(1H,s), 7.46(1H,d,J=8.0Hz), 7.50(1H,d,J=15.6Hz), 7.00(1H,t), 8.91(1H,s).
MS (EI) m/z: 524(M+), 347, 208, 176.
IR (KBr) νₘₐₓ cm⁻¹: 3260, 1660.
Melting point °C: 112.0-115.9.

### Compound 54: 2-[(3,5-dimethoxy-4-decyloxycinnamoylamino)-N-(2,6-diisopropylphenyl)acetamide

N-3,5-Dimethoxy-4-decyloxy-cinnamoylglycin (2.50 g) was dissolved in chloroform (100 mℓ) and 1,4-dioxane (30 mℓ), followed by the addition of 1-ethyl-3-(3-dimethylaminopropyl)-carbodiimide hydrochloride (0.91 g) and further of 2,6-diisopropylaniline (1.73 g). A reaction was conducted with stirring at room temperature. The reaction mixture was fractionated through a silica gel column and target fractions were obtained. After the target fractions were concentrated to dryness under reduced pressure, the residue was recrystallized from methanol, whereby slightly yellow crystalline Compound 54 (1.54 g) was obtained. The data of ¹H-NMR, mass spectrum, infrared absorption spectrum and melting point of this compound are as follows:
¹H-NMR (400MHz,CDCℓ₃) δ: 0.88(3H,t), 1.11(12H,d), 1.24-1.47(14H,m), 1.68-1.76(2H,m), 3.03-3.15(2H,m), 3.71(6H,s), 3.94(2H,t), 6.44-6.52(3H,m), 7.12(2H,d,J=7.8Hz), 7.22(1H,d.J=7.8Hz), 7.36(1H,d,J=15.4Hz), 8.23(1H,brs), 9.14(1H,s).
MS (EI) m/z: 580(M+), 348, 233, 208.
IR (KBr) νₘₐₓ cm⁻¹: 3340, 1700.
Melting point °C: 145.3-145.8.

### Compound 55: N-(4-phenoxyphenyl)-(2E)-3-(3,5-dimethoxy-4-decyloxyphenyl)-2-propenamide

3,5-Dimethoxy-4-decyloxysuccinic acid (5.00 g) was dissolved in chloroform (300 mℓ), followed by the addition of 1-ethyl-3-(3-dimethylaminopropyl)-carbodiimide hydrochloride (7.90 g) and further of 4-phenoxyaniline (7.46 g). A reaction was conducted with stirring at room temperature. The reaction mixture was fractionated through a silica gel column and target fractions were obtained. After the target fractions were concentrated to dryness under reduced pressure, the residue was recrystallized from methanol, whereby slightly yellow crystalline Compound 55 (3.01 g) was obtained. The data of ¹H-NMR, mass spectrum, infrared absorption spectrum and melting point of this compound are as follows:
¹H-NMR (400MHz,CDCℓ₃) δ: 0.87(3H,t), 1.22-1.48(14H,m), 1.69-1.77(2H,m), 3.72(6H,s), 3.98(2H,t), 6.65(2H,s), 6.66(1H,d,J=15.4Hz), 6.91-6.97(4H,m), 7.07-7.17(1H,m), 7.25-7.32(2H,m), 7.62-7.67(3H,m).
MS (EI) m/z: 531(M+), 347, 225, 208.
IR (KBr) νₘₐₓ cm⁻¹: 3270, 1655.
Melting point °C: 89.8-90.0.

### Compound 56: N-[4-(2-pyrimidyl)-1,4-diazacyclohexyl]-(2E)-3-(3,5-dimethoxy-4-decyloxyphenyl)-2-propenamide

3,5-Dimethoxy-4-decyloxysuccinic acid (3.00 g) was dissolved in chloroform (300 mℓ), followed by the addition of 1-ethyl-3-(3-dimethylaminopropyl)-carbodiimide hydrochloride (1.58 g) and further of 1-(2-pyrimidyl)piperazine (1.35 g). A reaction was conducted with stirring at room temperature. The reaction mixture was fractionated through a silica gel column and target fractions were obtained. After the target fractions were concentrated to dryness under reduced pressure, the residue was recrystallized from methanol, whereby slightly yellow crystalline Compound 56 (1.65 g) was obtained. The data of ¹H-NMR, mass spectrum, infrared absorption spectrum and melting point of this compound are as follows:
¹H-NMR (400MHz,CDCℓ₃) δ: 0.88(3H,t), 1.22-1.50(14H,m), 1.72-1.80(2H,m), 3.78(4H,brs), 3.89(6H,s), 3.91(4H,brs), 4.00(2H,t), 6.55(1H,t), 6.76(2H,s), 6.89(1H,d,J=15.2Hz), 7.63(1H,d,J=15.2Hz), 8.34(2H,d,J=4.9Hz).
MS (EI) m/z: 510(M+), 347, 208, 135.
IR (KBr) νₘₐₓ cm⁻¹: 1645.
Melting point °C: 104.3-105.0.

### Compound 57: N-[4-(2-pyridyl)-1,4-diazacyclohexyl]-(2E)-3-(3,5-dimethoxy-4-decyloxyphenyl)-2-propenamide

3,5-Dimethoxy-4-decyloxysuccinic acid (3.00 g) was dissolved in chloroform (300 mℓ), followed by the addition of 1-ethyl-3-(3-dimethylaminopropyl)-carbodiimide hydrochloride (1.58 g) and further of 1-(2-pyridyl)piperazine (1.34 g). A reaction was conducted with stirring at room temperature. The reaction mixture was fractionated through a silica gel column and target fractions were obtained. After the target fractions were concentrated to dryness under reduced pressure, the residue was recrystallized from methanol, whereby slightly yellow crystalline Compound 57 (1.61 g) was obtained. The data of ¹H-NMR, mass spectrum, infrared absorption spectrum and melting point of this compound are as follows:
¹H-NMR (400MHz,CDCℓ₃) δ: 0.88(3H,t), 1.22-1.49(14H,m), 1.72-1.80(2H,m), 3.65(4H,brs), 3.84(4H,brs), 3.88(6H,s), 4.00(2H,t), 6.63-6.69(2H,m), 6.76(2H,s), 6.80(1H,d,J=15.4Hz), 7.47-7.54(1H,m), 7.63(1H,d,J=15.4Hz), 8.19-8.23(1H,m).
MS (EI) m/z: 509(M+), 347, 208, 134.
IR (KBr) νₘₐₓ cm⁻¹: 1635.
Melting point °C: 68.6-71.7.

### Compound 58: N-benzyl-(2E)-3-(3,5-dimethoxy-4-benzyloxyphenyl)-2-propenamide

Benzylamine (10.20 g) was dissolved in chloroform (300 mℓ), followed by the addition of 1-ethyl-3-(3-dimethylaminopropyl)-carbodiimide hydrochloride (12.20 g) and further of 4-benzyloxy-3,5-dimethoxysuccinic acid (10.00 g). A reaction was conducted with stirring at room temperature. After completion of the reaction, the reaction mixture was concentrated to half the volume and then partitioned with chloroform-water. The thus-obtained chloroform layer was concentrated to dryness under reduced pressure. The residue was recrystallized from diisopropyl ether, whereby white crystalline Compound 58 (1.94 g) was obtained. The data of ¹H-NMR, mass spectrum, infrared absorption spectrum and melting point of this compound are as follows:
¹H-NMR (400MHz,CDCℓ₃) δ: 3.80(6H,s), 4.54(2H,d,J=5.6HZ), 5.02(2H,s), 6.08(1H,brt), 6.34(1H,d,J=15.6Hz), 6.69(2H,s),
7.24-7.48(10H,m), 7.56(1H,d,J=15.6Hz). MS (EI) m/z: 403(M+), 312, 226, 92.
IR (KBr) νₘₐₓ cm⁻¹: 3250, 1650.
Melting point °C: 146.5-147.9.

### Compound 59: N-(2-phenyl)ethyl-(2E)-3-(3,5-dimethoxy-4-benzyloxyphenyl)-2-propenamide

1-Amino-2-phenylethane (11.60 g) was dissolved in chloroform (300 mℓ), followed by the addition of 1-ethyl-3-(3-dimethylaminopropyl)-carbodiimide hydrochloride (12.20 g) and further of 4-benzyloxy-3,5-dimethoxysuccinic acid (10.00 g). A reaction was conducted with stirring at room temperature. After completion of the reaction, the reaction mixture was concentrated to half the volume and then partitioned with chloroform-water. The thus-obtained chloroform layer was concentrated to dryness under reduced pressure. The residue was recrystallized from diisopropyl ether, whereby white crystalline Compound 59 (6.18 g) was obtained. The data of ¹H-NMR, mass spectrum, infrared absorption spectrum and melting point of this compound are as follows:
¹H-NMR (400MHz,CDCℓ₃) δ: 2.88(2H,t), 3.66(2H,q), 3.82(6H,s), 5.02(2H,s), 5.69(1H,brt), 6.23(1H,d,J=15.4Hz), 6.69(2H,s), 7.20-7.55(11H,m).
MS (EI) m/z: 417(M+), 326, 226, 91.
IR (KBr) νₘₐₓ cm⁻¹: 3290, 1690.
Melting point °C: 115.5-119.4.

### Compound 60: N-(3-phenyl)propyl-(2E)-3-(3,5-dimethoxy-4-benzyloxyphenyl)-2-propenamide

1-Amino-3-phenylpropane (12.90 g) was dissolved in chloroform (300 mℓ), followed by the addition of 1-ethyl-3-(3-dimethylaminopropyl)-carbodiimide hydrochloride (12.20 g) and further of 4-benzyloxy-3,5-dimethoxysuccinic acid (10.00 g). A reaction was conducted with stirring at room temperature. After completion of the reaction, the reaction mixture was concentrated to half the volume and then partitioned with chloroform-water. The thus-obtained chloroform layer was concentrated to dryness under reduced pressure. The residue was recrystallized from diisopropyl ether, whereby white crystalline Compound 60 (0.50 g) was obtained. The data of ¹H-NMR, mass spectrum, infrared absorption spectrum and melting point of this compound are as follows:
¹H-NMR (400MHz,CDCℓ₃) δ: 1.89(2H,m), 2.68(2H,t), 3.41(2H,q), 3.81(6H,s), 5.03(2H,s), 5.72(1H,brt), 6.26(1H,d,J=15.4Hz), 6.69(2H,s), 7.15-7.52(11H,m).
MS (EI) m/z: 431(M+), 340, 207, 91.
IR (KBr) νₘₐₓ cm⁻¹: 3275, 1650.
Melting point °C: 115.2-116.1.

### Compound 61: N-(4-phenyl)butyl-(2E)-3-(3,5-dimethoxy-4-benzyloxyphenyl)-2-propenamide

1-Amino-4-phenylbutane (14.23 g) was dissolved in chloroform (300 mℓ), followed by the addition of 1-ethyl-3-(3-dimethylaminopropyl)-carbodiimide hydrochloride (12.20 g) and further of 4-benzyloxy-3,5-dimethoxysuccinic acid (10.00 g). A reaction was conducted with stirring at room temperature. After completion of the reaction, the reaction mixture was concentrated to half the volume and then partitioned with chloroform-water. The thus-obtained chloroform layer was concentrated to dryness under reduced pressure. The residue was recrystallized from diisopropyl ether, whereby white crystalline Compound 61 (0.60 g) was obtained. The data of ¹H-NMR, mass spectrum, infrared absorption spectrum and melting point of this compound are as follows:
¹H-NMR (400MHz,CDCℓ₃) δ: 1.52-1.72(4H,m), 2.63(2H,t), 3.38(2H,q), 3.80(6H,s), 5.02(2H,s), 5.78(1H,brt), 6.29(1H,d,J=15.4Hz), 6.69(2H,s), 7.13-7.54(11H,m).
MS (EI) m/z: 445(M+), 354, 226, 91.
IR (KBr) νₘₐₓ cm⁻¹: 3280, 1650.
Melting point °C: 132.7-135.3.

### Compound 62: N-[4-(1-benzyl)piperidino]-(2E)-3-(3,5-dimethoxy-4-benzyloxyphenyl)-2-propenamide

4-Amino-1-benzylpiperidine (18.20 g) was dissolved in chloroform (300 mℓ), followed by the addition of 1-ethyl-3-(3-dimethylaminopropyl)-carbodiimide hydrochloride (12.20 g) and further of 4-benzyloxy-3,5-dimethoxysuccinic acid (10.00 g). A reaction was conducted with stirring at room temperature. After completion of the reaction, the reaction mixture was concentrated to half the volume and then partitioned with chloroform-water. The thus-obtained chloroform layer was concentrated to dryness under reduced pressure. The residue was recrystallized from diisopropyl ether, whereby slightly yellow crystalline Compound 62 (1.78 g) was obtained. The data of ¹H-NMR, mass spectrum, infrared absorption spectrum and melting point of this compound are as follows:
¹H-NMR (400MHz,CDCℓ₃) δ: 2.08-2.20(2H,brm), 2.26-2.42(2H,brm), 2.76-2.92(2H,brm), 3.40-3.50(2H,brm), 3.77(6H,s), 4.14(3H,brs), 4.99(2H,s), 6.54(1H,brd,J=15.6Hz), 6.71(2H,s), 7.05(1H,brs), 7.23-7.64(11H,m).
MS (EI) m/z: 486(M+), 395, 208, 92.
IR (KBr) νₘₐₓ cm⁻¹: 3260,1670.
Melting point °C: 237.1-239.0.

### Compound 63: N-(2,4-dimethylphenyl)-(2E)-3-(3,5-dimethoxy-4-benzyloxyphenyl)-2-propenamide

4-Benzyloxy-3,5-dimethoxysuccinic acid (10.00 g) was dissolved in chloroform (320 mℓ), followed by the addition of 1-ethyl-3-(3-dimethylaminopropyl)-carbodiimide hydrochloride (6.10 g) and further of 2,4-dimethylaniline (11.50 g). A reaction was conducted with stirring at room temperature. The reaction mixture was washed with 1 N hydrochloric acid and then with a saturated aqueous solution of sodium chloride (brine), and was thereafter dried over anhydrous sodium sulfate. The reaction mixture was fractionated through a silica gel column, and target fractions were obtained. After the target fractions were concentrated to dryness under reduced pressure, the residue was recrystallized from ethanol, whereby white crystalline Compound 63 (4.05 g) was obtained. The data of ¹H-NMR, mass spectrum, infrared absorption spectrum and melting point of this compound are as follows:
¹H-NMR (400MHz,CDCℓ₃) δ: 2.26(3H,s), 2.30(3H,s), 3.84(6H,s), 5.05(2H,s), 6.48(1H,d,J=15.4Hz), 6.74(2H,s), 7.00-7.50(8H,m), 7.65(1H,d,J=15.4Hz), 7.75(1H,brs).
MS (EI) m/z: 417(M+), 326, 120, 92.
IR (KBr) νₘₐₓ cm⁻¹: 3200, 1665.
Melting point °C: 215.3-215.8.

### Compound 64: N-(2,3-dimethylphenyl)-(2E)-3-(3,5-dimethoxy-4-benzyloxyphenyl)-2-propenamide

4-Benzyloxy-3,5-dimethoxysuccinic acid (5.05 g) was dissolved in chloroform (160 mℓ), followed by the addition of 1-ethyl-3-(3-dimethylaminopropyl)-carbodiimide hydrochloride (3.08 g) and further of 2,3-dimethylaniline (1.95 g). A reaction was conducted with stirring at room temperature. The reaction mixture was washed with 1 N hydrochloric acid and then with brine, and was thereafter dried over anhydrous sodium sulfate. The reaction mixture was fractionated through a silica gel column, and target fractions were obtained. After the target fractions were concentrated to dryness under reduced pressure, the residue was recrystallized from ethanol, whereby white crystalline Compound 64 (1.96 g) was obtained. The data of ¹H-NMR, mass spectrum, infrared absorption spectrum and melting point of this compound are as follows:
¹H-NMR (400MHz,CDCℓ₃) δ: 2.18(3H,s), 2.30(3H,s), 3.83(6H,S), 5.04(2H,s), 6.46-7.49(11H,m), 7.65(1H,d,J=15.4Hz).
MS (EI) m/z: 417(M+), 326, 120, 91.
IR (KBr) νₘₐₓ cm⁻¹: 3240, 1650.
Melting point °C: 173.4-173.8.

### Compound 65: N-(3,4-dimethylphenyl)-(2E)-3-(3,5-dimethoxy-4-benzyloxyphenyl)-2-propenamide

4-Benzyloxy-3,5-dimethoxysuccinic acid (10.00 g) was dissolved in chloroform (320 mℓ), followed by the addition of 1-ethyl-3-(3-dimethylaminopropyl)-carbodiimide hydrochloride (6.10 g) and further of 3,4-dimethylaniline (11.56 g). A reaction was conducted with stirring at room temperature. The reaction mixture was washed with 1 N hydrochloric acid and then with brine, and was thereafter dried over anhydrous sodium sulfate. The reaction mixture was fractionated through a silica gel column, and target fractions were obtained. After the target fractions were concentrated to dryness under reduced pressure, the residue was recrystallized from ethanol, whereby white crystalline Compound 65 (7.34 g) was obtained. The data of ¹H-NMR, mass spectrum, infrared absorption spectrum and melting point of this compound are as follows:
¹H-NMR (400MHz,CDCℓ₃) δ: 2.21(3H,s), 2.22(3H,s), 3.80(6H,s), 5.04(2H,s), 6.48(1H,d,J=15.6Hz), 6.70(2H,s), 7.07(1H,d,J=8.0Hz), 7.26-7.48(8H,m), 7.63(1H,d,J=15.6Hz).
MS (EI) m/z: 417(M+), 326, 120, 91.
IR (KBr) νₘₐₓ cm⁻¹: 3310, 1660.
Melting point °C: 148.0-149.1.

### Compound 66: N-(2,6-dimethylphenyl)-(2E)-3-(3,5-dimethoxy-4-benzyloxyphenyl)-2-propenamide

4-Benzyloxy-3,5-dimethoxysuccinic acid (10.00 g) was dissolved in chloroform (320 mℓ), followed by the addition of 1-ethyl-3-(3-dimethylaminopropyl)-carbodiimide hydrochloride (6.10 g) and further of 2,6-dimethylaniline (11.60 g). A reaction was conducted with stirring at room temperature. The reaction mixture was washed with 1 N hydrochloric acid and then with brine, and was thereafter dried over anhydrous sodium sulfate. The reaction mixture was fractionated through a silica gel column, and target fractions were obtained. After the target fractions were concentrated to dryness under reduced pressure, the residue was recrystallized from ethanol, whereby white crystalline Compound 66 (1.04 g) was obtained. The data of ¹H-NMR, mass spectrum, infrared absorption spectrum and melting point of this compound are as follows:
¹H-NMR (400MHz,CDCℓ₃) δ: 2.27(6H,s), 3.86(6H,s), 5.06(2H,s), 6.56(1H,d,J=15.4Hz), 6.78(2H,s), 7.07-7.50(9H,m), 7.66(1H,d,J=15.4Hz).
MS (EI) m/z: 417(M+), 326, 120, 91.
IR (KBr) νₘₐₓ cm⁻¹: 3230, 1660.
Melting point °C: 236.0-236.8.

### Compound 67: N-(2,4,6-trimethylphenyl)-(2E)-3-(3,5-dimethoxy-4-benzyloxyphenyl)-2-propenamide

4-Benzyloxy-3,5-dimethoxysuccinic acid (10.00 g) was dissolved in chloroform (320 mℓ), followed by the addition of 1-ethyl-3-(3-dimethylaminopropyl)-carbodiimide hydrochloride (6.10 g) and further of 2,4,6-trimethylaniline (12.90 g). A reaction was conducted with stirring at room temperature. The reaction mixture was washed with 1 N hydrochloric acid and then with brine, and was thereafter dried over anhydrous sodium sulfate. The reaction mixture was fractionated through a silica gel column, and target fractions were obtained. After the target fractions were concentrated to dryness under reduced pressure, the residue was recrystallized from ethanol, whereby white crystalline Compound 67 (1.92 g) was obtained. The data of ¹H-NMR, mass spectrum, infrared absorption spectrum and melting point of this compound are as follows:
¹H-NMR (400MHz,CDCℓ₃) δ: 2.21(6H,s), 2.27(3H,s), 3.86(6H,s), 5.05(2H,s), 6.56(1H,d,J=15.4Hz), 6.76(2H,s), 6.96(2H,s), 7.25-7.50(5H,m), 7.64(1H,d,J=15.4Hz).
MS (EI) m/z: 431(M+), 340, 135, 91.
IR (KBr) νₘₐₓ cm⁻¹: 3230, 1650.
Melting point °C: 226.5-227.5.

### Compound 68: N-(2,6-diisopropylphenyl)-(2E)-3-(3,5-dimethoxy-4-benzyloxyphenyl)-2-propenamide

4-Benzyloxy-3,5-dimethoxysuccinic acid (10.00 g) was dissolved in chloroform (320 mℓ), followed by the addition of 1-ethyl-3-(3-dimethylaminopropyl)-carbodiimide hydrochloride (6.10 g) and further of 2,6-diisopropylaniline (16.91 g). A reaction was conducted with stirring at room temperature. The reaction mixture was washed with 1 N hydrochloric acid and then with brine, and was thereafter dried over anhydrous sodium sulfate. The reaction mixture was fractionated through a silica gel column, and target fractions were obtained. After the target fractions were concentrated to dryness under reduced pressure, the residue was recrystallized from ethanol, whereby white crystalline Compound 68 (4.91 g) was obtained. The data of ¹H-NMR, mass spectrum, infrared absorption spectrum and melting point of this compound are as follows:
¹H-NMR (400MHz,CDCℓ₃) δ: 1.20(12H,d,J=6.8Hz), 3.06-3.17(2H,m), 3.84(6H,s), 5.06(2H,s), 6.61(1H,d,J=15.4Hz), 6.78(2H,s), 6.94(1H,s), 7.17-7.50(8H,m), 7.66(1H,d,J=15.4Hz).
MS (EI) m/z: 473(M+), 382, 175, 91.
IR (KBr) νₘₐₓ cm⁻¹: 3220, 1675.
Melting point °C: 119.9-200.5.

### Compound 69: N-(4-tert-butylphenyl)-(2E)-3-(3,5-dimethoxy-4-benzyloxyphenyl)-2-propenamide

4-Benzyloxy-3,5-dimethoxysuccinic acid (10.00 g) was dissolved in chloroform (320 mℓ), followed by the addition of 1-ethyl-3-(3-dimethylaminopropyl)-carbodiimide hydrochloride (6.10 g) and further of 4-tert-butylaniline (14.24 g). A reaction was conducted with stirring at room temperature. The reaction mixture was washed with 1 N hydrochloric acid and then with brine, and was thereafter dried over anhydrous sodium sulfate. The reaction mixture was fractionated through a silica gel column, and target fractions were obtained. After the target fractions were concentrated to dryness under reduced pressure, the residue was recrystallized from ethanol, whereby slightly yellow crystalline Compound 69 (4.64 g) was obtained. The data of ¹H-NMR, mass spectrum, infrared absorption spectrum and melting point of this compound are as follows:
¹H-NMR (400MHz,CDCℓ₃) δ: 1.31(9H,s), 3.82(6H,s), 5.04(2H,s), 6.48(1H,d,J=15.4Hz), 6.73(2H,s), 7.27-7.58(9H,m), 7.64(1H,d,J=15.4Hz).
MS (EI) m/z: 445(M+), 354, 189, 91.
IR (KBr) νₘₐₓ cm⁻1: 3260, 1655.
Melting point °C: 218.2-227.9.

### Compound 70: N-(2-tert-butylphenyl)-(2E)-3-(3,5-dimethoxy-4-benzyloxyphenyl)-2-propenamide

4-Benzyloxy-3,5-dimethoxysuccinic acid (9.79 g) was dissolved in chloroform (300 mℓ), followed by the addition of 1-ethyl-3-(3-dimethylaminopropyl)-carbodiimide hydrochloride (8.96 g) and further of 2-tert-butylaniline (13.90 g). A reaction was conducted with stirring at room temperature. The reaction mixture was washed with 1 N hydrochloric acid and then with brine, and was thereafter dried over anhydrous sodium sulfate. The reaction mixture was fractionated through a silica gel column, and target fractions were obtained. After the target fractions were concentrated to dryness under reduced pressure, the residue was recrystallized from ethanol, whereby slightly red crystalline Compound 70 (0.40 g) was obtained. The data of ¹H-NMR, mass spectrum, infrared absorption spectrum and melting point of this compound are as follows:
¹H-NMR (400MHz,CDCℓ₃) δ: 1.43(9H,s), 3.83(6H,s), 5.04(2H,s), 6.50(1H,brs), 6.75(2H,brs), 7.15-7.63(10H,m), 7.67(1H,d,J=15.4Hz).
MS (EI) m/z: 445(M+), 354, 189, 91.
IR (KBr) νₘₐₓ cm⁻¹: 3235, 1650.
Melting point °C: 144.0-148.7.

### Compound 71: N-(4-methylphenyl)-(2E)-3-(3,5-dimethoxy-4-benzyloxyphenyl)-2-propenamide

4-Benzyloxy-3,5-dimethoxysuccinic acid (10.00 g) was dissolved in chloroform (320 mℓ), followed by the addition of 1-ethyl-3-(3-dimethylaminopropyl)-carbodiimide hydrochloride (6.10 g) and further of 4-methylaniline (10.23 g). A reaction was conducted with stirring at room temperature. The reaction mixture was washed with 1 N hydrochloric acid and then with brine, and was thereafter dried over anhydrous sodium sulfate. The reaction mixture was fractionated through a silica gel column, and target fractions were obtained. After the target fractions were concentrated to dryness under reduced pressure, the residue was recrystallized from ethanol, whereby slightly yellow crystalline Compound 71 (2.74 g) was obtained. The data of ¹H-NMR, mass spectrum, infrared absorption spectrum and melting point of this compound are as follows:
¹H-NMR (400MHz,CDCℓ₃) δ: 2.31(3H,s), 3.78(6H,s), 5.03(2H,s), 6.49(1H,d,J=15.4Hz), 6.69(2H,s), 7.12(2H,d,J=8.0Hz), 7.25-7.55(7H,m), 7.62(1H,d,J=15.4Hz), 7.70(1H,brs).
MS (EI) m/z: 403(M+), 312, 147, 92.
IR (KBr) νₘₐₓ cm⁻¹: 1650.
Melting point °C: 136.5-140.7.

### Compound 72: N-(4-methoxyphenyl)-(2E)-3-(3,5-dimethoxy-4-benzyloxyphenyl)-2-propenamide

4-Benzyloxy-3,5-dimethoxysuccinic acid (10.00 g) was dissolved in chloroform (320 mℓ), followed by the addition of 1-ethyl-3-(3-dimethylaminopropyl)-carbodiimide hydrochloride (6.10 g) and further of 4-methoxyaniline (11.75 g). A reaction was conducted with stirring at room temperature. The reaction mixture was washed with 1 N hydrochloric acid and then with brine, and was thereafter dried over anhydrous sodium sulfate. The reaction mixture was fractionated through a silica gel column, and target fractions were obtained. After the target fractions were concentrated to dryness under reduced pressure, the residue was recrystallized from ethanol, whereby slightly yellow crystalline Compound 72 (2.31 g) was obtained. The data of ¹H-NMR, mass spectrum, infrared absorption spectrum and melting point of this compound are as follows:
¹H-NMR (400MHz,CDCℓ₃) δ: 3.77(9H,s), 5.03(2H,s), 6.50(1H,d,J=15.4Hz), 6.68(2H,s), 6.85(2H,d,J=9.0Hz), 7.26-7.57(7H,m), 7.61(1H,d,J=15.4Hz), 7.83(1H,brs).
MS (EI) m/z: 419(M+), 328, 163, 91.
IR (KBr) νₘₐₓ cm⁻¹: 3260, 1655.
Melting point °C: 141.1-142.3.

### Compound 73: N-(4-isopropyloxyphenyl)-(2E)-3-(3,5-dimethoxy-4-benzyloxyphenyl)-2-propenamide

4-Benzyloxy-3,5-dimethoxysuccinic acid (10.42 g) was dissolved in chloroform (320 mℓ), followed by the addition of 1-ethyl-3-(3-dimethylaminopropyl)-carbodiimide hydrochloride (6.35 g) and further of 4-isopropyloxyaniline (15.04 g). A reaction was conducted with stirring at room temperature. The reaction mixture was washed with 1 N hydrochloric acid and then with brine, and was thereafter dried over anhydrous sodium sulfate. The reaction mixture was fractionated through a silica gel column, and target fractions were obtained. After the target fractions were concentrated to dryness under reduced pressure, the residue was recrystallized from ethanol, whereby slightly yellow crystalline Compound 73 (5.10 g) was obtained. The data of ¹H-NMR, mass spectrum, infrared absorption spectrum and melting point of this compound are as follows:
¹H-NMR (400MHz,CDCℓ₃) δ: 1.27(6H,d,J=6.1Hz), 3.67(6H,s), 4.38-4.47(1H,m), 5.00(2H,s), 6.58-6.83(5H,m), 7.22-7.62(8H,m), 8.63(1H,s).
MS (EI) m/z: 447(M+), 356, 191, 92.
IR (KBr) νₘₐₓ cm⁻¹: 3250, 1655.
Melting point °C: 155.0-155.4.

### Compound 74: N-[4-(N',N^{'}-dimethylamino)phenyl]-(2E)-3-(3,5-dimethoxy-4-benzyloxyphenyl)-2-propenamide

4-Benzyloxy-3,5-dimethoxysuccinic acid (10.00 g) was dissolved in chloroform (320 mℓ), followed by the addition of 1-ethyl-3-(3-dimethylaminopropyl)-carbodiimide hydrochloride (12.20 g) and further of 4-(N,N-dimethylamino)aniline (13.00 g). A reaction was conducted with stirring at room temperature. The reaction mixture was washed with 1 N hydrochloric acid and then with brine, and was thereafter dried over anhydrous sodium sulfate. The reaction mixture was fractionated through a silica gel column, and target fractions were obtained. After the target fractions were concentrated to dryness under reduced pressure, the residue was recrystallized from ethanol, whereby yellow crystalline Compound 74 (6.27 g) was obtained. The data of ¹H-NMR, mass spectrum, infrared absorption spectrum and melting point of this compound are as follows:
¹H-NMR (400MHz,CDCℓ₃) δ: 2.86(6H,s), 3.70(6H,s), 5.00(2H,s), 6.55-6.68(5H,m), 7.22-7.63(8H,m), 8.31(1H,s).
MS (EI) m/z: 432(M+), 341, 176, 91.
IR (KBr) νₘₐₓ cm⁻¹: 3245, 1650.
Melting point °C: 187.0-187.2.

### Compound 75: N-(4-benzyloxyphenyl)-(2E)-3-(3,5-dimethoxy-4-benzyloxyphenyl)-2-propenamide

4-Benzyloxy-3,5-dimethoxysuccinic acid (10.00 g) was dissolved in chloroform (320 mℓ), followed by the addition of 1-ethyl-3-(3-dimethylaminopropyl)-carbodiimide hydrochloride (12.20 g) and further of 4-benzyloxyaniline (22.49 g). A reaction was conducted with stirring at room temperature. The reaction mixture was washed with 1 N hydrochloric acid and then with brine, and was thereafter dried over anhydrous sodium sulfate. The reaction mixture was fractionated through a silica gel column, and target fractions were obtained. After the target fractions were concentrated to dryness under reduced pressure, the residue was recrystallized from ethanol, whereby slightly red crystalline Compound 75 (6.22 g) was obtained. The data of ¹H-NMR, mass spectrum, infrared absorption spectrum and melting point of this compound are as follows:
¹H-NMR (400MHz,CDCℓ₃) δ: 3.76(6H,s), 5.01(2H,s), 5.02(2H,s), 6.49(1H,d,J=15.4Hz), 6.67(2H,s), 6.91(1H,d,J=8.8Hz), 7.24-7.56(13H,m), 7.61(1H,d,J=15.4Hz), 7.83(1H,s).
MS (EI) m/z: 495(M+), 404, 238, 92.
IR (KBr) νₘₐₓ cm⁻¹: 3275, 1655.
Melting point °C: 160.9-163.2.

### Compound 76: N-phenyl-(2E)-3-(3,5-dimethoxy-4-benzyloxyphenyl)-2-propenamide

4-Benzyloxy-3,5-dimethoxysuccinic acid (10.00 g) was dissolved in chloroform (320 mℓ), followed by the addition of 1-ethyl-3-(3-dimethylaminopropyl)-carbodiimide hydrochloride (12.20 g) and further of aniline (8.89 g). A reaction was conducted with stirring at room temperature. The reaction mixture was washed with 1 N hydrochloric acid and then with brine, and was thereafter dried over anhydrous sodium sulfate. The reaction mixture was fractionated through a silica gel column, and target fractions were obtained. After the target fractions were concentrated to dryness under reduced pressure, the residue was recrystallized from ethanol, whereby white crystalline Compound 76 (8.85 g) was obtained. The data of ¹H-NMR, mass spectrum, infrared absorption spectrum and melting point of this compound are as follows:
¹H-NMR (400MHz,CDCℓ₃) δ: 3.64(6H,s), 5.00(2H,s), 6.59(2H,s), 6.66(1H,d,J=15.4Hz), 7.06(1H,t), 7.22-7.32(5H,m), 7.40-7.72(5H,m), 8.75(1H,s).
MS (EI) m/z: 389(M+), 280, 132, 91.
IR (KBr) νₘₐₓ cm⁻¹: 3245, 1655.
Melting point °C: 109.1-110.2.

### Compound 77: N-(4-fluorophenyl)-(2E)-3-(3,5-dimethoxy-4-benzyloxyphenyl)-2-propenamide

4-Benzyloxy-3,5-dimethoxysuccinic acid (10.00 g) was dissolved in chloroform (320 mℓ), followed by the addition of 1-ethyl-3-(3-dimethylaminopropyl)-carbodiimide hydrochloride (12.20 g) and further of 4-fluoroaniline (10.60 g). A reaction was conducted with stirring at room temperature. The reaction mixture was washed with 1 N hydrochloric acid and then with brine, and was thereafter dried over anhydrous sodium sulfate. The reaction mixture was fractionated through a silica gel column, and target fractions were obtained. After the target fractions were concentrated to dryness under reduced pressure, the residue was recrystallized from ethanol, whereby slightly yellow crystalline Compound 77 (4.62 g) was obtained. The data of ¹H-NMR, mass spectrum, infrared absorption spectrum and melting point of this compound are as follows:
¹H-NMR (400MHz,CDCℓ₃) δ: 3.73(6H,s), 5.03(2H,s), 6.51(1H,d,J=15.4Hz), 6.64(2H,s), 6.94-7.63(10H,m), 8.13(1H,s).
MS (EI) m/z: 407(M+), 316, 151, 91.
IR (KBr) νₘₐₓ cm⁻¹: 1660.
Melting point °C: 154.5-155.3.

### Compound 78: N-(4-chlorophenyl)-(2E)-3-(3,5-dimethoxy-4-benzyloxyphenyl)-2-propenamide

4-Benzyloxy-3,5-dimethoxysuccinic acid (11.30 g) was dissolved in chloroform (320 mℓ), followed by the addition of 1-ethyl-3-(3-dimethylaminopropyl)-3rbodiimide hydrochloride (6.88 g) and further of 4-chloroaniline (13.70 g). A reaction was conducted with stirring at room temperature. The reaction mixture was washed with 1 N hydrochloric acid and then with brine, and was thereafter dried over anhydrous sodium sulfate. The reaction mixture was fractionated through a silica gel column, and target fractions were obtained. After the target fractions were concentrated to dryness under reduced pressure, the residue was recrystallized from ethanol, whereby slightly yellow crystalline Compound 78 (6.03 g) was obtained. The data of ¹H-NMR, mass spectrum, infrared absorption spectrum and melting point of this compound are as follows:
¹H-NMR (400MHz,CDCℓ₃) δ: 3.77(6H,s), 5.04(2H,s), 6.46(1H,d,J=15.4Hz), 6.67(2H,s), 7.24-7.64(10H,m), 7.86(1H,brs).
MS (EI) m/z: 423(M+), 332, 167, 92.
IR (KBr) νₘₐₓ cm⁻¹: 1655.
Melting point °C: 161.6-163.0.

### Compound 79: N-(4-bromophenyl)-(2E)-3-(3,5-dimethoxy-4-benzyloxyphenyl)-2-propenamide

4-Benzyloxy-3,5-dimethoxysuccinic acid (10.00 g) was dissolved in chloroform (320 mℓ), followed by the addition of 1-ethyl-3-(3-dimethylaminopropyl)-carbodiimide hydrochloride (12.20 g) and further of 4-bromoaniline (16.40 g). A reaction was conducted with stirring at room temperature. The reaction mixture was washed with 1 N hydrochloric acid and then with brine, and was thereafter dried over anhydrous sodium sulfate. The reaction mixture was fractionated through a silica gel column, and target fractions were obtained. After the target fractions were concentrated to dryness under reduced pressure, the residue was recrystallized from ethanol, whereby slightly yellow crystalline Compound 79 (11.1 g) was obtained. The data of ¹H-NMR, mass spectrum, infrared absorption spectrum and melting point of this compound are as follows:
¹H-NMR (400MHz,CDCℓ₃) δ: 3.75(6H,s), 5.04(2H,s), 6.47(1H,d,J=15.4Hz), 6.65(2H,s), 7.25-7.63(10H,m), 7.96(1H,brs),
MS (EI) m/z: 467(M+), 378, 210, 91.
IR (KBr) νₘₐₓ cm⁻¹: 1645.
Melting point °C: 159.6-170.8.

### Compound 80: N-(4-iodophenyl)-(2E)-3-(3,5-dimethoxy-4-benzyloxyphenyl)-2-propenamide

4-Benzyloxy-3,5-dimethoxysuccinic acid (10.00 g) was dissolved in chloroform (320 mℓ), followed by the addition of 1-ethyl-3-(3-dimethylaminopropyl)-carbodiimide hydrochloride (12.20 g) and further of 4-iodoaniline (20.90 g). A reaction was conducted with stirring at room temperature. The reaction mixture was washed with 1 N hydrochloric acid and then with brine, and was thereafter dried over anhydrous sodium sulfate. The reaction mixture was fractionated through a silica gel column, and target fractions were obtained. After the target fractions were concentrated to dryness under reduced pressure, the residue was recrystallized from ethanol, whereby white crystalline Compound 80 (8.06 g) was obtained. The data of ¹H-NMR, mass spectrum, infrared absorption spectrum and melting point of this compound are as follows:
¹H-NMR (400MHz,CDCℓ₃) δ: 3.72(6H,s), 5.03(2H,s), 6.48(1H,d,J=15.6Hz), 6.62(2H,s), 7.25-7.62(10H,m), 8.14(1H,brs).
MS (EI) m/z: 515(M+), 424, 234, 91.
IR (KBr) νₘₐₓ cm⁻¹: 3200, 1655.
Melting point °C: 180.0-182.4.

### Compound 81: N-(3,4-dichlorophenyl)-(2E)-3-(3,5-dimethoxy-4-benzyloxyphenyl)-2-propenamide

4-Benzyloxy-3,5-dimethoxysuccinic acid (10.42 g) was dissolved in chloroform (320 mℓ), followed by the addition of 1-ethyl-3-(3-dimethylaminopropyl)-carbodiimide hydrochloride (6.35 g) and further of 3,4-dichloroaniline (16.11 g). A reaction was conducted with stirring at room temperature. The reaction mixture was washed with 1 N hydrochloric acid and then with brine, and was thereafter dried over anhydrous sodium sulfate. The reaction mixture was fractionated through a silica gel column, and target fractions were obtained. After the target fractions were concentrated to dryness under reduced pressure, the residue was recrystallized from ethanol, whereby slightly yellow crystalline Compound 81 (2.82 g) was obtained. The data of ¹H-NMR, mass spectrum, infrared absorption spectrum and melting point of this compound are as follows:
¹H-NMR (400MHz,CDCℓ₃) δ: 3.72(6H,s), 5.04(2H,s), 6.48(1H,d,J=15.4Hz), 6.63(2H,s), 7.23-7.48(7H,m), 7.59(1H,d,J=15.4Hz), 7.81(1H,d,J=2.2Hz), 8.31(1H,brs).
MS (EI) m/z: 457(M+), 366, 201, 92.
IR (KBr) νₘₐₓ cm⁻¹: 1655.
Melting point °C: 144.8-145.4.

### Compound 82: N-(4-trifluoromethylphenyl)-(2E)-3-(3,5-dimethoxy-4-benzyloxyphenyl)-2-propenamide

4-Benzyloxy-3,5-dimethoxysuccinic acid (10.00 g) was dissolved in chloroform (320 mℓ), followed by the addition of 1-ethyl-3-(3-dimethylaminopropyl)-carbodiimide hydrochloride (12.20 g) and further of 4-trifluoroaniline (15.40 g). A reaction was conducted with stirring at room temperature. The reaction mixture was washed with 1 N hydrochloric acid and then with brine, and was thereafter dried over anhydrous sodium sulfate. The reaction mixture was fractionated through a silica gel column, and target fractions were obtained. After the target fractions were concentrated to dryness under reduced pressure, the residue was recrystallized from ethanol, whereby white crystalline Compound 82 (0.52 g) was obtained. The data of ¹H-NMR, mass spectrum, infrared absorption spectrum and melting point of this compound are as follows:
¹H-NMR (400MHz,CDCℓ₃) δ: 3.80(6H,s), 5.05(2H,s), 6.46(1H,d,J=15.6Hz), 6.70(2H,s), 7.25-7.80(11H,m).
MS (EI) m/z: 457(M+), 366, 201, 92.
IR (KBr) νₘₐₓ cm⁻¹: 3300, 1660.
Melting point °C: 170.5-172.5.

### Compound 83: N-(3,5-bistrifluoromethylphenyl)-(2E)-3-(3,5-dimethoxy-4-benzyloxyphenyl)-2-propenamide

4-Benzyloxy-3,5-dimethoxysuccinic acid (10.00 g) was dissolved in chloroform (320 mℓ), followed by the addition of 1-ethyl-3-(3-dimethylaminopropyl)-carbodiimide hydrochloride (12.20 g) and further of 3,5-bis(trifluoromethyl)aniline (20.00 g). A reaction was conducted with stirring at room temperature. The reaction mixture was washed with 1 N hydrochloric acid and then with brine, and was thereafter dried over anhydrous sodium sulfate. The reaction mixture was fractionated through a silica gel column, and target fractions were obtained. After the target fractions were concentrated to dryness under reduced pressure, the residue was recrystallized from ethanol, whereby white crystalline Compound 83 (0.86 g) was obtained. The data of ¹H-NMR, mass spectrum, infrared absorption spectrum and melting point of this compound are as follows:
¹H-NMR (400MHz,CDCℓ₃) δ: 3.86(6H,s), 4.97(2H,s), 6.74(1H,d,J=15.6Hz), 7.01(2H,s), 7.30-7.50(5H,m), 7.64(1H,d,J=15.6Hz), 7.75(1H,s), 8.38(2H,s), 10.85(1H,s).
MS (EI) m/z: 525(M+), 434, 208, 92.
IR (KBr) νₘₐₓ cm⁻¹: 3355, 1695.
Melting point °C: 203.4-206.9.

### Compound 84: N-(4-acetylphenyl)-(2E)-3-(3,5-dimethoxy-4-benzyloxyphenyl)-2-propenamide

4-Benzyloxy-3,5-dimethoxysuccinic acid (10.00 g) was dissolved in chloroform (320 mℓ), followed by the addition of 1-ethyl-3-(3-dimethylaminopropyl)-carbodiimide hydrochloride (12.20 g) and further of 4-aminoacetophenone (12.90 g). A reaction was conducted with stirring at room temperature. The reaction mixture was washed with 1 N hydrochloric acid and then with brine, and was thereafter dried over anhydrous sodium sulfate. The reaction mixture was fractionated through a silica gel column, and target fractions were obtained. After the target fractions were concentrated to dryness under reduced pressure, the residue was recrystallized from ethanol, whereby white crystalline Compound 84 (1.68 g) was obtained. The data of ¹H-NMR, mass spectrum, infrared absorption spectrum and melting point of this compound are as follows:
¹H-NMR (400MHz,CDCℓ₃) δ: 2.58(3H,s), 3.86(6H,s), 5.03(2H,s), 6.71(1H,d,J=15.4Hz), 6.79(2H,s), 7.27-7.50(5H,m), 7.63(1H,d,J=15.4Hz), 7.82-7.96(4H,m).
MS (EI) m/z: 431(M+), 340, 208, 91.
IR (KBr) νₘₐₓ cm⁻¹: 3270, 1650.
Melting point °C: 176.9-178.2.

### Compound 85: N-(4-phenoxyphenyl)-(2E)-3-(3,5-dimethoxy-4-benzyloxyphenyl)-2-propenamide

4-Benzyloxy-3,5-dimethoxysuccinic acid (10.00 g) was dissolved in chloroform (320 mℓ), followed by the addition of 1-ethyl-3-(3-dimethylaminopropyl)-carbodiimide hydrochloride (12.20 g) and further of 4-phenoxyaniline (17.70 g). A reaction was conducted with stirring at room temperature. The reaction mixture was washed with 1 N hydrochloric acid and then with brine, and was thereafter dried over anhydrous sodium sulfate. The reaction mixture was fractionated through a silica gel column, and target fractions were obtained. After the target fractions were concentrated to dryness under reduced pressure, the residue was recrystallized from ethanol, whereby slightly yellow crystalline Compound 85 (4.60 g) was obtained. The data of ¹H-NMR, mass spectrum, infrared absorption spectrum and melting point of this compound are as follows:
¹H-NMR (400MHz,CDCℓ₃) δ: 3.76(6H,s), 5.03(2H,s), 6.52(1H,d,J=15.4Hz), 6.68(2H,s), 6.96(4H,d,J=8.8Hz), 7.07(1H,t), 7.24-7.66(10H,m), 7.98(1H,brs).
MS (EI) m/z: 481(M+), 390, 224, 91.
IR (KBr) νₘₐₓ cm⁻¹: 3245, 1655.
Melting point °C: 145.6-146.6.

### Tests

Advantageous effects of the present invention will next be described in further detail by Tests. It should however be borne in mind that the present invention is by no means limited to these Tests.

### (Test 1)

### 〈Inhibitory effects on rat-derived ACAT〉

To test effects on ACAT, a reaction was conducted *in vitro* to form (¹⁴C)-cholesteryl oleate from endogeneous cholesterol in the presence of (¹⁴C)-oleoyl-CoA as a substrate by using a crude enzyme prepared from microzome protein fractions of a rat liver.

The reaction mixture had the following composition: 20 µℓ of microsome protein (1.83 mg/mℓ), 20 µℓ of (¹⁴C)-oleoyl-CoA (5 µCi/mℓ, BSA: 96 nmol/mℓ), 106 µℓ of 100 mM phosphate buffer (containing 5 mM of dithiothreitol, pH 7.4) and a sample dissolved in 4 µℓ of methanol or dimethyl sulfoxide; 150 µℓ in total.

The reaction was conducted at 37°C. After an elapse of a predetermined reaction time (18 minutes in general), a 2:1 mixture of chloroform and methanol (5.65 mℓ) was added to terminate the reaction. A 0.04 N aquous solution of hydrochloric acid (0.98 mℓ) and as a carrier, cholesteryl oleate were added, followed by extraction for 20 minutes on a shaking apparatus. The mixture was then allowed to stand overnight in a cold dark place. On the following day, the separated lower chloroform layer was extracted in its entirety and was dried under a nitrogen gas stream. The remaining upper layer was added with the lower layer (5.65 mℓ) of the Folch's partition solution, and was extracted again. After the mixture was allowed to stand overnight in a dark place, the lower layer was drawn in its entirety. This lower layer was combined with the lower chloroform layer fraction, which had already been dried to solid, and the mixture was then dried to solid.

The solid was then dissolved in chlorofrom (200 µℓ), and the solution was coated in its entirety on a G60 plate (aluminum sheet) for silica gel thin layer chromatography. After the solid was developedover 10 cm with a 10:190 mixture of diethyl ether and petroleum ether, autoradiography was conducted and the formation of (¹⁴C)-colesteryl oleate was confirmed. This section was then cut off. The radioactivity of each sample was measured by subjecting it to liquid scintillation counting for 5 minutes in toluene or was measured for 30 minutes by a "Betascope" (manufactured by BETA-GEN INC. The results so obtained are shown in Table 1.

**Table 1**

| ACAT Inhibitory Activity | | | |
|---|---|---|---|
| Compound | ACAT inhibitory activity (IC₅₀: nM) | Compound | ACAT inhibitory activity (IC₅₀: nM) |
| 4 | 54 | 33 | 35 |
| 5 | 38 | 34 | 17 |
| 6 | 11 | 35 | 65 |
| 7 | 20 | 36 | 37 |
| 8 | 10 | 37 | 45 |
| 9 | 45 | 43 | 47 |
| 10 | 71 | 45 | 29 |
| 11 | 60 | 56 | 55 |
| 13 | 79 | 57 | 70 |
| 14 | 15 | 63 | 52 |
| 15 | 15 | 64 | 35 |
| 16 | 19 | 65 | 47 |
| 17 | 22 | 66 | 47 |
| 18 | 63 | 68 | 72 |
| 19 | 70 | 71 | 37 |
| 20 | 62 | 72 | 35 |
| 21 | 6 | 74 | 90 |
| 22 | 5 | 75 | 36 |
| 26 | 18 | 77 | 76 |
| 27 | 38 | 78 | 35 |
| 28 | 23 | 82 | 42 |
| 29 | 24 | 83 | 60 |
| 32 | 93 | 84 | 64 |

### (Test 2)

### 〈Inhibitory effects on the accumulation of cholesterol esters in macrophage〉

Inhibitory effects on the accumulation of cholesterol esters in macrophage were tested using J774.1 strain, an established cell line of mouse macrophage.

Metabolism of LDL in endothelial cells or smooth muscle cells causes downregulation of the LDL receptor by excess free cholesterol in the cells, but in a macrophage, uptake of modified lDL takes place via a scavenger receptor. As no regulation is applied to this pathway, cholesterol esters progressively accumulate without any limitation.

It was therefore studied whether or not corydaline would inhibit intracellular accumulation of cholesterol esters by having liposomes, which were formed of ¹⁴C-labelled cholesterol and phosphatidylcholine, uptaken in macrophage cells and then causing them to act on acyl-CoA:cholesterol acyltransferase which existed on rough-surfaced endoplasmic reticula.

The macrophage cells were incubated for 2 days under 5% CO₂ on an RPMI 1640 medium (product of NISSUI PHARMACEUTICAL CO., LTD.) which had been added with 10% fetal bovine serum, penicilin (50 U/mℓ) and streptomycin (50 µg/mℓ). Trypsinization was then conducted, and cells attached to the bottom of each well were peeled off and formulated into a cell suspension. The cells were washed with a fresh supply of the above-described culture medium, and the cell solution was then seeded at 2 x 10⁶ cells/mℓ on a 24-well multiple well plate ("25820-24", product of CORNING INC.). After incubation at 37°C under 5% CO₂ for 37 hours, the culture medium was replaced with a fresh supply of the above-described culture medium and, after a 0.3 M glucose solution (40 µℓ) with cholesterol [80 µg; containing 0.4 µCi of (³H)-cholesterol] and phosphatidylcholine (160 µg) contained therein and a methanol solution (10 µℓ)with a test sample dissolved therein were added, incubation was conducted for 12 hours. These procedures were carried out aseptically.

After completion of the incubation, cells attached to each bottom were washed 5 times with a serum-free RPMI 1640 medium by a graduated pipet, and isopropanol (1 mℓ) was added to extract intracellular cholesterol and formed cholesterol esters. The extract was coated in its entirety on a silica gel thin layer chromatography ("Art 5553", product of MERCK & Co., INC.) and subsequent to development with a 80:20:2 mixture of hexane, ethyl ether and formic acid, autoradiography was performed.

A section, which contained ³H-labelled cholesterol esters and cholesterol, was cut off. Its radioactivity was measured by a liquid scintillation counter and compared with a control, whereby a cholesterol ester accumulation inhibition rate was determined. The results so obtained are shown in Table 2.

**Table 2**

| Inhibitory Activity against Accumulation of Intracellular Cholesterol Esters in Macrophage | | | |
|---|---|---|---|
| Compound | Cholesterol accumulation inhibitory activity (IC₅₀: nM) | Compound | Cholesterol accumulation inhibitory activity (IC₅₀: nM) |
| 11 | 189 | 57 | 736 |
| 12 | 860 | 58 | 930 |
| 13 | 200 | 59 | 620 |
| 14 | 270 | 60 | 870 |
| 15 | 310 | 61 | 840 |
| 16 | 160 | 62 | 840 |
| 17 | 69 | 63 | 520 |
| 18 | 87 | 64 | 900 |
| 19 | 250 | 65 | 46 |
| 20 | 28 | 66 | 900 |
| 21 | 312 | 67 | 870 |
| 22 | 73 | 68 | 790 |
| 23 | 32 | 69 | 28 |
| 24 | 720 | 70 | 840 |
| 26 | 109 | 71 | 120 |
| 27 | 720 | 72 | 170 |
| 28 | 394 | 73 | 31 |
| 29 | 700 | 74 | 990 |
| 30 | 192 | 75 | 22 |
| 31 | 700 | 76 | 490 |
| 33 | 832 | 77 | 470 |
| 34 | 48 | 78 | 170 |
| 35 | 377 | 79 | 78 |
| 36 | 104 | 80 | 43 |
| 37 | 91 | 81 | 150 |
| 40 | 833 | 82 | 30 |
| 41 | 792 | 83 | 430 |
| 43 | 253 | 84 | 30 |
| 48 | 300 | 85 | 26 |
| 56 | 734 | | |

As is appreciated from the foregoing, the various compounds according to the present invention exhibit substantial inhibitory activities against ACAT activity and intracellular cholesterol accumulation inhibitory effects, so that they are considered to be useful for the prevention and treatment of disases caused by accumulation of cholesterol in humans.

### (Test 3)

### 〈Plasma Cholesterol Elevation Inhibitory Effects on Rats Loaded with High Cholesterl Diet〉

Using 5-week old male SD rats (120-150 g, 10 rats per group, individual feeding), plasma cholesterol elevation inhibitory effects of various compounds in the Examples on rats were studied. To a control group, a feed which had been prepared by adding 1.5% of cholesterol, 0.5% of cholic acid, 10% of coconut oil and 10% of sugar to "F-2 Diet" (product of FUNABASHI FARM CO., LTD.) was given *ad libitum* as a hyperlipemia inducing diet.

To each test-compound-administered group, on the other hand, a feed which had been prepared by adding 0.005% of the corresponding compound in the Examples to the hyperlipemia inducing diet of the above-described composition was given *ad libitum.* Further, a normal diet group to which a feed similar to the hyperlipemia inducing diet except for the exclusion of cholesterol was also tested similarly. As a testing method, after these feeds were given *ad libitum* for 3 days, bloods were drawn on the 4th day from the abdominal aortas of the individual rats under anesthesia with Nembutal. Subsequent to collection of plasma from each blood sample, the plasma cholesterol was quantitated by a "DETERMINER TC555 KIT" (product of Kyowa Medex Co., Ltd.) while the HDL cholesterol was quantitated by "DETERMINER HDL KIT" (product of Kyowa Medex Co., Ltd.). The liver cholesterol was measured by extracting it in accordance with the Folch's method and then measuring it with a "DETERMINER TC555 KIT". Statistical processing of the data so obtained was conducted by the Danett's test.

The results obtained upon feed-mixed administration of the individual compounds in the Examples are shown in Table 3.

**Table 3**

| Elevation Rate (%) of Plasma Total Cholesterol in Rats | |
|---|---|
| Compound | Inhibitory rate (%) of plasma total cholesterol elevation in rat |
| 4 | 20 |
| 5 | 46 |
| 6 | 79 |
| 7 | 59 |
| 8 | 75 |
| 9 | 28 |
| 10 | 33 |
| 16 | 49 |
| 17 | 44 |
| 24 | 20 |
| 26 | 27 |
| 28 | 55 |
| 29 | 22 |
| 30 | 31 |
| 32 | 20 |
| 33 | 28 |
| 36 | 50 |
| 37 | 64 |
| 40 | 20 |
| 45 | 22 |
| 46 | 57 |
| 52 | 23 |
| 55 | 34 |

### (Test 4)

### N-[4-(2,4-Dimethylphenyl)-1,4-diazaocyclohexyl]-(2E)-3-(3,5-dimethoxy-4-benzyloxyphenyl)-2-propenamide

Using 5-week old male SD rats (120-150 g, 8 rats per group, individual feeding), serum cholesterol elevation inhibitory effects of Compound 20 in the Examples on rats were studied. To a control group, a feed which had been prepared by adding 1.5% of cholesterol, 0.5% of cholic acid, 10% of coconut oil and 10% of sugar to "F-2 Diet" (product of FUNABASHI FARM CO., LTD.) was given *ad libitum* as a hyperlipemia inducing diet.

To test-compound-administered groups, on the other hand, feeds which had been prepared by adding 0.010, 0.025, 0.050, 0.075 and 0.100% of compound 20 in the Examples to the hyperlipemia inducing diet of the above-described composition, respectively, were given *ad libitum*. Further, a normal diet group to which a feed similar to the hyperlipemia inducing diet except for the exclusion of cholesterol was also tested similarly.

After these feeds were given *ad libitum* for 3 days, bloods were drawn on the 4th day from the abdominal aortas of the individual rats under anesthesia with Nembutal. Subsequent to collection of serum from each blood sample, the serum cholesterol was quantitated by an "AUTOSERA S-CHO KIT" (product of Daiichi Pure Chemicals Co., Ltd.), the serum triglyceride was quantitated by an "AUTOSERA TG-N KIT" (product of Daiichi Pure Chemicals Co., Ltd.), and the serum phospholipid was quantitated by an "AUTOSERA PL KIT". Further, the HDL cholesterol was quantitated by an "AUTOSERA S-CHO KIT" (product of Daiichi Pure Chemicals Co., Ltd.) after fractionating, it by a "DETERMINER HDL KIT" (product of Kyowa Medex Co., Ltd.). Statistical processing of the data was conducted by the Danett's test.

The results are shown in FIG. 1 and Table 4 and Table 5.

**Table 4**

| Inhibitory Effects of Compound 20 on Serum Total Cholesterol Elevation | |
|---|---|
| Amount (%) of medicine added to feed | Inhibitory rate (%) of serum total cholesterol elevation |
| 0.075 | 50 |
| 0.010 | 63 |

**Table 5**

| Effects of Compound 20 on Serum Lipid | | | |
|---|---|---|---|
| Test group | TC(mg/dℓ) | HDLC(mg/dℓ) | TG(mg/dℓ) |
| Control (hyperlipemia inducing diet) | 724.1±333.5 | 11.6±4.5 | 161.5±65.8 |
| Compound 20 (0.010%) | 539.7±251.3 | 16.8±7.3 | 149.5±43.1 |
| Compound 20 (0.025%) | 529.3±235.4 | 15.3±4.9 | 169.3±62.1 |
| Compound 20 (0.050%) | 567.3±168.9 | 13.4±3.2 | 219.4±103.2 |
| Compound 20 (0.075%) | 413.7±146.3* | 16.5±4.1 | 198.6±34.19 |
| Compound 20 (0.100%) | 337.6±145.2** | 18.2±5.0* | 218.4±57.06 |
| TC: total cholesterol, HDLC: HDL cholesterol, TG: triglyceride, PL: phospholipid. The Dunnet's test was conducted relative to the control (hyperlipeia inducing diet). | | | |

| | | | |
|---|---|---|---|
| *: P<0.05, | | | |
| **: P<0.01. | | | |

### (Test 5)

### N-[4-(2,4-Dimethylphenyl)-1,4-diazaocyclohexyl]-(2E)-3-(3,5-dimethoxy-4-nonyloxyphenyl)-2-propenamide

Using 5-week old male SD rats (120-150 g, 8 rats per group, individual feeding), plasma cholesterol elevation inhibitory effects of Compound 16 on rats were studied.

To a control group, a feed which had been prepared by adding 1.5% of cholesterol, 0.5% of cholic acid, 10% of coconut oil and 10% of sugar to "F-2 Diet" (product of FUNABASHI FARM CO., LTD.) was given *ad libitum* as a hyperlipemia inducing diet.

To test-compound-administered groups, on the other hand, feeds which had been prepared by adding 0.0025, 0.0050, 0.0100 and 0.0200% of Compound 16 to the hyperlipemia inducing diet of the above-described composition, respectively, were given *ad libitum.* Further, a normal diet group to which a feed similar to the hyperlipemia inducing diet except for the exclusion of cholesterol was also tested similarly.

After these feeds were given *ad libitum* for 3 days, bloods were drawn on the 4th day from the abdominal aortas of the individual rats under anesthesia with Nembutal. Subsequent to collection of plasma from each blood sample, the plasma cholesterol was quantitated by an "AUTOSERA S-CHO KIT" (product of Daiichi Pure Chemicals Co., Ltd.), the plasma triglyceride was quantitated by an "AUTOSERA TG-N KIT" (product of Daiichi Pure Chemicals Co., Ltd.), and the plasma phospholipid was quantitated by an "AUTOSERA PL KIT" (product of Daiichi Pure Chemicals Co., Ltd.). Further, the HDL cholesterol was quantitated by an "AUTOSERA S-CHO KIT" (product of Daiichi Pure Chemicals Co., Ltd.) after fractionating it by a "DETERMINER HDL KIT" (product of Kyowa Medex Co., Ltd.). The liver lipid was quantitated subsequent to its extraction by the Folch's method. Statistical processing of the data so obtained was conducted by the Dunnet's test.

The results are shown in FIG. 2 and Table 6 to Table 8.

**Table 6**

| Inhibitory Effects of Compound 16 on Plasma Total Cholesterol Elevation | |
|---|---|
| Amount (%) of Compound 16 added to feed | Inhibitory rate (%) of plasma total cholesterol elevation |
| 0.0050 | 52 |
| 0.0100 | 78 |
| 0.0200 | 91 |

**Table 7**

| Effects of Compound 16 on Plasma Lipid | | | | |
|---|---|---|---|---|
| Test group | TC(mg/dℓ) | HDLC(mg/dℓ) | TC(mg/dℓ) | PL(mg/dℓ) |
| Control(hyperlipemia inducing diet) | 493.1±221.5 | 19.8±1.8 | 206.5±83.3 | 339.0±119.9 |
| Control (normal diet) | 109.8±8.2** | 38.1±5.7** | 110.3±38.8 | 207.7±14.4** |
| Compound 16 (0.0025%) | 509.5±269.3 | 15.8±3.4 | 128.2±39.3 | 296.6±80.2 |
| Compound 16 (0.0050%) | 292.8±68.3* | 18.0±4.0 | 142.0±23.0 | 228.4±30.6** |
| Compound 16 (0.0100%) | 194.1±59.0** | 24.1±6.3 | 112.9±39.0 | 197.1±39.3** |
| Compound 16 (0.0200%) | 145.9±56.4** | 27.0±4.1** | 62.8±18.2** | 141.5±19.7** |
| TC: total cholesterol, HDLC: HDL cholesterol, TG: triglyceride, PL: phospholipid. The Dunnet's test was conducted relative to the control (hyperlipemia inducing diet). | | | | |

| | | | | |
|---|---|---|---|---|
| *: P<0.05, | | | | |
| **: P<0.01. | | | | |

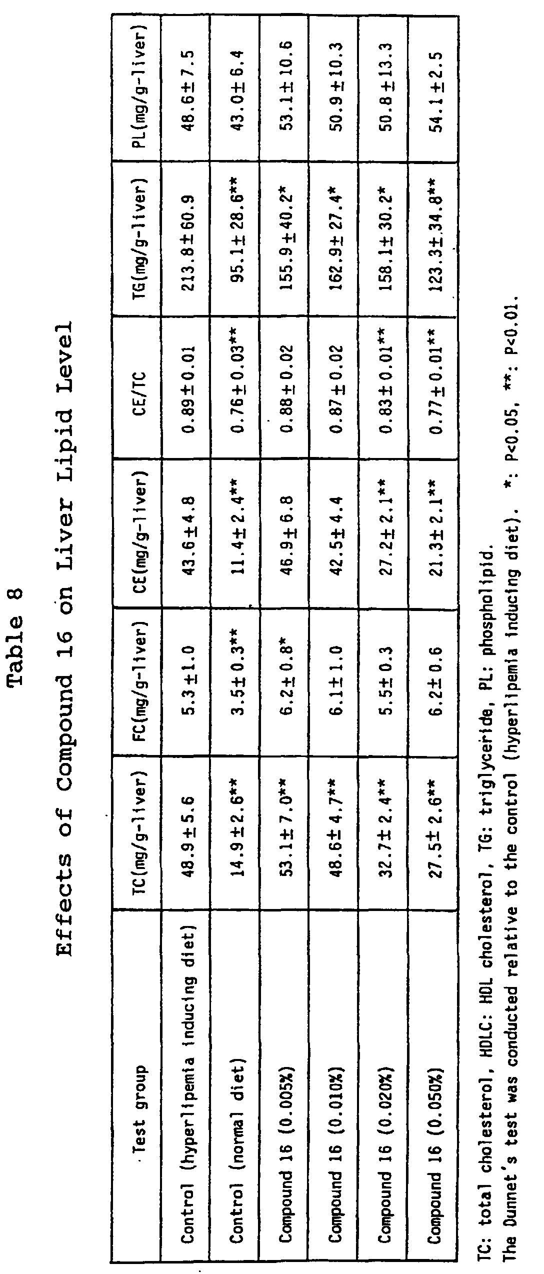

### (Test 6)

### N-[4-(2,4-Dimethylphenyl)-1,4-diazaocyclohexyl]-(2E)-3-(3,5-dimethoxy-4-nonyloxyphenyl)-2-propenamide

Using 5-week old male SD rats (120-150 g, 8 rats per group, group feeding), plasma cholesterol elevation inhibitory effects of compound 16 in Examples on rats were studied.

To rats in a control group, only a 1.5% aqueous solution of carboxymethylcellulose was orally administered once a day for 7 days. During the last 3 days, a hyperlipemia inducing diet [a feed prepared by adding 1.5% of cholesterol, 0.5% of cholic acid, 10% of coconut oil and 10% of sugar to "F-2 Diet" (product of FUNABASHI FARM CO., LTD.)] was given *ad libitum.* To rats in a control group (normal diet), however, a feed (normal diet) similar to the hyperlipemia inducing diet except for the exclusion of cholesterol was given *ad libitum.*

To test-compound-administered groups, on the other hand, Compound 16 suspended in a 1.5% aqueous solution of carboxymethylcellulose was continuously and orally administered at dosages of 10, 20 and 40 mg/kg/day, respectively, once a day for 7 days. During the last 3 days, a hyperlipemia inducing diet [a feed prepared by adding 1.5% of cholesterol, 0.5% of cholic acid, 10% of coconut oil and 10% of sugar to "F-2 Diet" (product of FUNABASHI FARM CO., LTD.)] was given *ad libitum.*

On the 4th day after the initiation of feeding of the hyperlipemia inducing diet and the normal diet, bloods were drawn from the abdominal aortas of the individual rats under anesthesia with Nembutal. Subsequent to collection of plasma from each blood sample, the plasma cholesterol was quantitated by an "AUTOSERA S-CHO KIT" (product of Daiichi Pure Chemicals Co., Ltd.), the plasma triglyceride was quantitated by an "AUTOSERA TG-N KIT" (product of Daiichi Pure Chemicals Co., Ltd.), and the plasma phospholipid was quantitated by an "AUTOSERA PL KIT" (product of Daiichi Pure Chemicals Co., Ltd.). Further, the HDL cholesterol was quantitated by an "AUTOSERA S-CHO KIT" (product of Daiichi Pure Chemicals Co., Ltd.) after fractionating it by a "DETERMINER HDL KIT" (product of Kyowa Medex Co., Ltd.). The liver lipid level was quantitated by a commercial measuring kit in a similar manner as in the measurement of plasma lipid subsequent to its extraction by the Folch's method. Statistical processing of the data so obtained was conducted by the Dunnet's test.

The results are shown in FIG. 3 and Table 9 to Table 11.

**Table 9**

| Inhibitory Effects of Compound 16 on Plasma Total Cholesterol Elevation | |
|---|---|
| Dosage (mg/kg/day) | Inhibitory rate (%) of plasma total cholesterol elevation |
| 10 | 9 |
| 20 | 58 |
| 40 | 69 |

**Table 10**

| Effects of Compound 16 on Plasma Lipid | | | | |
|---|---|---|---|---|
| Test group | TC(mg/dℓ) | HDLC(mg/dℓ) | TG(mg/dℓ) | PL(mg/dℓ) |
| Control (hyperlipemia inducing diet) | 360.4 ± 211.3 | 21.7 ± 5.7 | 182.0 ± 164.1 | 264.4 ± 90.5 |
| Control (normal diet) | 92.9 ± 5.5** | 31.3 ± 8.5** | 98.1 ± 62.8 | 129.9 ± 20.0** |
| Compound 16 (10 mg/kg) | 337.1 ± 87.0 | 20.5 ± 3.8 | 182.5 ± 81.1 | 275.5 ± 89.7 |
| Compound 16 (20 mg/kg) | 205.2 ± 74.8* | 23.2 ± 5.1 | 158.8 ± 83.2 | 186.3 ± 64.6 |
| Compound 16 (40 mg/kg) | 176.1 ± 67.3* | 23.4 ± 3.2 | 129.6 ± 75.6 | 160.6 ± 66.4* |
| TC: total cholesterol, HDLC: HDL cholesterol, TG: triglyceride, PL: phospholipid. The Dunnet's test was conducted relative to the control. | | | | |

| | | | | |
|---|---|---|---|---|
| *: P<0.05, | | | | |
| **: P<0.01 | | | | |

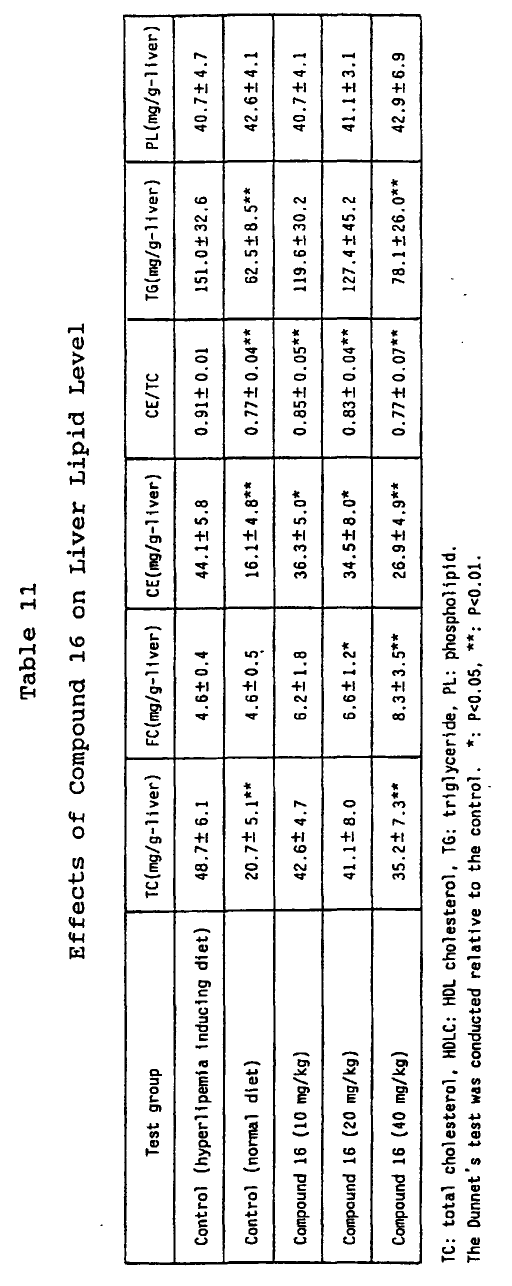

### (Test 7)

### N-[4-(2,4-Dimethylphenyl)-1,4-diazaocyclohexyl]-(2E)-3-(3,5-dimethoxy-4-nonyloxyphenyl)-2-propenamide

Using 5-week old male SD rats (120-150 g, 8 rats per group, group feeding), plasma cholesterol elevation inhibitory effects of Compound 16 in Examples on rats were studied.

To rats in a control group, only olive oil was continuously and orally administered once a day for 7 days. During the last 3 days, a hyperlipemia inducing diet [a feed prepared by adding 1.5% of cholesterol, 0.5% of cholic acid, 10% of coconut oil and 10% of sugar to "F-2 Diet" (product of FUNABASHI FARM CO., LTD.)] was given *ad libitum.*

To test-compound-administered groups, on the other hand, Compound 16 dissolved in olive oil was continuously and orally administered at dosages of 10, 20 and 40 mg/kg/day, respectively, once a day for 7 days. During the last 3 days, a hyperlipemia inducing diet [a feed prepared by adding 1.5% of cholesterol, 0.5% of cholic acid, 10% of coconut oil and 10% of sugar to "F-2 Diet" (product of FUNABASHI FARM CO., LTD.)] was given *ad libitum.*

On the 4th day after the initiation of feeding of the hyperlipemia inducing diet, bloods were drawn from the abdominal aortas of the individual rats under anesthesia with Nembutal. Subsequent to collection of plasma from each blood sample, the plasma cholesterol was quantitated by an "AUTOSERA S-CHO KIT" (product of Daiichi Pure Chemicals Co., Ltd.), the plasma triglyceride was quantitated by an "AUTOSERA TG-N KIT" (product of Daiichi Pure Chemicals Co., Ltd.), and the plasma phospholipid was quantitated by an "AUTOSERA PL KIT" (product of Daiichi Pure Chemicals Co., Ltd.). Further, the HDL cholesterol was quantitated by an "AUTOSERA S-CHO KIT" (product of Daiichi Pure Chemicals Co., Ltd.) after fractionating it by a "DETERMINER HDL KIT" (product of Kyowa Medex Co., Ltd.). The liver lipid level was quantitated by a commercial measuring kit in a similar manner as in the measurement of plasma lipid subsequent to its extraction by the Folch's method. Statistical processing of the data so obtained was conducted by the Dunnet's test.

The results are shown in FIG. 4 and Table 12 to Table 14.

**Table 12**

| Inhibitory Effects of Compound 16 on Plasma Total Cholesterol Elevation | |
|---|---|
| Dosage (mg/kg/day) | Inhibitory rate (%) of plasma total cholesterol elevation |
| 10 | 58 |
| 20 | 72 |
| 40 | 91 |

**Table 13**

| Effects of Compound 16 on Plasma Lipid | | | | |
|---|---|---|---|---|
| Test group | TC(mg/dℓ) | HDLC(mg/dℓ) | TG(mg/dℓ) | PL(mg/dℓ) |
| Control (hyperlipemia inducing diet) | 377.6 ± 121.9 | 18.2 ± 2.3 | 186.6 ± 132.3 | 253.9 ± 57.1 |
| Compound 16 (10 mg/kg) | 217.1 ± 72.9* | 24.8 ± 2.4* | 148.2 ± 26.1 | 239.0 ± 32.3 |
| Compound 16 (20 mg/kg) | 179.1 ± 73.9** | 20.8 ± 4.4 | 168.9 ± 78.8 | 183.2 ± 52.6** |
| Compound 16 (40 mg/kg) | 124.8 ± 28.9** | 33.3 ± 7.2** | 75.9 ± 34.8 | 157.3 ± 28.6** |
| TC: total cholesterol, HDLC: HDL cholesterol, TG: triglyceride, PL: phospholipid. The Dunnet's test was conducted relative to the control. | | | | |

| | | | | |
|---|---|---|---|---|
| *: P<0.05, | | | | |
| **; P<0.01. | | | | |

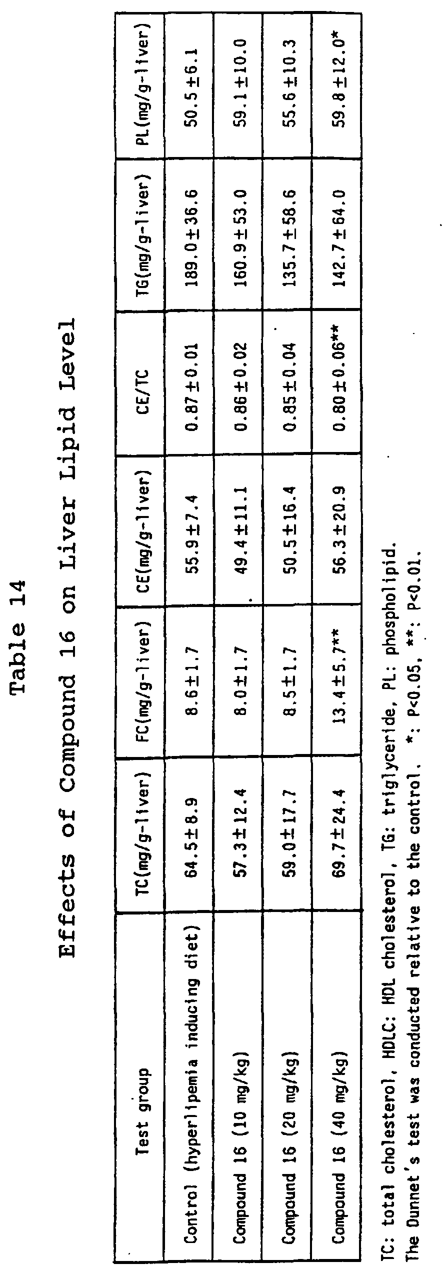

### (Test 8)

### N-[4-(2,4-Dimethylphenyl)-1,4-diazaocyclohexyl]-(2E)-3-(3,5-dimethoxy-4-decyloxyphenyl)-2-propenamide

Using 5-week old male SD rats (120-150 g, 8 rats per group, individual feeding), plasma cholesterol elevation inhibitory effects of Compound 17 in the Examples on rats were studied. To a control group, a feed which had been prepared by adding 1.5% of cholesterol, 0.5% of cholic acid, 10% of coconut oil and 10% of sugar to "F-2 Diet" (product of FUNABASHI FARM CO., LTD.) was given *ad libitum* as a hyperlipemia inducing diet.

To test-compound-administered groups, on the other hand, feeds which had been prepared by adding 0.005, 0.010, 0.020 and 0.050% of Compound 17 to the hyperlipemia inducing diet of the above-described composition, respectively, were given *ad libitum*. Further, a normal diet group to which a feed similar to the hyperlipemia inducing diet except for the exclusion of cholesterol was also tested similarly.

After these feeds were given *ad libitum* for 3 days, bloods were drawn on the 4th day from the abdominal aortas of the individual rats under anesthesia with Nembutal. Subsequent to collection of plasma from each blood sample, the plasma cholesterol was quantitated by an "AUTOSERA S-CHO KIT" (product of Daiichi Pure Chemicals Co., Ltd.), the plasma triglyceride was quantitated by an "AUTOSERA TG-N KIT" (product of Daiichi Pure Chemicals Co., Ltd.), and the plasma phospholipid was quantitated by an "AUTOSERA PL KIT". Further, the HDL cholesterol was quantitated by an "AUTOSERA S-CHO KIT" (product of Daiichi Pure Chemicals Co., Ltd.) after fractionating it by a "DETERMINER HDL KIT" (product of Kyowa Medex Co., Ltd.). The liver lipid was quantitated subsequent to its extraction by the Folch's method. Statistical processing of the data so obtained was conducted by the Dunnet's test.

The results are shown in FIG. 5 and Table 15 to Table 17.

**Table 15**

| Inhibitory Effects of Compound 17 on Plasma Total Cholesterol Elevation | |
|---|---|
| Amount (%) of Compound 17 added to feed | Inhibitory rate (%) of plasma plasma cholesterol elevation |
| 0.005 | 49 |
| 0.010 | 82 |
| 0.020 | 93 |
| 0.050 | 101 |

**Table 16**

| Effects of Compound 17 on Plasma Lipid | | | |
|---|---|---|---|
| Test group | TC(mg/dℓ) | HDLC(mg/dℓ) | TG(mg/dℓ) |
| Control (hyperlipemia inducing diet) | 677.5±268.6 | 20.4±6.3 | 123.2±61.1 |
| Control (normal diet) | 119.7±13.0** | 45.0±3.8** | 128.4±45.4 |
| Compound 17 (0.005%) | 401.9±119.2** | 21.2±6.4 | 170.9±50.5 |
| Compound 17 (0.010%) | 221.4±44.3** | 29.3±3.8** | 135.4±54.5 |
| Compound 17 (0.020%) | 141.1±38.8** | 28.1±4.0* | 102.3±34.5 |
| Compound 17 (0.050%) | 114.3±18.5** | 31.9±5.3** | 80.0±28.3 |
| TC: total cholesterol, HDLC: HDL cholesterol, TG: triglyceride. The Dunnet's test was conducted relative to the control (hyperlipemia inducing diet). | | | |

| | | | |
|---|---|---|---|
| *: P<0.05, | | | |
| **: P<0.01. | | | |

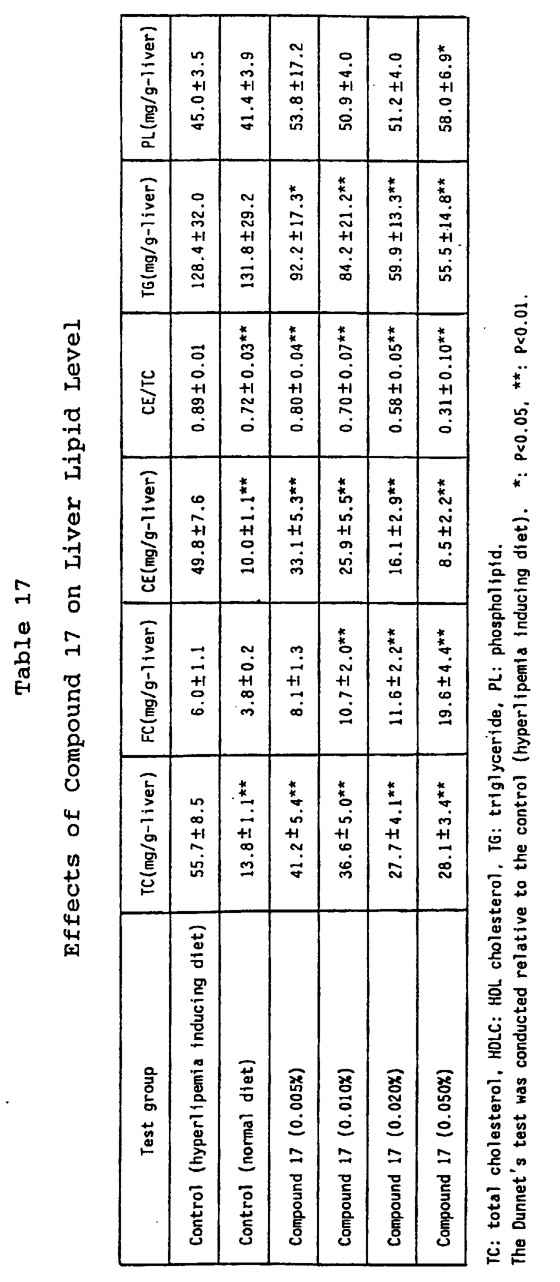

### (Test 9)

### N-[4-(3,4-Dimethylphenyl)-1,4-diazaocyclohexyl]-(2E)-3-(3,5-dimethoxy-4-heptyloxyphenyl)-2-propenamide

Using 5-week old male SD rats (120-150 g, 8 rats per group, group feeding), plasma cholesterol elevation inhibitory effects of Compound 5 in Examples on rats were studied.

To rats in a control group, only olive oil was continuously and orally administered once a day for 7 days. During the last 3 days, a hyperlipemia inducing diet [a feed prepared by adding 1.5% of cholesterol, 0.5% of cholic acid, 10% of coconut oil and 10% of sugar to "F-2 Diet" (product of FUNABASHI FARM CO., LTD.)] was given *ad libitum.* To rats in a control group (normal diet), however, a feed (normal diet) similar to the hyperlipemia inducing diet except for the exclusion of cholesterol was given *ad libitum.*

To rats in test-compound-administered groups, on the other hand, Compound 5 dissolved in olive oil was continuously and orally administered at dosages of 20 and 40 mg/kg/day, respectively, once a day for 7 days. During the last 3 days, a hyperlipemia inducing diet [a feed prepared by adding 1.5% of cholesterol, 0.5% of cholic acid, 10% of coconut oil and 10% of sugar to "F-2 Diet" (product of FUNABASHI FARM CO., LTD.)] was given *ad libitum.*

On the 4th day after the initiation of feeding of the hyperlipemia inducing diet or the normal diet, bloods were drawn from the abdominal aortas of the individual rats under anesthesia with Nembutal. Subsequent to collection of plasma from each blood sample, the plasma cholesterol was quantitated by an "AUTOSERA S-CHO KIT" (product of Daiichi Pure Chemicals Co., Ltd.), the plasma triglyceride was quantitated by an "AUTOSERA TG-N KIT" (product of Daiichi Pure Chemicals Co., Ltd.), and the plasma phospholipid was quantitated by an "AUTOSERA PL KIT" (product of Daiichi Pure Chemicals Co., Ltd.). Further, the HDL cholesterol was quantitated by an "AUTOSERA S-CHO KIT" (product of Daiichi Pure Chemicals Co., Ltd.) after fractionating it by a "DETERMINER HDL KIT" (product of Kyowa Medex Co., Ltd.). The liver lipid level was quantitated by a commercial measuring kit in a similar manner as in the measurement of plasma lipid subsequent to its extraction by the Folch's method. Statistical processing of the data so obtained was conducted by the Dunnet's test.

The results are shown in FIG. 6 and Table 18 to Table 20.

**Table 18**

| Inhibitory Effects of Compound 5 on Plasma Total Cholesterol Elevation | |
|---|---|
| Dosage (mg/kg/day) | Inhibitory rate (%) of plasma total cholesterol elevation |
| 20 | 1 |
| 40 | 43 |

**Table 19**

| Effects of Compound 5 on Plasma Lipid | | | | |
|---|---|---|---|---|
| Test group | TC(mg/dℓ) | HDLC(mg/dℓ) | TG(mg/dℓ) | PL(mg/dℓ) |
| Control (hyperlipemia inducing diet) | 416.5 ± 57.2 | 17.1 ± 3.3 | 109.6 ± 55.7 | 264.9 ± 41.6 |
| Control (normal diet) | 99.8 ± 8.1** | 41.3 ± 10.1** | 99.2 ± 56.3 | 183.6 ± 28.4 |
| Compound 5 (20 mg/kg) | 412.9 ± 158.9 | 19.7 ± 3.0 | 116.8 ± 49.4 | 266.1 ± 90.0 |
| Compound 5 (40 mg/kg) | 279.8 ± 85.3* | 22.3 ± 3.5* | 126.1 ± 66.1 | 223.8 ± 57.6 |
| TC: total cholesterol, HDLC: HDL cholesterol, TG: triglyceride, PL: phospholipid. The Dunnet's test was conducted relative to the control. | | | | |

| | | | | |
|---|---|---|---|---|
| *: P<0.05 | | | | |
| **: P<0.01. | | | | |

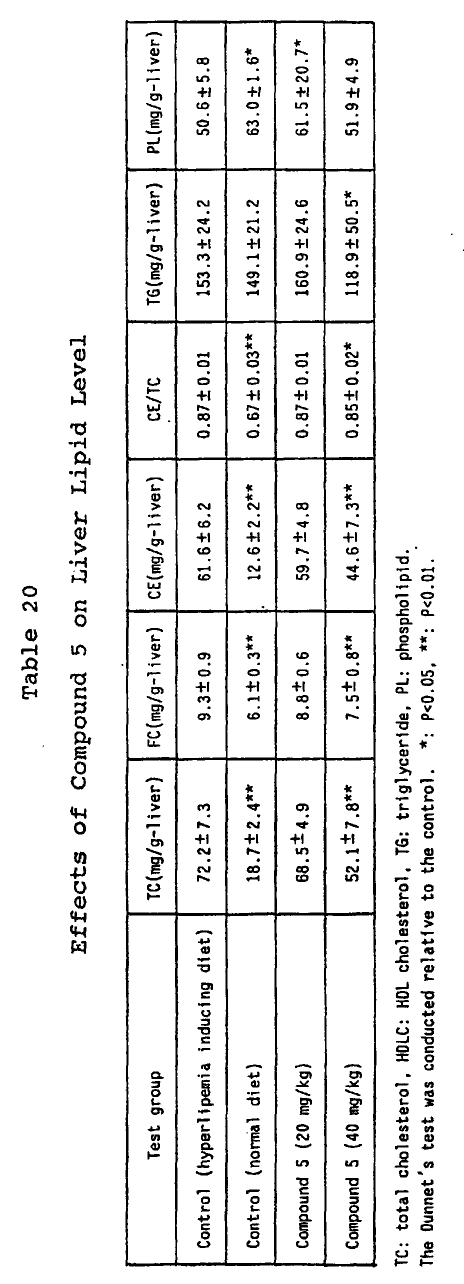

### (Test 10)

### N-[4-3,4-Dimethylphenyl)-1,4-diazaocyclohexyl]-(2E)-3-(3,5-dimethoxy-4-octyloxyphenyl)-2-propenamide

Using 5-week old male SD rats (120-150 g, 8 rats per group, group feeding), plasma cholesterol elevation inhibitory effects of compound 6 in Examples on rats were studied.

To rats in a control group, only a 1.5% aqueous solution of carboxymethylcellulose was orally administered once a day for 7 days. During the last 3 days, a hyperlipemia inducing diet [a feed prepared by adding 1.5% of cholesterol, 0.5% of cholic acid, 10% of coconut oil and 10% of sugar to "F-2 Diet" (product of FUNABASHI FARM CO., LTD.)] was give*n ad libitum.* To rats in a control group (normal diet), however, a feed (normal diet) similar to the hyperlipemia inducing diet except for the exclusion of cholesterol was given *ad libitum.*

To test-compound-administered groups, on the other hand, compound 6 suspended in a 1.5% aqueous solution of carboxymethylcellulose was continuously and orally administered at dosages of 5, 10 and 20 mg/kg/day, respectively, once a day for 7 days. During the last 3 days, a hyperlipemia inducing diet [a feed prepared by adding 1.5% of cholesterol, 0.5% of cholic acid, 10% of coconut oil and 10% of sugar to "F-2 Diet" (product of FUNABASHI FARM CO., LTD.)] was given *ad libitum.*

On the 4th day after the initiation of feeding of the hyperlipemia inducing diet and the normal diet, bloods were drawn from the abdominal aortas of the individual rats under anesthesia with Nembutal. Subsequent to collection of plasma from each blood sample, the plasma cholesterol was quantitated by an "AUTOSERA S-CHO KIT" (product of Daiichi Pure Chemicals Co., Ltd.), the plasma triglyceride was guantitated by an "AUTOSERA TG-N KIT" (product of Daiichi Pure Chemicals Co., Ltd.), and the plasma phospholipid was quantitated by an "AUTOSERA PL KIT" (product of Daiichi Pure Chemicals Co., Ltd.). Further, the HDL cholesterol was quantitated by an "AUTOSERA S-CHO KIT" (product of Daiichi Pure Chemicals Co., Ltd.) after fractionating it by a "DETERMINER HDL KIT" (product of Kyowa Medex Co., Ltd.). The liver lipid level was quantitated by a commercial measuring kit in a similar manner as in the measurement of plasma lipid subsequent to its extraction by the Folch's method. Statistical processing of the data so obtained was conducted by the Dunnet's test.

The results are shown in FIG. 7 and Table 21 to Table 23.

**Table 21**

| Inhibitory Effects of Compound 6 on Plasma Total Cholesterol Elevation | |
|---|---|
| Dosage (mg/kg/day) | Inhibitory rate (%) of plasma total cholesterol elevation |
| 5 | 34 |
| 10 | 57 |
| 20 | 66 |

**Table 22**

| Effects of Compound 6 on Plasma Lipid | | | | |
|---|---|---|---|---|
| Test group | TC(mg/dℓ) | HDLC(mg/dℓ) | TG(mg/dℓ) | PL(mg/dℓ) |
| Control (hyperlipemia inducing diet) | 413.6 ± 164.1 | 17.3 ± 4.9 | 113.2 ± 27.9 | 247.9 ± 32.8 |
| Control (normal diet) | 85.3 ± 7.4** | 33.6 ± 4.8** | 99.7 ± 63.0 | 163.8 ± 16.6 |
| Compound 6 (5 mg/kg) | 303.1 ± 99.4* | 19.3 ± 4.5 | 202.3 ± 99.7* | 243.2 ± 42.5 |
| Compound 6 (10 mg/kg) | 226.6 ± 94.7** | 20.5 ± 4.0 | 159.0 ± 68.6 | 206.7 ± 50.6 |
| Compound 6 (20 mg/kg) | 196.8 ± 44.1** | 22.8 ± 2.2** | 187.6 ± 79.3* | 203.6 ± 21.3 |
| TC: total cholesterol, HDLC: HDL cholesterol, TC: triglyceride, PL: phospholipid. The Dunnet's test was conducted relative to the control (hyperlipemia inducing diet). | | | | |

| | | | | |
|---|---|---|---|---|
| *: P<0.05, | | | | |
| **: P<0.01. | | | | |

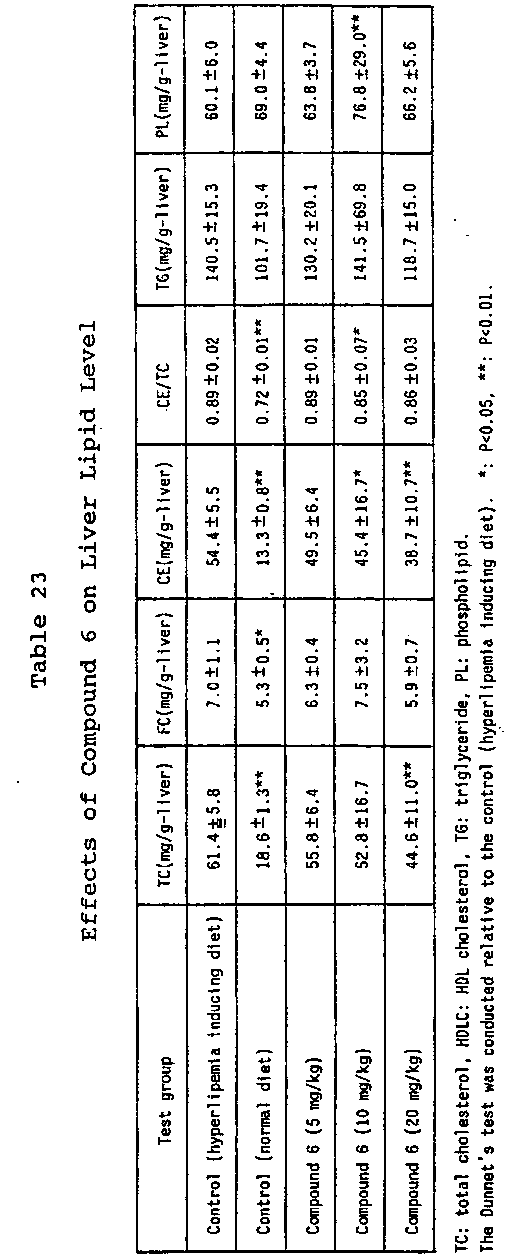

### (Toxicity Test)

Concerning Compound 6, a provisional toxicity test was conducted.

### (Testing Method)

Using female SD rats (5-weeks old), the test was conducted at a size of 6 rats per group. The animals were subjected to group feeding, and were allowed to take both "F-2 Feed" (product of FUNABASHI FARM CO., LTD.) and tap wate*r ad libitum.*

Compound 6 which had been suspended in a 1.5% aqueous solution of CMC was continuously and orally administered by a stomach tube for 14 days or 28 days such that the dosage became 100, 250 or 250 mg/kg/day. To a control group, on the other hand, an aqueous solution of CMC was administered.

Both during the administration and after the administration, no difference was observed between the administered groups and the control group in external appearance and also in the results of various biochemical tests. Therefore, safety has been confirmed.

| Preparation 1 (tablets) | |
|---|---|
| Individual compound | 10 g |
| Corn starch | 40 g |
| Crystalline cellulose | 45 g |
| Carboxymethylcellulose sodium | 4 g |
| Light anhydrous silicic acid | 500 mg |
| Magnesium stearate | 500 mg |
| Total | 100 g |

The above ingredients were mixed into a uniform mixture by a mixer and were then compression-formed by a tableting machine, whereby tablets of 250 mg per tablet were obtained. Each of the tablets contained the compound in an amount of 25 mg, and an adult should be given 5 to 30 tablets in several portions per day.

| Preparation 2 (capsules) | |
|---|---|
| Individual compound | 20 g |
| Corn starch | 79.5 g |
| Light anhydrous silicic acid | 500 mg |
| Total | 100 g |

The above ingredients were mixed by a mixer, and each capsule was filled with 200 mg of the mixture to obtain a capsule preparation. Each capsule contained one of the compounds, which are described in Table 1, in an amount of 40 mg, and an adult should be given 1 to 30 capsules per day.

| Preparation 3 (granule) | |
|---|---|
| Individual compound | 10 g |
| Corn starch | 40 g |
| 10% Ethanol solution of hydroxypropylcellulose | 50 g |
| Total | 100 g |

The above ingredients were mixed and kneaded by a mixer, granulated by an extrusion granulator, and then dried, whereby a granule was obtained. The granule contains one of the compounds, which are described in Table 1, in an amount of 100 mg per gram, and an adult should be given 1 to 8 g of the granule in several portions per day.

### Capability of Exploitation in Industry

The compounds according to the present invention have strong ACAT inhibitory activity, lower blood cholesterol and inhibit accumulation of cholesterol, and are hence useful as prophylactic and therapeutic agents for human arteriosclerosis.

## Claims

1. A phenylpropenone compound represented by the following formula (1): 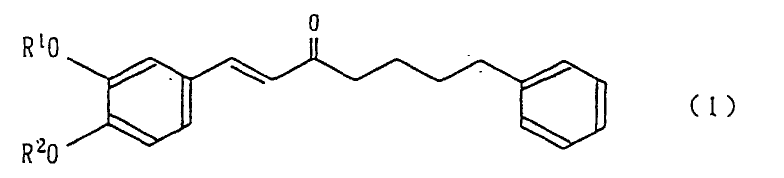 wherein R¹ and R² may be the same or different and each independently represent a hydrogen atom, a phenyl group, an aralkyl group, or a saturated or unsaturated, linear or branched hydrocarbon group.

2. A phenylpropenone compound represented by the following formula (2): 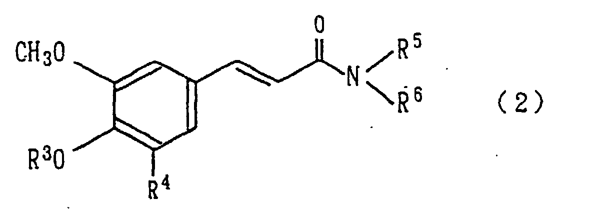 wherein R³ represents a hydrogen atom, a phenyl group, an aralkyl group, a saturated or unsaturated, linear or branched hydrocarbon group, an alkoxyalkyl group or an aryloxyalkyl group, R⁴ represents a hydrogen atom, a halogen atom, a lower alkyl group, a lower alkoxyl group, a phenyl group, an aralkyl group, a halogeno(lower alkyl) group or a nitro group, and concerning R⁵ and R⁶, R⁶ represents a substituted or unsubstituted phenyl group, a substituted or unsubstituted aralkyl group, a substituted or unsubstituted aralkylpiperidino group or a substituted or unsubstituted anilinocarbonylmethyl group when R⁵ is a hydrogen atom, or R⁵ and R⁶ may be coupled together with the adjacent nitrogen atom to form a piperazine ring which may be substituted at the 4-position thereof with a substituted or unsubstituted phenyl group or with a substituted or unsubstituted heterocyclic ring group.

3. An acyl-CoA:cholesterol acyltransferase (ACAT) inhibitor comprising the compound of claim 1 or 2 as an active ingredient.

4. A medicine comprising the compound of claim 1 or 2 as an active ingredient.

5. A medicine of claim 4, which is a blood cholesterol lowering agent, a fatty liver inhibiting agent, or a prophylatic and therapeutic agent for arteriosclerosis.

6. A medicinal composition comprising the compound of claim 1 or 2 and a medicinally acceptable carrier.

7. Use of the compound of claim 1 or 2 as an acyl-CoA:cholesterol acyltransferase (ACAT) inhibitor.

8. Use of the compound of claim 1 or 2 as a medicine.

9. A method for the treatment of hypercholesterolemia, fatty liver or arteriosclerosis, which comprises administering an effective amount of the compound of claim 1 or 2 to a patient.
